# EUROPEAN PATENT SPECIFICATION

(11) **EP 4 217 007 B1**
(45) Date of publication and mention of the grant of the patent: **06.11.2024**
(21) Application number: 21783267.4
(22) Date of filing: 27.09.2021
(51) Int. Cl.: A61K 47/64, A61P 35/00

(54) **PEPTIDIC CONJUGATES OF SN38 USEFUL IN THE TREATMENT OF CANCER**
IN DER BEHANDLUNG VON KREBS NÜTZLICHE, PEPTIDISCHE KONJUGATE VON SN38
CONJUGUÉS PEPTIDIQUES DE SN38 UTILES POUR LE TRAITEMENT DU CANCER

(30) Priority: 28.09.2020 EP 20382854
(43) Date of publication of application: 02.08.2023
(73) Proprietor: Fundació Institut de Recerca Biomèdica (IRB Barcelona), 08028 Barcelona (ES); Universitat de Barcelona, 08028 Barcelona (ES); Hospital Sant Joan de Deu, 08950 Esplugues de Llobregat Barcelona (ES)
(72) Inventor: SANCHEZ NAVARRO, Macarena, 08028 BARCELONA (ES); TEIXIDÓ TURÁ, Meritxell, 08028 BARCELONA (ES); GENÉ OLACIREGUI, Nagore, 08950 ESPLUGUES DEL LLOBREGAT (ES); GIRALT LLEDÓ, Ernest, 08028 BARCELONA (ES); MONTERO CARCABOSO, Angel, 08950 ESPLUGUES DE LLOBREGAT (ES)
(74) Representative: ZBM Patents - Zea, Barlocci & Markvardsen
(86) International application number: PCT/EP2021/076546
(87) International publication number: WO 2022/064052

(56) References cited:
- WO-A1-2015/001015
- WO-A1-2018/064683
- NONAKA MOTOHIRO ET AL: "Overcoming the blood-brain barrier by Annexin A1-binding peptide to target brain tumours", BRITISH JOURNAL OF CANCER, vol. 123, no. 11, 14 September 2020 (2020-09-14), pages 1633 - 1643, XP037304397, ISSN: 0007-0920, DOI: 10.1038/S41416-020-01066-2
- OLLER-SALVIA BENJAMÍ ET AL: "MiniAp-4: A Venom-Inspired Peptidomimetic for Brain Delivery", vol. 55, no. 2, 23 October 2015 (2015-10-23), pages 572 - 575, XP055785613, ISSN: 1433-7851, Retrieved from the Internet <URL:https://api.wiley.com/onlinelibrary/tdm/v1/articles/10.1002%2Fanie.201508445> DOI: 10.1002/anie.201508445

## Description

This application claims the benefit of European Patent Application EP20382854.6 filed September 28th, 2020

### Technical Field

The present invention relates to the field of delivery systems for SN38, which is an anticancer drug and their therapeutical indications.

### Background Art

Cancer is a heterogeneous disease characterized by the accumulation of tumor cells, which can cause the death of both animals and humans. It is also one of the leading cause of death from disease among children and adolescents. Although substantial progress has been made in the treatment of several types of cancer over the past five decades, in particular, childhood cancer, progress against other types has been limited. The annual incidence of tumors in children is between 100-160 cases per million. There is an annual risk of 1 in 500 children under 15. This incidence is slightly lower in industrialized countries. Brain and spinal cord tumors account for 25% of all neoplasms (40-50% of all pediatric solid tumors). Despite progress, survival data for these patients remains low, at around 55%. This contrasts with the substantial survival gains in recent years seen in other types of patients, such as those affected by leukaemia or extracranial tumours.

Conventional methods of treating cancer include surgical treatments, the administration of chemotherapeutic agents, and recently immune response-based therapy which involve the administration of an antibody or antibody fragment which may be conjugated to a therapeutic moiety. However, to date, such treatments have been of limited success. Camptothecin, one of the four major structural classifications of plant-derived anti-cancerous compounds, is a cytotoxic alkaloid which consists of a pentacyclic ring structure containing a pyrrole (3, 4 β) quinoline moiety, an S-configured lactone form, and a carboxylate form. Irinotecan is made from natural camptothecin which is found in the Chinese ornamental tree *Camptotheca acuminata.* Several types of cancers have been treated at some point with irinotecan, among them glioblastoma multiforme (GBM) in the case of adults, and diffuse intrinsic pontine glioma (DIPG), paediatric glioblastoma (*pGBM*), neuroblastoma, rhabdomyosarcoma, Ewing sarcoma, and retinoblastoma in the case of children.

Gliomas are a group of tumors that start in the glial cells of the brain or the spine, comprise about 30 percent of all brain tumors and central nervous system tumours, and 80 percent of all malignant brain tumours. Often, treatment for brain gliomas is a combined approach, using surgery, radiation therapy, and chemotherapy. DIPG primarily affects children, usually between the ages of 5 and 7. Unfortunately, this is the majority of brain stem tumors, comprising 60-70%, and it is the one with the worst prognosis of all. No treatment has been shown to be effective and the average survival is 9 months. Radiation and the administration of steroids is the only thing that has a palliative effect and that increases survival very discreetly. So far, there is no useful chemotherapy and multiple clinical trials have been tested, without favourable results. Pediatric glioblastomas are another group of tumors comprises one third of hemispheric tumors. It has a peak incidence at 8 and 12 years. Glioblastomas also affects adult patients, aroung 2-3 cases per 100.000 population.

Ewing Sarcoma is the second cause of malignant bone tumor in children and adolescents. The annual incidence is 0.6 per million inhabitants. It is rare before the age of 5 and the peak incidence is between 10 and 15 years, affecting more boys than girls, but this relationship in the sex varies according to the age range. The most common places for it to start are the pelvic (hip) bones, the chest wall (such as the ribs or shoulder blades), or in the middle of the leg bones. Ewing sarcoma can also present as an extra-skeletal lesion in the absence of bone injury. In this variant, there is a high risk of lymphatic spread and treatment is usually similar to rhabdomyosarcoma.

Soft tissue sarcomas are divided into rhabdomyosarcoma and non-rhabdomyosarcoma. Rhabdomyosarcoma accounts for 50% of all soft tissue sarcomas in children. It is the third solid extracranial tumor in frequency, after neuroblastoma and Wilms' tumor. The age peak is bimodal, with a first peak between 2 and 5 years, and a second peak in adolescence, between 15 and 19 years. While sarcomas in adults occur mostly in the extremities, in children they can originate in any location in the body, both in skeletal muscle and soft tissue. The most affected area in children is the head and neck and the urogenital tract. The extremities are affected in 20% of patients. Overall survival is poor, except if the tumor is found in locations where it can be completely resected, which sometimes requires limb amputation or voiding. Survival varies from 7-70%, depending on the location.

Neuroblastoma is the most common extracranial solid tumor of the childhood. Due to its embryonic origin, neuroblastomas can be virtually found in any part of the sympathetic nervous system, but the most common localization of the locoregional disease is on the adrenal gland (44%). Children between 1.5 - 6 years of age at diagnosis still can still be cured with conventional treatments, but their probability of survival decreases when they are diagnosed with metastatic disease (stage 4 neuroblastoma). About 50% of the newly diagnosed patients already present metastasis to bone (60%), bone marrow (50%), lymph nodes (42%) and/or liver (15%) and need intensive chemotherapy treatment, surgery and radiotherapy, but their survival remains poor and few advances have been made over the last decades.

Finally, retinoblastoma is the most common cause of eye tumor in children, with a global incidence of 1 in 20,000 live births. It typically occurs in the first 2 years of life. Of the 30-40% are bilateral, in these cases there is always a positive family history. Of the unilaterals, 10% have a germline mutation of the Rb gene, located on chromosome 13. If detected early, they have a 95% survival rate. In certain parts of the world, when the diagnosis is late, the survival is drastically reduced, to less than 20%. Treatment depends on the tumor control achieved. When tumor regression is not controlled by chemotherapy and brachytherapy, enucleation is recommended. Even if enucleation is performed, in some cases there is tumor invasion into the optic nerve, which will force extra chemotherapy treatment.

The efficacy of irinotecan has proven to be limited by its hepatic activation that results in a low conversion rate, high interpatient variability, and dose-limiting gastrointestinal toxicity.

SN38 is the active metabolite of irinotecan and is formed via hydrolysis of irinotecan by liver carboxylesterases and metabolized via glucuronidation by UGT1A1. It has the following formula

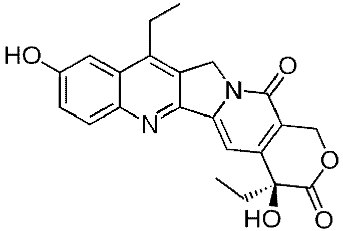

Since its discovery it has attracted the attention of all scientists for its potent antineoplastic effect. SN38 has 1000 times more activity than irinotecan itself and could be used to treat the same type of cancer than irinotecan. In vitro cytotoxicity assays show that the potency of SN38 relative to irinotecan varies from 2- to 2000-fold. Even so, SN38 has also significant limitations at the chemical, pharmacological and toxic level. At the chemical level, unlike irinotecan that is soluble in water, SN38 is practically insoluble in water, and in most solvents and oils, so it is unfeasible to administer SN38 to patients. Many solvents have been tested and it is only possible to solubilize SN38 at 0.5%, with dimethyl sulfoxide, formic acid and Transcutol^{®} HP, as well as with NaOH 0.1 M, but the basic pH of this NaOH 0.1 M water solution inactivates SN38. The high lipophilic character and its instability in aqueous solutions of the drug, makes it necessary to be administered with a polar aprotic solvent, with all the inconveniences that this entails. In addition, a second problem is the presence of a terminal lactone ring, which makes it unstable in aqueous solutions, producing non-enzymatic and pH-dependent hydrolysis to form a hydrocarboxylic ring, which is not powerful in inhibiting topoisomerase. Thus, at neutral or basic pH, the balance is shifted towards less active species, while at more acidic pH the formation of lactone is favored, with greater inhibitory power. The drug also contains an asymmetric carbon in position 20, with the S form being the pharmacologically active configuration.

Given the unfavourable characteristics of the drug, several investigations have led to formulations of SN38 as a prodrug, in order to solve the problem of insolubility and thus take advantage of the powerful mechanism of action. Several conjugate strategies have been applied to SN38 to release the drug instead from the use of the prodrug.

Some of them are based on a slow release of SN38 from a soluble conjugate, for instance US8299089B2 proposes the conjugation to poly(ethylene glycol) to a multi-armed PEGs to improve the solubility and that releases the SN38 due to ester hydrolysis. However, these conjugates must be administered at high levels in order to be efficacious.

WO2015/051307A1 discloses some conjugates that release SN38 from a polyethylene glycol through a β-elimination mechanism at slow rates to enable low-dose and long-term exposure regimes.

F. Koizumi et al in "Novel SN38-incorporating polymeric micelles, NK012, eradicate vascular endothelial growth factor-secreting bulky tumors", Cancer Res 2006, vol. 66(20) pp. 10048-56 discloses a poly(ethylene glycol)-poly(glutamic acid) block copolymer chemically conjugated to SN38 from self-assembly nanoparticles (NPs) that outperformed irinotecan in preclinical studies,
Other conjugate strategies are for instance based on the use of peptidic conjugates to bypass the hepatic activation and reduce the gastrointestinal toxicity and interpatient variability compared to irinotecan.

Thus, F Meyer-Losic et al in "DTS-108, A novel Peptidic prodrug of SN38: In vivo Efficacy and Toxicokinetic Studies", Clinical Cancer Research 2008, vol.14, issue 7, pp. 2145-2153, proposes to conjugate SN38 to a cationic peptide (Vectocell) via an esterase cleavable linker to deliver significantly higher levels of SN38 than irinotecan, without the associated toxicity of irinotecan, resulting in an increased therapeutic window for DTS-108 in preclinical models.

WO2007/113687A2 discloses the preparation of camptothecin-cell penetrating peptide conjugates through a linker, by means of a thioether bond, such as the conjugate DPV1047-MIC-SN38 (DPV-1047 is the peptide VKRGLKLRHVRPRVTRMDV; MIC is 6-maleimidohexanoic acid residue; SN38 is 7-ethyl-10-hydroxycamptothecin) which has shown to have significant anti-tumoral activity against the human HCT-116 cell line.

Finally, WO2018/064683A1 discloses the synthesis of a tumor-vascular targeting antitumor agent, IF7-SN38, wherein the antitumor agent comprises an annexin-1 binding peptide having the sequence IFLLWQR (IF7) conjugated to an anticancer drug (SN38) through the linker 4-{4-[(N-maleimydomethyl)cycloheanecarboxamido]methyl}cyclohexane -1-carboylic acid. This compound targets brain tumor and overcomes blood-brain barriers by passive transcytosis mechanism.

Nonaka, M., Suzuki-Anekoji, M., Nakayama, J. et al. Overcoming the blood-brain barrier by Annexin A1-binding peptide to target brain tumours. Br J Cancer 123, 1633-1643 (2020) describes that IF7C(RR)-SN38 crosses the blood-brain barrier and suppresses growth of brain tumours in mouse models.

WO 2015/001015 describes actively transported and protease-resistant peptides as BBB shuttles and shuttle-cargo constructs.

Despite the efforts to provide conjugates of SN38 to allow the adequate administration and bioavailability of SN38 for the treatment of cancer, there is still an unmet medical need of finding improved systems to efficiently administer SN38.

### Summary of Invention

The inventors have developed a conjugate of SN38 with a specific group of peptides, which are joined to the SN38 through a specific type of linker, and which maintains an antitumoral activity against several cell lines from brain and extracranial cancers, similar to SN38. The conjugates of SN38 present high solubility in water and unlike SN38 which is practically insoluble, it could be administered to patients due to such solubility. The activity of the new conjugates of SN38 is much higher than the activity of irinotecan *in vitro.* Advantageously, they show good stability in human serum *in vitro.*

The peptides forming part of the conjugate are known and were previously described in WO2015/001015A1. This document discloses that these peptides have the capacity to cross the blood-brain barrier (BBB) and that have the capacity of transporting cargoes which cannot cross the BBB by themselves, in particular big cargoes such as proteins and antibodies. They use an active transport mechanism to cross the BBB. B. Oller et al., in "MiniAp-4: A Venom-Inspired Peptidomimetic for Brain Delivery", Angew Chem Int Ed 2016, vol.55, pp.572-575 focused on the peptide DapKAPETALD and disclosed that it is resistant to proteases and is able to efficiently deliver diverse cargoes across the blood-brain barrier in human-cell-based model and also in vivo.

The inventors have found that for the case of SN38 good results of antitumoral activity are obtained by its conjugation with certain peptides of those disclosed in WO2015/001015A1 and linkers of a certain size that are larger than the linkers disclosed in the mentioned document. These conjugates are advantageous with respect to other conjugates because they allow to maintain an antitumoral activity similar to SN38 and up to 100 times superior to the *in vitro* activity of irinotecan.

The comparative data provided in the experimental section show that, when the SN38 is conjugated through the same linker to another peptide (THRre) part/all of the antitumoral activity is lost (Example 7 of the invention and comparative Example 1) and the results are even worse if a different linker to that of the invention is also used (comparative Example 2).

Thus, a first aspect of the present invention relates to a compound of formula (I) or a pharmaceutically acceptable salt thereof,

(Z)-(L)-P-(W)ₛ-(Y) (I)

wherein:
Z is a radical of the pharmaceutical active ingredient SN38 or a pharmaceutically acceptable salt thereof, wherein the pharmaceutical active ingredient SN38 has formula (II), and wherein Z is attached to a linker L independently by only one of the two hydroxyl groups (a) or (b) of the pharmaceutical active ingredient; 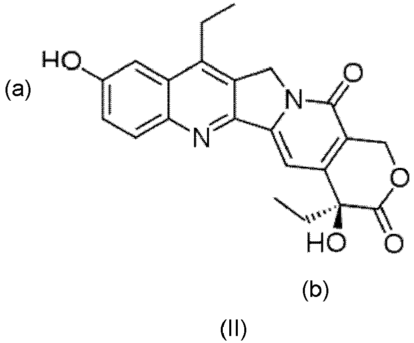
L is a linker which is a biradical composed from 2 to 8 biradicals L' and has the formula: -L'ₐ-(L'_{b})ₙ-L'_{c};
Lₐ' is a biradical selected from the group consisting of: -C(=O)-(CH₂)ᵣ-C(=O)-; -C(=O)-(CH₂)ᵣ-NH-; -C(=O)-(CH₂)ᵣ-S-; -C(=O)-(CH₂)ᵣ-O-; -C(=O)-NH-(CH₂)ᵣ-C(=O)-; -C(=O)-NH-(CH₂)ᵣ-NH-; -C(=O)-NH-(CH₂)ᵣ-S-; -C(=O)-NH-(CH₂)ᵣ-O-; -(CH₂)ᵣ-C(=O)-; -(CH₂)ᵣ-NH-; -(CH₂)ᵣ-S-; -(CH₂)ᵣ-O-; -Si(R₁)(R₂)-(CH₂)ᵣ-NH-; -Si(R₁)(R₂)-(CH₂)ᵣ-C(=O)-; -Si(R₁)(R₂)-(CH₂)rO-; -Si(R₁)(R₂)-(CH₂)ᵣ-S-; -SO₂-(CH₂)ᵣ-NH-; -SO₂-(CH₂)ᵣ-C(=O)-; -SO₂-(CH₂)ᵣ-O-;-SO₂-(CH₂)ᵣ-S-; -P(=O)(OR₁)-O-(CH₂)ᵣ-N_{H}-; -P(=O)(OR₁)-O-(CH₂)ᵣ-C(=O)-; -P(=O)(OR₁)-O-(CH₂)-O-; -P(=O)(OR₁)-O-(CH₂)ᵣ-S-; -CH(OH)-(CH₂)ᵣ-NH-; -CH(OH)-(CH₂)ᵣC(=O)-; -CH(OH)-(CH₂)ᵣ-O-; -CH(OH)-(CH₂)ᵣ-S-; 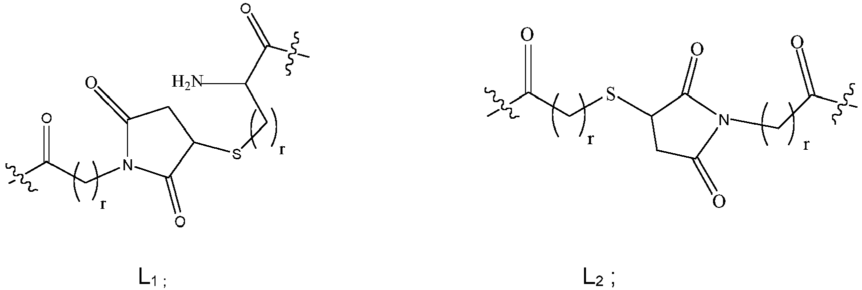 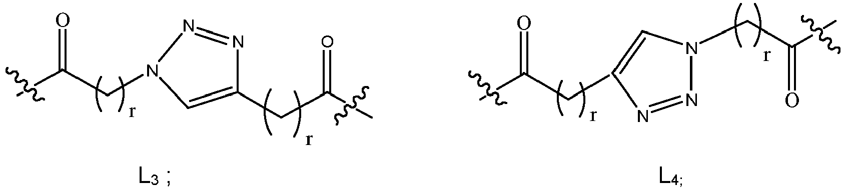 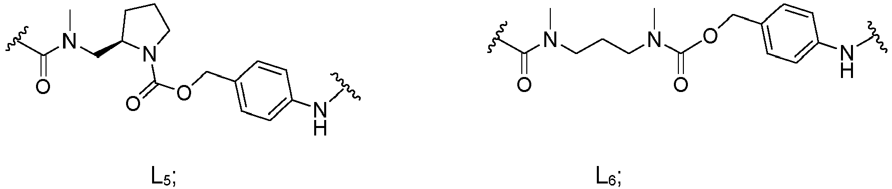 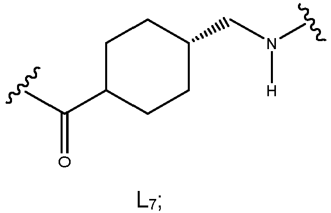 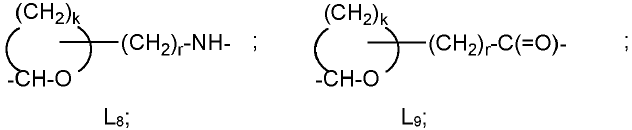 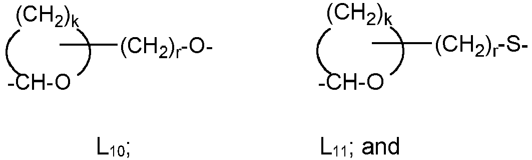
the substituent in any of L₈-L₁₁ is in any position of the cycles; 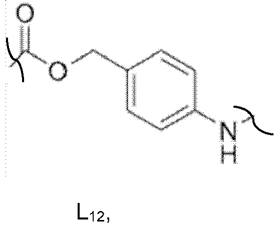
L_{b}' is a biradical independently selected from the group consisting of: -NH-(CH₂)ᵣ-C(=O)-; -C(=O)-(CH₂)ᵣ-C(=O)-; -S-(CH₂)ᵣ-C(=O)-; -O-(CH₂)ᵣ-C(=O)-; -NH-(CH₂)ᵣ; -C(=O)-(CH₂)ᵣ-; -S-(CH₂)ᵣ-; -O-(CH₂)ᵣ-; -NH-CH-((CH₂)ᵣNH₂)-C(=O)-; -S-CH₂-CH(NH₂)-C(=O)-; -(CH₂)ᵣC(=O)-; -(CH₂)ᵣO-; -(CH₂)ᵣ-NH-; -(CH₂)ᵣ-S-; -C(=O)-(CH₂)ᵣ-NH-; -C(=O)-(CH₂)ᵣ-O-; -C(=O)-(CH₂)ᵣ-S-; -NH-(CH₂)ᵣO-; -NH-(CH₂)ᵣ-NH-; -NH-(CH₂)ᵣ-S-; L₁; L₂; L₃; L₄; and combinations thereof;
L_{c}' is a biradical selected from the group consisting of: -NH-(CH₂)ᵣ-C(=O)-; -NH-CH-((CH₂)ᵣ-NH₂)-C(=O)-; -C(=O)-(CH₂)ᵣ-C(=O)-; -S-(CH₂)ᵣ-C(=O)-; -S-CH₂-CH(NH₂)-C(=O)-; -O-(CH₂)ᵣ-C(=O)-; -(CH₂)ᵣ-C(=O)-; L₁, L_{2;} L_{3;} L_{4;} 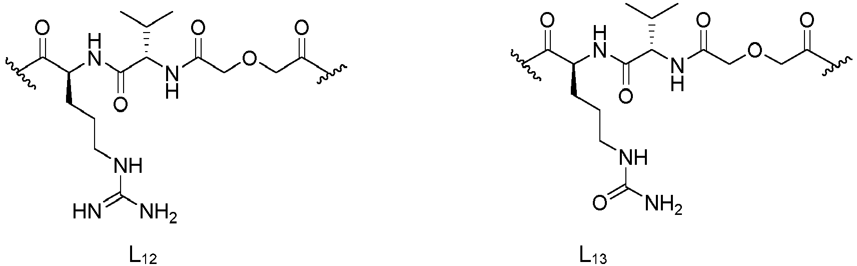 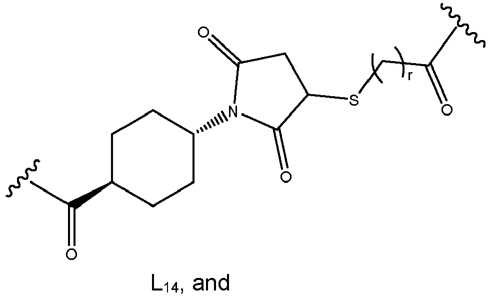 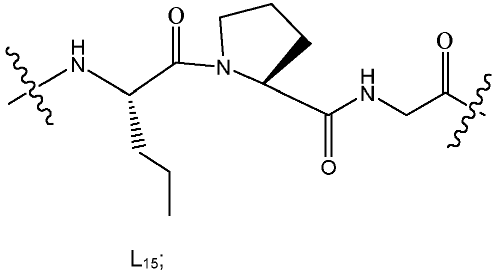
P is a biradical of a peptide selected from the group consisting of: (a) a peptide which comprises the amino acid sequence X₁KAPETALX₂ with an intrapeptide bond between the X₁ and X₂ which is an amide bond; wherein X₁ is selected from the group consisting of Dap (2,3-diaminopropionic acid) and Dab (2,4-diaminobutanoic acid); and X₂ is selected from the group consisting of D (aspartic acid) and E (glutamic acid); i.e.

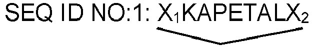
SEQ ID NO:1: X1KAPETALX2

For the amino acid Dap the code Dap an Dpr have been equally used herein;
(b) a peptide having 12-20 amino acids residues in length having at least an intrapeptide bond which is a disulfide or diselenide bond, and comprises an amino acid sequence which is: X₃KAPETALX₄AAA; having at least an intrapeptide disulfide or diselenide bond between X₃ and X₄, wherein X₃ and X₄ are equal and are selected from the group consisting of C (cysteines), Sec (selenocysteines), and Pen (penicillamines); i.e. 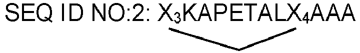
SEQ ID NO:2: X3KAPETALX4AAA
(c) a peptide having 9-11 amino acids residues in length having at least an intrapeptide bond which is a disulfide or diselenide bond and consists of an amino acid sequence selected from the group consisting of X₅KAPETALX₆; X₅KAPETALX₆A; and X₅KAPETALX₆AA having at least an intrapeptide disulfide or diselenide bond between X₅ and X₆; wherein X₅ and X₆ are equal and are selected from the group consisting of C (cysteines), Sec (selenocysteines), and Pen (penicillamines), i.e. 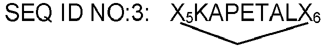
SEQ ID NO:3: X5KAPETALX6
 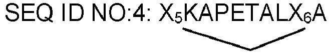
SEQ ID NO:4: X5KAPETALX6A
 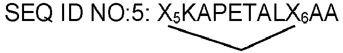
SEQ ID NO:5: X5KAPETALX6AA
(d) a peptide which has 16 amino acid residues and comprises the amino acid sequence X₇NX₈KAPETALX₉AAAX₁₀H with an intrapeptide disulfide or diselenide bond between the X₇ and X₉, and between X₈ and X₁₀; wherein X₇-X₁₀ are independently selected from the group consisting of C (cysteines), Sec (selenocysteines), and Pen (penicillamines); provided that X₇ and X₉ are equal, and X₈-X₁₀ are equal; i.e. 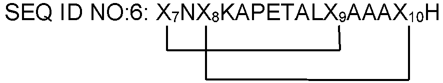
SEQ ID NO:6: X7NX8KAPETALX9AAAX10H
 and (e) peptide which comprises the amino acid sequence X₁KAPETALX₂ wherein X₁ is selected from the group consisting of Dap and Dab; and X₂ is selected from the group consisting of D (aspartic acid) and E (glutamic acid) (SEQ ID NO:7) i.e linear peptide;
W is a biradical selected from the group consisting of -NH-(CH₂)ᵣ₋C(=O)-, and -NH-CH((CH₂)ᵣNH₂)-C(=O)-; Y is a radical is selected from the group consisting of -NH₂, -OH, -OR₃, and -NHR₃;
s is an integer independently selected from 0 to 1; n is an integer from 0 to 6; r is an integer independently selected from 1 to 5; k is an integer from 5 to 8; R₁ and R₂ are independently selected from an (C₁-C₆)-alkyl; and R₃ is a radical selected from the group consisting of (C₁-C₆)-alkyl;
Lₐ' is attached to the radical Z through a bond which is selected from the group consisting of an ester, ether, urethane, silyl ether, sulphonate, phosphate, ketal, hemiketal, carbonate, and carbamate bond, the bond being formed between the C=O, SO₂, Si, P, CH or CH₂ groups on the left side of the draw Lₐ' formulas and one of the hydroxyl groups of the SN38;
when n=0, Lₐ' is attached to the radical L_{c}' through a chemically feasible bond which is selected from the group consisting of amine, amide, ether, thioether, disulfide, ester, and thioester, the bond being formed between the functional groups on the right side of the draw Lₐ' formulas and the functional groups of the left side of the L_{c}' formulas;
when n=1, Lₐ' is attached to the radical L_{b}' through a chemically feasible bond which is selected from the group consisting of amine, amide, ether, thioether, disulfide, ester, and thioester, the bond being formed between the functional groups on the right side of the draw Lₐ' formulas and the functional groups on the left side of the L_{b}' formulas; and L_{b}' is attached to the radical L_{c}' through a chemically feasible bond which is selected from the group consisting of amine, amide, ether, thioether, disulfide, ester, and thioester, the bond being formed between the functional groups on the right side of the draw L_{b}' formulas and the functional groups on the left side of the draw L_{c}' formulas;
when n is higher than 1, L_{b}' are equal or different and are attached among them through a chemically feasible bond selected from the group consisting of amine, amide, ether, thioether, disulfide, ester, and thioester; being one L_{b}' terminal attached to Lₐ' through a chemically feasible bond which is selected from the group consisting of amine, amide, ether, thioether, disulfide, ester, and thioester, the bond being formed between the functional groups on the right side of the draw Lₐ' formulas and the functional groups of the left side of the draw L_{b}' formulas; and being another L_{b}' terminal attached to L_{c}' through a chemically feasible bond which is selected from the group consisting of amine, amide, ether, thioether, disulfide, ester, and thioester, the bond being formed between the functional group on the right side of the draw L_{b}' formulas and the functional group on the left side of the draw L_{c}' formulas;
L_{c}' is attached to the to the biradical P through an amide bond formed with the carbonyl group on the right side of the draw L_{c}' formulas and an amino group of the first amino acid of the peptide sequence P;
when s=0, P is directly attached to Y through an amide, carboxylic acid or ester bond, the bond being formed between the C=O of the C-terminal of the last amino acid of the sequence P, and the radical Y which is -NH₂, -OH, -OR₃, or -NHR_{3;} and
when s=1, P is attached to a radical W through an amide bond formed with a C=O of the C-terminal of the last amino acid of the sequence P, the bond being formed between the functional groups on the left side of the draw W formulas and the functional groups (C=O) of the C-terminal of the last amino acid of the sequence P on the right side of the draw sequence; and W is attached to Y as follows: -C(=O)-NH-(CH₂)ᵣ₋C(=O)-Y, or -C(=O)-NH-CH((CH₂)ᵣNH₂)-C(=O)-Y.

The lines between two amino acids of the sequences above or below represent the intrapeptide bond between the side chains of the two amino acids. In a particular embodiment, the lines between two amino acids of the sequences above or below represent the intrapeptide bond between the side chains of the two amino acids.

A second aspect of the present invention relates to a pharmaceutical composition comprising a therapeutically effective amount of the compound as defined above, together with appropriate amounts of pharmaceutically acceptable carriers or excipients.

A third aspect of the present invention relates to a compound as defined above, for use as a medicament.

A fourth aspect of the present invention relates to a compound as above, for use in the treatment of cancer in a mammal, including a human.

The present disclosure further relates to a compound of formula (I) for use in the treatment of cancer, wherein the compound of formula (I) is for use in combination therapy with a chemotherapeutic agent, and/or in combination with a carboxylesterase inhibitor.

### Brief Description of Drawings

Compound (Ia) is also named as G2B-001 or SN38-linker A-MiniAp4. These names have been equally used herein.Compound (Ib) is also named as G2B-001 lineal. Compound (Ic) is also named as G2B-003. Compound (Id) is also named as G2B-004. Compound (le) is also named as G2B-005.

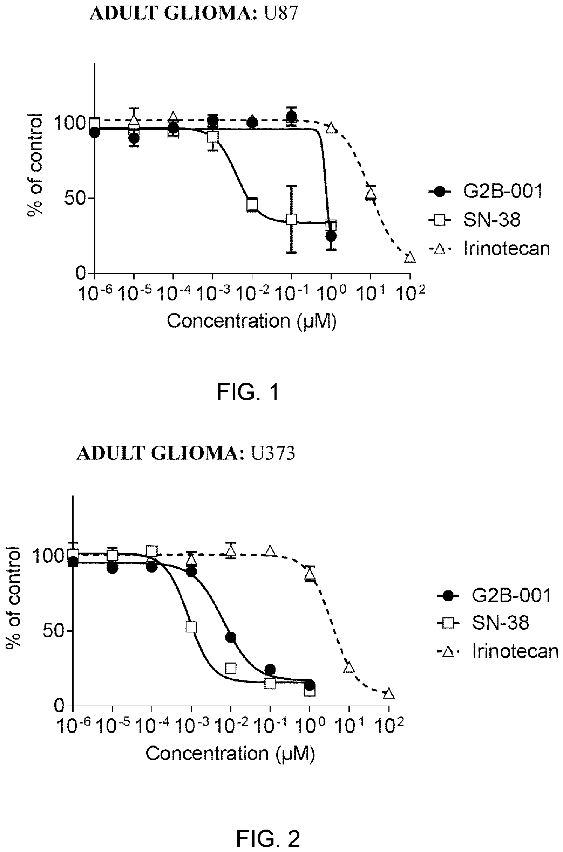

FIG. 1 shows a comparative antiproliferative activity of compound (Ia) also named G2B-001 or SN38-linker A-MiniAp4 herein (example 7) and related compounds SN38 and irinotecan, against cancer cell line of adult gliomas U87. Values are expressed as % of MTS signal of control untreated cells that were considered 100%. Values in dots represent means and SD from three-six replicates at compound concentration. MiniAp4 is DapKAPETALD with an amide intrapeptide bond between Dap and D.
FIG. 2 shows a comparative antiproliferative activity of SN38-linker A-MiniAp4 (Ia) and related compounds SN38 and irinotecan, against cancer cell line of adult gliomas U373. Values are expressed as % of MTS signal of control untreated cells that were considered 100%. Values in dots represent means and SD from three-six replicates at compound concentration.

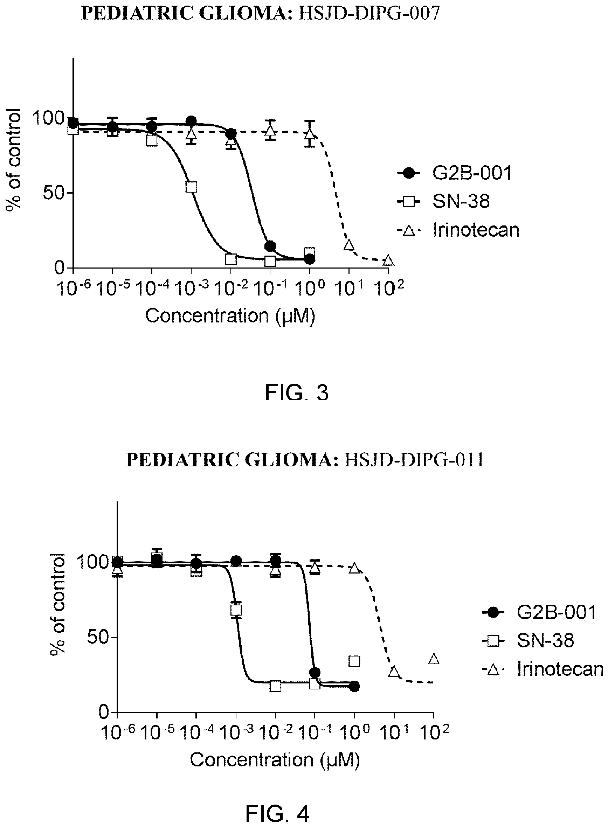

FIG. 3 shows a comparative antiproliferative activity of SN38-linker A-MiniAp4 (Ia) and related compounds SN38 and irinotecan, against DIPG cell model HSJD-DIPG-007. Values are expressed as % of MTS signal of control untreated cells that were considered 100%. Values in dots represent means and SD from three-six replicates at compound concentration.
FIG. 4 shows a comparative antiproliferative activity of SN38-linker A-MiniAp4 (Ia) and related compounds SN38 and irinotecan, against DIPG model HSJD-DIPG-011. Values are expressed as % of MTS signal of control untreated cells that were considered 100%. Values in dots represent means and SD from three-six replicates at compound concentration.

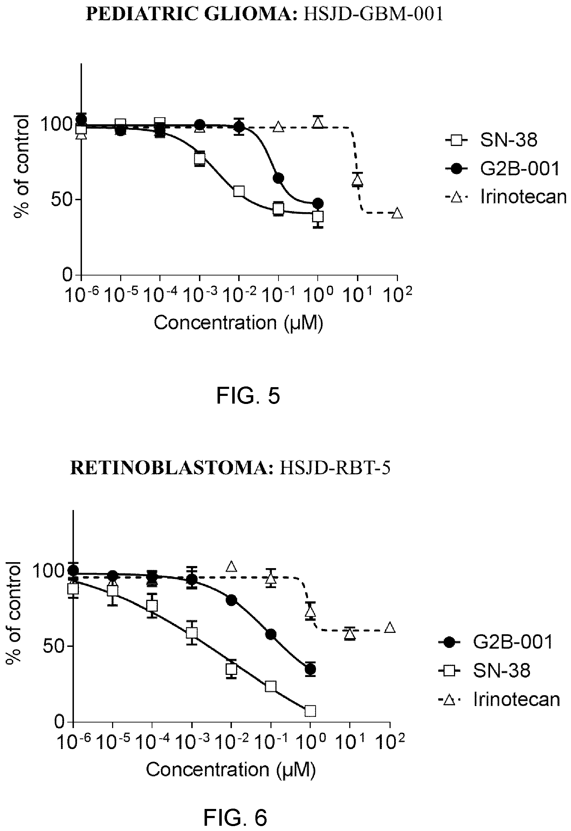

FIG. 5 shows a comparative antiproliferative activity of SN38-linker A-MiniAp4 (Ia) and related compounds SN38 and irinotecan, against pediatric high-grade glioma model HSJD-GBM-001. Values are expressed as % of MTS signal of control untreated cells that were considered 100%. Values in dots represent means and SD from three-six replicates at compound concentration.
FIG. 6 shows a comparative antiproliferative activity of SN38-linker A-MiniAp4 (Ia) and related compounds SN38 and irinotecan, against retinoblastoma cell model HSJD-RBT-5. Values are expressed as % of MTS signal of control untreated cells that were considered 100%. Values in dots represent means and SD from three-six replicates at compound concentration.

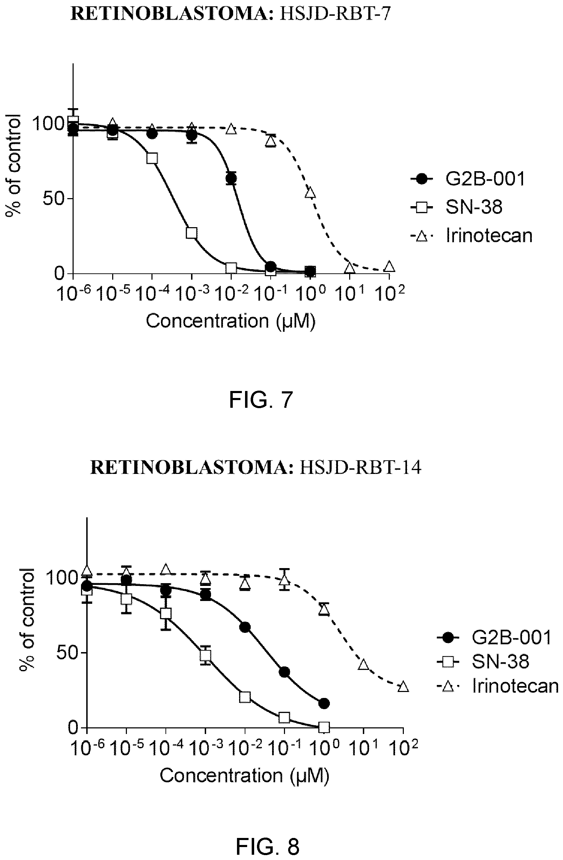

FIG. 7 shows a comparative antiproliferative activity of SN38-linker A-MiniAp4 (Ia) and related compounds SN38 and irinotecan, against retinoblastoma cell model HSJD-RBT-7. Values are expressed as % of MTS signal of control untreated cells that were considered 100%. Values in dots represent means and SD from three-six replicates at compound concentration.
FIG. 8 shows a comparative antiproliferative activity of SN38-linker A-MiniAp4 (Ia) and related compounds SN38 and irinotecan, against retinoblastoma cell model HSJD-RBT-14. Values are expressed as % of MTS signal of control untreated cells that were considered 100%. Values in dots represent means and SD from three-six replicates at compound concentration.

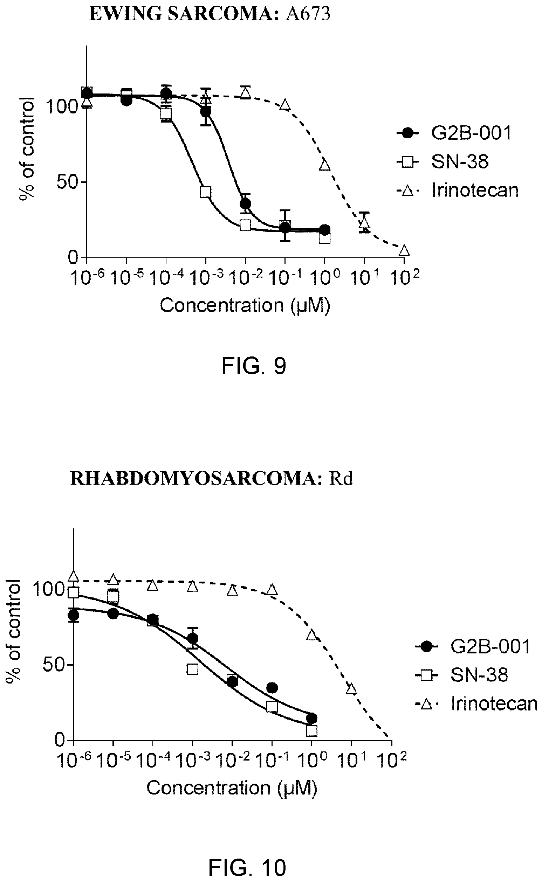

FIG. 9 shows a comparative anti proliferative activity of SN38-linker A-MiniAp4 (Ia) and related compounds SN38 and irinotecan, against Ewing sarcoma cell line A673. Values are expressed as % of MTS signal of control untreated cells that were considered 100%. Values in dots represent means and SD from three-six replicates at compound concentration.
FIG. 10 shows a comparative antiproliferative activity of SN38-linker A-MiniAp4 (Ia) and related compounds SN38 and irinotecan, against rhabdomyosarcoma cell line Rd. Values are expressed as % of MTS signal of control untreated cells that were considered 100%. Values in dots represent means and SD from three-six replicates at compound concentration.

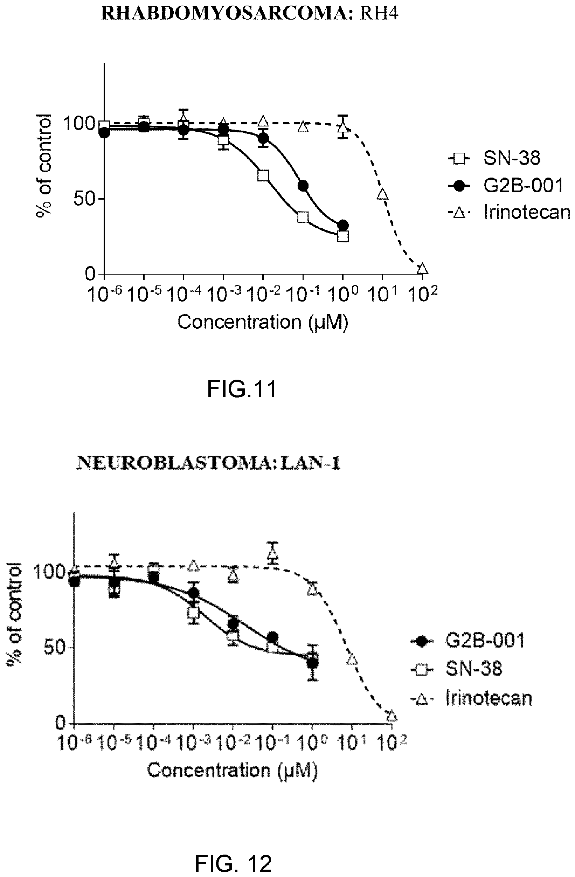

FIG. 11 shows a comparative antiproliferative activity of SN38-linker A-MiniAp4 (Ia) and related compounds SN38 and irinotecan, against rhabdomyosarcoma cell line RH4. Values are expressed as % of MTS signal of control untreated cells that were considered 100%. Values in dots represent means and SD from three-six replicates at compound concentration.
FIG 12 shows a comparative antiproliferative activity of SN38-linker A-MiniAp4 (Ia) and related compounds SN38 and irinotecan, against neuroblastoma cell line LAN-1. Values are expressed as % of MTS signal of control untreated cells that were considered 100%. Values in dots represent means and SD from three-six replicates at compound concentration.

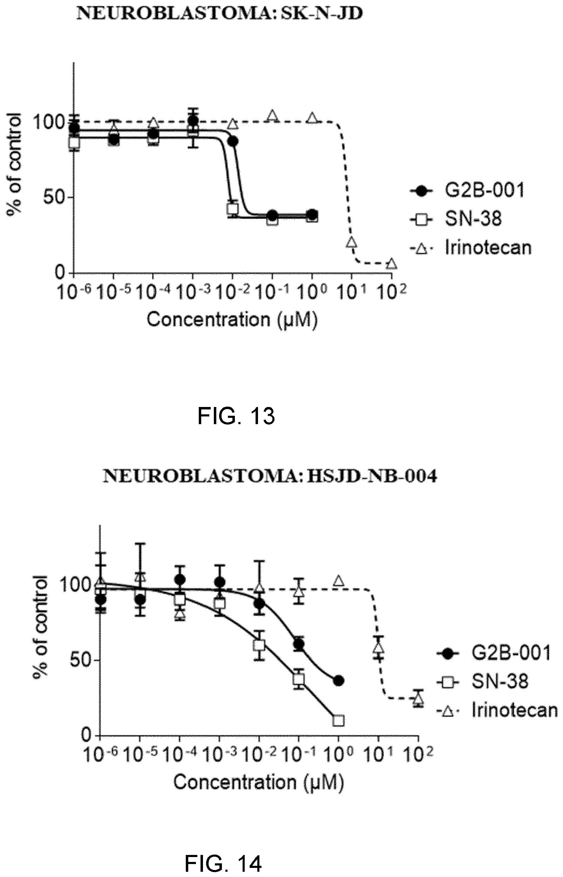

FIG 13 shows a comparative antiproliferative activity of SN38-linker A-MiniAp4 (Ia) and related compounds SN38 and irinotecan, against neuroblastoma cell line Sk-N-JD. Values are expressed as % of MTS signal of control untreated cells that were considered 100%. Values in dots represent means and SD from three-six replicates at compound concentration.
FIG 14 shows a comparative antiproliferative activity of SN38-linker A-MiniAp4 (Ia) and related compounds SN38 and irinotecan, against neuroblastoma cell model HSJD-NB-004. Values are expressed as % of MTS signal of control untreated cells that were considered 100%. Values in dots represent means and SD from three-six replicates at compound concentration.

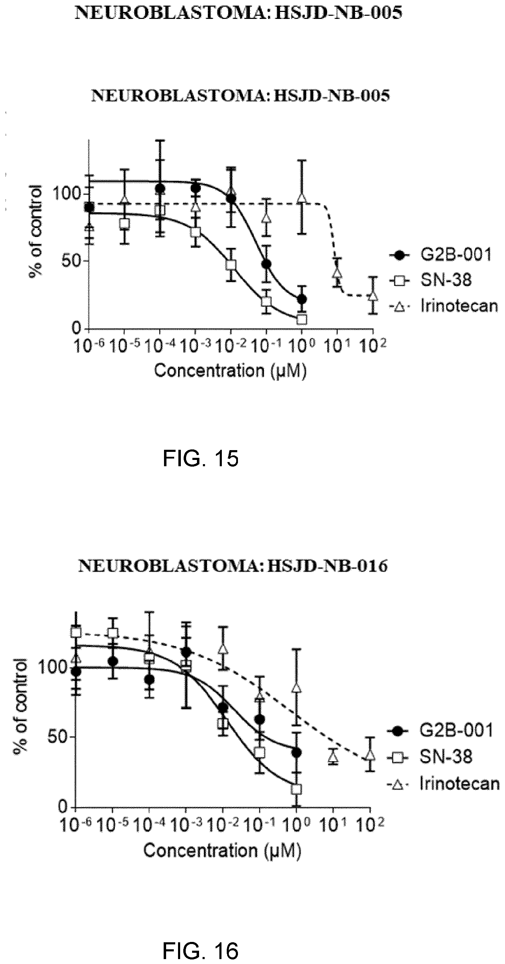

FIG 15 shows a comparative antiproliferative activity of SN38-linker A-MiniAp4 (Ia) and related compounds SN38 and irinotecan, against neuroblastoma cell model HSJD-NB-005. Values are expressed as % of MTS signal of control untreated cells that were considered 100%. Values in dots represent means and SD from three-six replicates at compound concentration.
FIG 16 shows a comparative antiproliferative activity of SN38-linker A-MiniAp4 (Ia) and related compounds SN38 and irinotecan, against neuroblastoma cell model HSJD-NB-016. Values are expressed as % of MTS signal of control untreated cells that were considered 100%. Values in dots represent means and SD from three-six replicates at compound concentration.

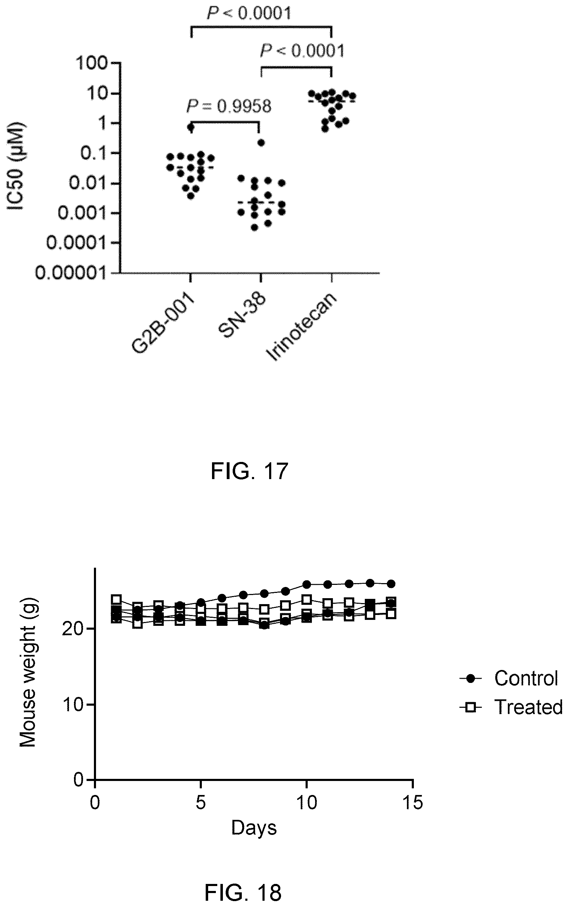

FIG. 17 shows a comparison of SN38-linker A-MiniAp4 (Ia) drug concentration inhibiting 50% of tumor cell proliferation (IC50 values) between cultures. Individual data (dots) are represented. Statistics: Repeated measures (paired) two-way ANOVA, with Tukey's multiple comparisons test.
FIG. 18 shows individual weight curves of 3 mice treated with SN38-linker A-MiniAp4 compound (Ia) at the MTD of 200 mg/kg (Treated), one single i.v. injection, at day 0, with 2 control mice (Controls) injected i.v. with saline at day 0.

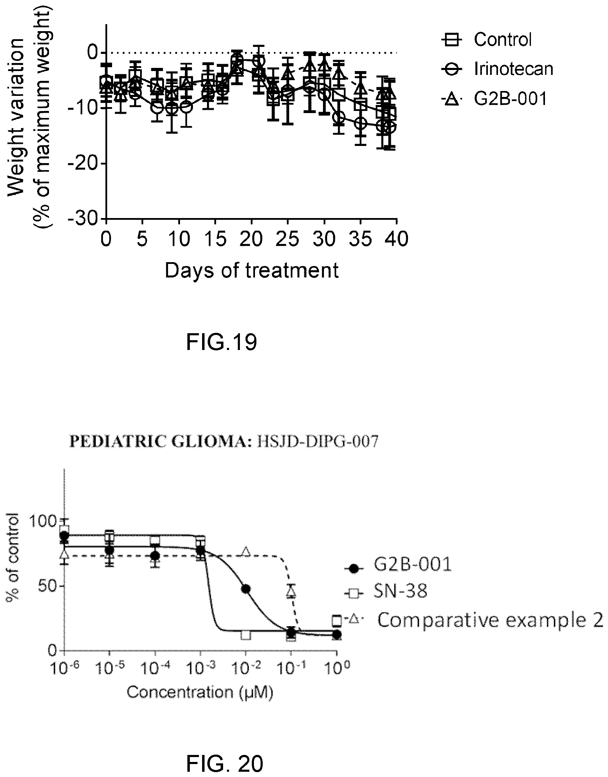

FIG.19 shows the mean weight variation, expressed as the % of weight loss from the maximum individual weight, of mice treated with irinotecan, SN38-linker A-MiniAp4 (Ia), or saline (Control) as detailed in example 10. Mean data (dots) and SD (bars) are represented.
FIG. 20 shows a comparative antiproliferative activity of SN38-linker A-MiniAp4 (Ia), and comparative example 2 (SN38-linker B-THRre) against cell line HSJD-DIPG-007. Values are expressed as % of MTS signal of control untreated cells that were considered 100%. Values in dots represent means and SD from six replicates at compound concentration.

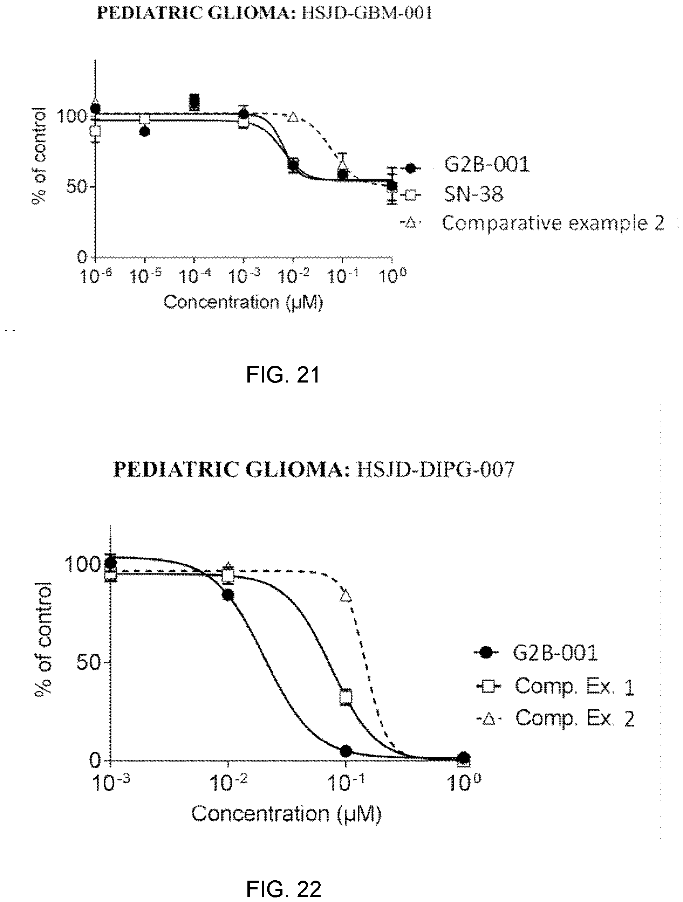

FIG. 21 shows a comparative antiproliferative activity of SN38-linker A-MiniAp4 (Ia), and comparative example 2 (SN38-linker B-THRre) against cell line HSJD-GBM-001. Values are expressed as % of MTS signal of control untreated cells that were considered 100%. Values in dots represent means and SD from six replicates at compound concentration.
FIG. 22 shows a comparative antiproliferative activity of SN38-linker A-MiniAp4 (Ia), comparative example 1 (SN38-linker A-THRre) and comparative example 2 (SN38-linker B-THRre) against cell line HSJD-DIPG-007. Values are expressed as % of MTS signal of control untreated cells that were considered 100%. Values in dots represent means and SD from six replicates at compound concentration.

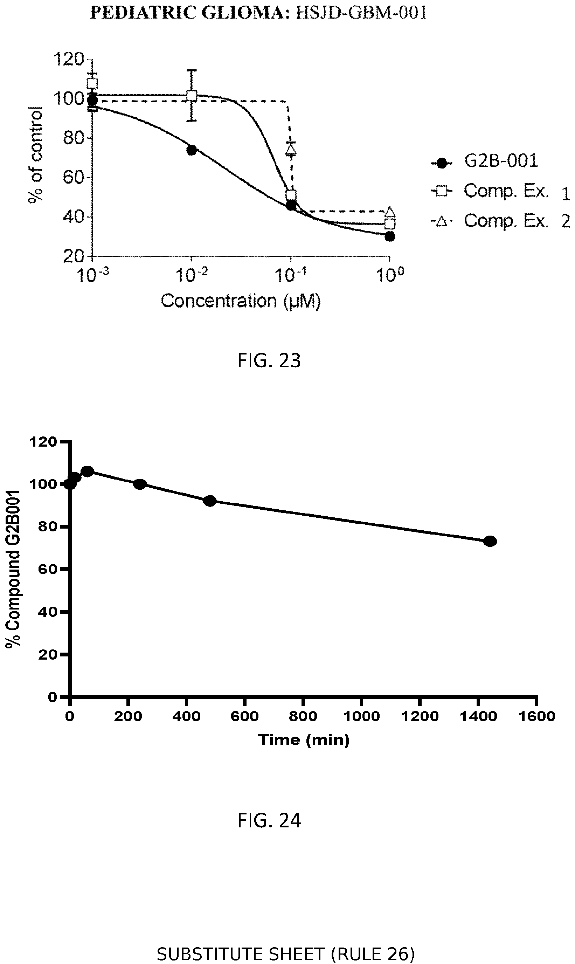

FIG. 23 shows a comparative antiproliferative activity of SN38-linker A-MiniAp4 (Ia), comparative example 1 (SN38-linker A-THRre) and comparative example 2 (SN38-linker B-THRre) against cell line HSJD-GBM-001. Values are expressed as % of MTS signal of control untreated cells that were considered 100%. Values in dots represent means and SD from six replicates at compound concentration.
FIG 24 shows the stability of SN38-Linker A-MiniAp4 (Ia) in human serum at 37°C in vitro versus time.

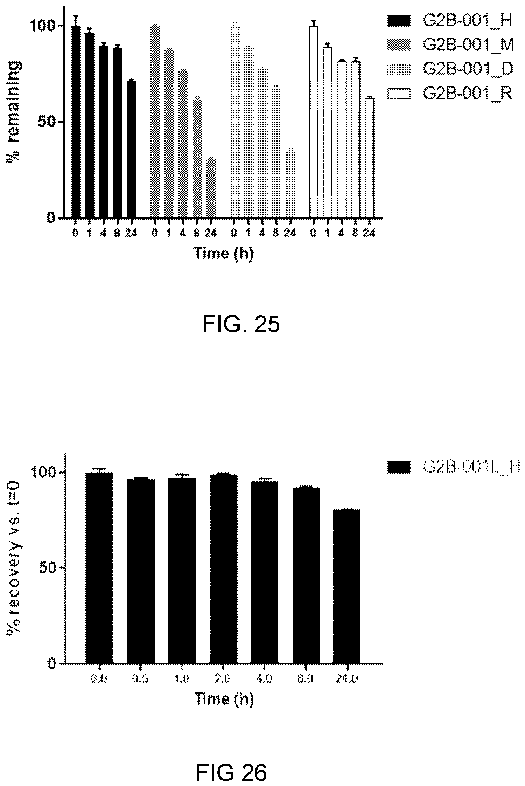

FIG. 25 shows the stability of SN38-Linker A-MiniAp4 (Ia) in rat, mouse, dog and human plasma at 37°C in vitro versus time.
FIG. 26 shows the stability of G2B-001 lineal (Ib) in human serum at 37°C in vitro versus time.

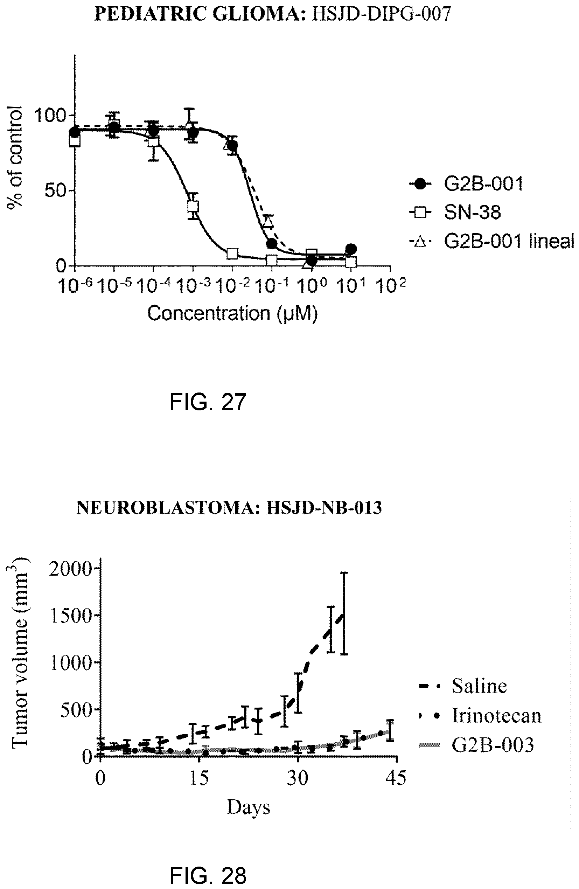

FIG. 27 shows a comparative anti proliferative activity of G2B-001 lineal (Ib), G2B-001 (Ia) and related compound SN38, against DIPG cell model HSJD-DIPG-007. Values are expressed as % of MTS signal of control untreated cells that were considered 100%. Values in dots represent means and SD from three-six replicates at compound concentration.
FIG. 28 shows subcutaneous neuroblastoma tumor volumes (mean and SD of two tumors) in three mice treated with either irinotecan (black dots), G2B-003 (Ic) (solid line) or saline control (dashed line). Tumor model was patient-derived xenograft named HSJD-NB-013.

### Detailed description of the invention

Unless otherwise stated, the amino acids cited herein are L-amino acids. The 1-letter code and the 3-letter code have been used indistinctly. For the following amino acids the following abbreviations have been used: diaminopropionic acid (Dap), Diaminobutiric (Dab), Selenocystein (Sec) and Penicillamine (Pen). In the context of the present invention penicillamine only embraces D-penicillamine.

The compounds of the present invention mentioned above may be in the form of pharmaceutically acceptable salts. The term "pharmaceutically acceptable salts" used herein encompasses any salt formed from pharmaceutically acceptable non-toxic acids or bases including inorganic or organic acids or bases. There is no limitation regarding the salts, except that if used for therapeutic purposes, they must be pharmaceutically acceptable.

As some of the compounds of formula (I) are basic compounds, salts may be prepared from pharmaceutically acceptable non-toxic acids, including inorganic and organic acids. Such acids include for instance chlorhidric, acetic, benzenesulfonic, benzoic, camphorsulfonic, citric, ethansulfonic, fumaric, gluconic, glutamic, hydrobromic, hydrochloric, lactic, maleic, malic, mandelic, methanesulfonic, phosphoric, succinic, sulfuric, tartaric, p-toluensulfonic acid, and the like.

In a particular embodiment, the compounds of formula (I) are those where Z is attached to a linker L by the hydroxyl group (b) of the pharmaceutical active ingredient.

In another particular embodiment, the compounds of formula (I) are those where Z is attached to a linker L by the hydroxyl group (a) of the pharmaceutical active ingredient.

In another particular embodiment, the compounds of formula (I) are those where P is a biradical of a peptide selected from the group consisting of: (a) a peptide which comprises the amino acid sequence DapKAPETALD with an intrapeptide bond between the Dap and D which is an amide bond, i.e. 
SEQ ID NO:8: DapKAPETALD
NHCO
(b) a peptide having 9-20 amino acids residues in length having at least an intrapeptide bond which is a disulfide bond, and comprises an amino acid sequence which is: CKAPETALCAAA; having at least an intrapeptide disulfide bond between cysteines 1 and 9, i.e. 
SEQ ID NO:9: CKAPETALCAAA
S-S
(c) a peptide having 9-11 amino acids residues in length having at least an intrapeptide bond which is a disulfide bond and consists of an amino acid sequence selected from the group consisting of CKAPETALC; CKAPETALCA; and CKAPETALCAA having at least an intrapeptide disulfide bond between cysteines 1 and 9, i.e. 
SEQ ID NO:10: CKAPETALC
S-S
 
SEQ ID NO:11 CKAPETALCA
S-S
 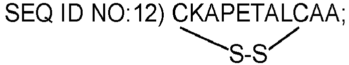
SEQ ID NO:12) CKAPETALCAA;
S-S
 and
(d) a peptide which has 16 amino acid residues and comprises the amino acid sequence CNCKAPETALCAAACH with an intrapeptide disulfide bond between the first and third cysteine which are cysteines 1 and 11, and between the second and the fourth cysteine which are cysteine 3 and 15, i.e.

SEQ ID NO:13: CNCKAPETALCAAACH
S-S
S-S

In sequences SEQ ID NO:8 to SEQ ID NO:13 in which specific amino acids have been selected for X₁-X₁₀ the specific intrapeptide bond has been drawn in the sequence.

In another particular embodiment, the at least one intrapeptide bond in the peptides of the present invention is one intrapeptide bond. In another particular embodiment, the at least one intrapeptide bond in the peptides of the present invention refers to two intrapeptide bonds.

In another particular embodiment, the compounds of formula (I) are those where P is a biradical of a peptide DapKAPETALD (SEQ ID NO:14), i.e linear peptide.

In a particular embodiment, in combination of any of the particular embodiments above or below, the compounds of formula (I) are those where P is a biradical of a peptide selected from the group consisting of: (a) a peptide which comprises the amino acid sequence DapKAPETALD with an intrapeptide bond between the Dap and D which is an amide bond (also named MiniAp4) (SEQ ID NO:8); and (b) CKAPETALC having at least an intrapeptide disulfide bond between cysteines in position 1 and 9 (SEQ ID NO:10).

In another particular embodiment, in combination of any of the particular embodiments above or below, the compounds of formula (I) are those where P is a biradical of the peptide DapKAPETALD with an intrapeptide bond between the Dap and D which is an amide bond (SEQ ID NO:8).

In another particular embodiment, in combination of any of the particular embodiments above or below, the compounds of formula (I) are those where Lₐ' is a biradical selected from the group consisting of: -C(=O)-(CH₂)ᵣ-C(=O)-, -C(=O)-(CH₂)ᵣ-NH-, -C(=O)-(CH₂)ᵣ-S-, -C(=O)-(CH_{Z})ᵣ-O-; -C(=O)-NH-(CH₂)ᵣC(=O)-; L₁, L₂, L₃, L₄, L₅, L₆, L₇, and L₁₂.

In another particular embodiment, in combination of any of the particular embodiments above or below, the compounds of formula (I) are those where Lₐ' is a biradical selected from the group consisting of: -C(=O)-(CH₂)ᵣ-C(=O)-, -C(=O)-(CH₂)ᵣ-NH-, -C(=O)-(CH₂)ᵣ-S-, -C(=O)-(CH₂)ᵣO-; ; L₁, L₂, L₃, L₄, L₅, L₆, and L₇,.

In another particular embodiment, in combination of any of the particular embodiments above or below, the compounds of formula (I) are those where L is a linker which is a biradical composed from 3 to 8 biradicals, and n is an integer from 1 to 6. In another particular embodiment, in combination of any of the particular embodiments above or below, the compounds of formula (I) are those where L is a linker which is a biradical composed from 5 to 8 biradicals. In another particular embodiment, in combination of any of the particular embodiments above or below, the compounds of formula (I) are those where L is a linker which is a biradical composed from 6 to 8 biradicals. In another particular embodiment, in combination of any of the particular embodiments above or below, the compounds of formula (I) are those where L is a linker which is a biradical composed from 6 to 7 biradicals.

In another particular embodiment, in combination of any of the particular embodiments above or below, the compounds of formula (I) are those where L is a linker which is a biradical composed from 6 biradicals.

In another particular embodiment, in combination of any of the particular embodiments above or below, the compounds of formula (I) are those where Lₐ' is L₃ and L_{c}' is -C(=O)-(CH₂)ᵣ-C(=O)-. In another more particular embodiment, the compounds of formula (I) are those where Lₐ' is L₃; L_{b}' is selected from the group consisting of -NH-(CH₂)ᵣ-O-, -(CH₂)ᵣ-O-; and -(CH₂)ᵣ-NH- and combinations thereof; L_{c}' is -C(=O)-(CH₂)ᵣ-C(=O)-.

In another particular embodiment, in combination of any of the particular embodiments above or below, the compounds of formula (I) are those where L is a linker which is a biradical composed from 2 biradicals, and n=0.

In another particular embodiment, in combination of any of the particular embodiments above or below, the compounds of formula (I) are those where Lₐ' is L₁₂ and L_{c}' is L₁₃.

In another particular embodiment, in combination of any of the particular embodiments above or below, the compounds of formula (I) are those where Lₐ' is -C(=O)-NH-(CH₂)ᵣ-C(=O)- and L_{c}' is L₁₅.

In another particular embodiment, in combination of any of the particular embodiments above or below, the compounds of formula (I) are those where Lₐ' is attached to the radical Z through a bond which is an ester bond formed with the C=O group on the left side of the draw Lₐ' formulas, and to the radical L_{b}' through a chemically feasible bond which is selected from the group consisting of amine, amide, ether, thioether, disulfide, ester, and thioester, formed with the functional groups on the right side of the draw formulas.

In another particular embodiment, in combination of any of the particular embodiments above or below, the compounds of formula (I) are those where Lₐ' is attached to the radical Z through a bond which is an ester bond formed with the C=O group on the left side of the draw Lₐ' formulas, and to the radical L_{b}' through a chemically feasible bond which is amide bond, formed with the functional groups on the right side of the draw formulas.

In another particular embodiment, in combination of any of the particular embodiments above or below, the compounds of formula (I) are those where Lₐ' is attached to the radical Z through a bond which is a carbonate or a carbamate bond formed with the C=O group on the left side of the draw Lₐ' formulas, and to the radical L_{b}' through a chemically feasible bond which is amide bond (NH-CO or CO-NH), formed with the functional groups on the right side of the draw formulas.

In another particular embodiment, in combination of any of the particular embodiments above or below, the compounds of formula (I) are those where L_{b}' forms the chemically feasible bond with the radical Lₐ' with the functional groups on the left side of the L_{b}' drawn formulas; and L_{b}' is attached to the radical L_{c}' through a chemically feasible bond which is selected from the group consisting of amine, amide, ether, thioether, disulfide, ester, and thioester, formed with the functional groups on the right side of the L_{b}' drawn formulas; wherein when n is higher than 1, L_{b}' are equal or different and are attached among them through a chemically feasible bond selected from the group consisting of amine, amide, ether, thioether, disulfide ester, and thioester; being one L_{b}' terminal attached to Lₐ' and the other L_{b}' terminal attached to L_{c}'.

In another particular embodiment, in combination of any of the particular embodiments above or below, the compounds of formula (I) are those where L_{c}' is attached to the to the biradical P through an amide bond formed with the carbonyl group on the right side of the drawn L_{c}' formulas and an amino group of the first amino acid of the peptide sequence P, and to the radical L_{b}' through a chemically feasible bond which is selected from the group consisting of amine, amide, ether, thioether, disulfide, ester, and thioester, formed with the functional groups on the left side of the draw formulas.

In another particular embodiment, the compound of formula (I) is compound of formula (Ia) below or a pharmaceutically acceptable salt thereof, where in formula (I) P is MiniAp4=DapKAPETALD with an intrapeptide bond between Dap and D, Y= CONH₂, W=0, the linker is linker A below, and the SN38 is attached to the linker by the hydroxyl (b).

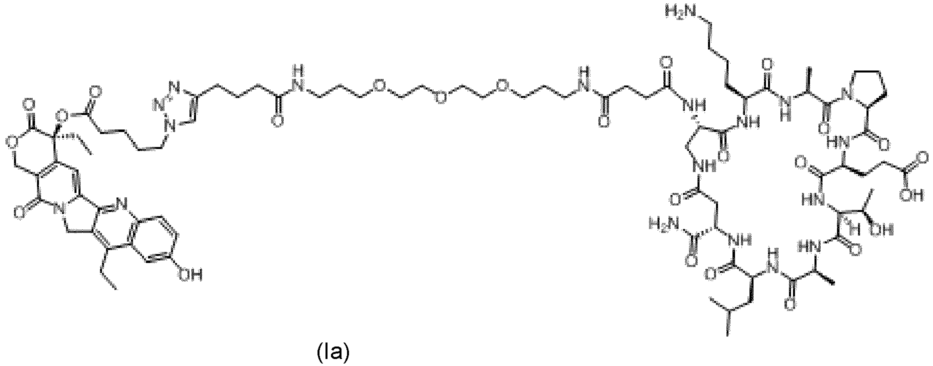

This compound is also named SN38-linker A-MiniAp4;
In another particular embodiment, the compound of formula (I) is compound of formula (Ib) below or a pharmaceutically acceptable salt thereof, where in formula (I) P is MiniAp4=DapKAPETALD lineal without the intrapeptide bond between Dap and D, Y= CONH₂, W=0, the linker is linker A below, and the SN38 is attached to the linker by the hydroxyl (b).

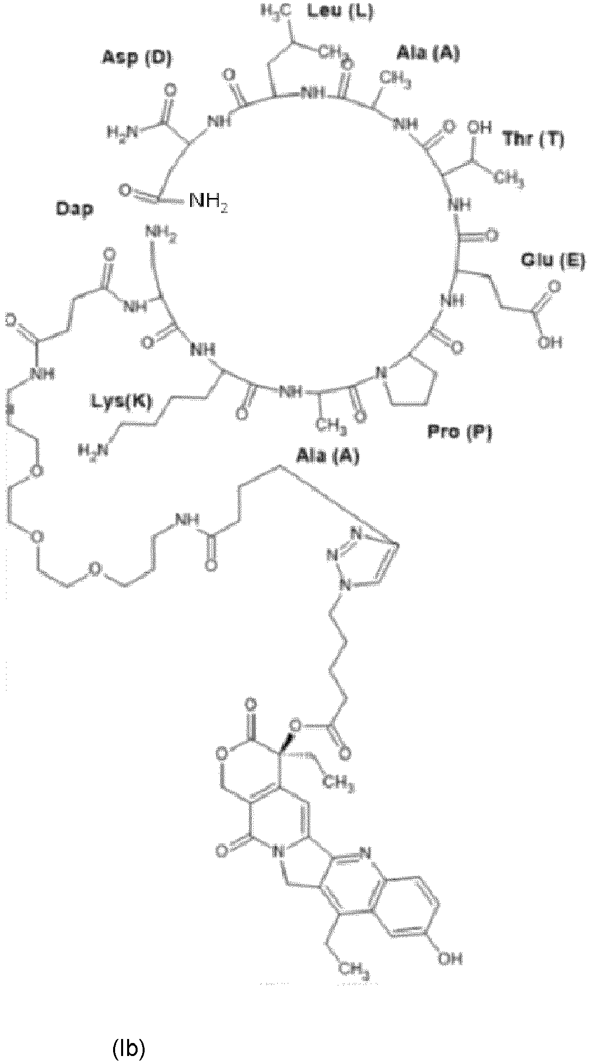

This compound is also named herein G2B-001 lineal.

In another particular embodiment, the compound of formula (I) is compound of formula (Ic) below or a pharmaceutically acceptable salt thereof, where in formula (I) P is MiniAp4=DapKAPETALD with an intrapeptide bond between Dap and D, Y= CONH₂, W=0, the linker is linker A below, and the SN38 is attached to the linker by the hydroxyl (a).

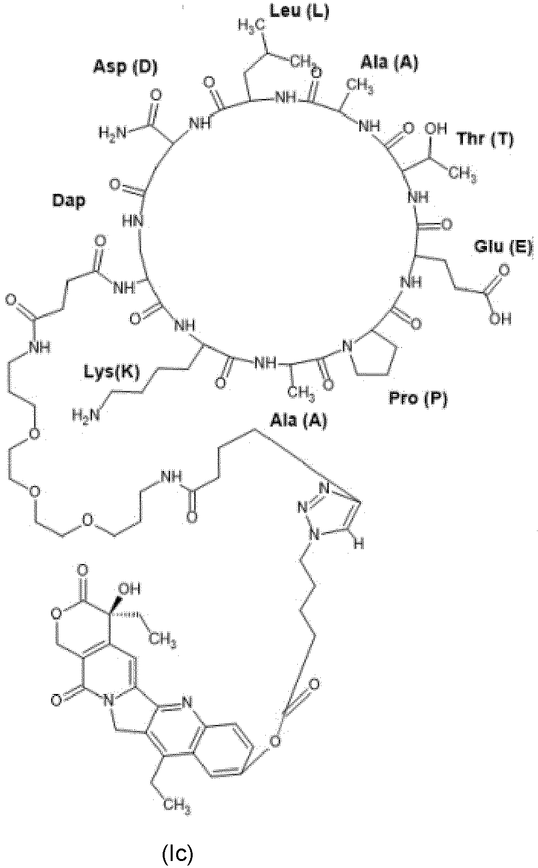

This compound is also named herein G2B-003.

Linker A is formed by:
La': L₃ with r= 4 on the right and 3 on the left: 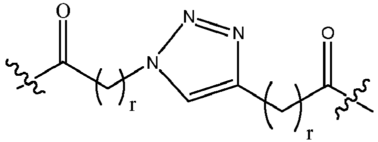
Lb': 1 unit of biradical -NH-(CH₂)₃-O-, 2 units of biradical -(CH₂)₂-O- and 1 unit of biradical -(CH₂)₃-NH-; and Lc': -C(=O)-(CH₂)₂-C(=O)-; and the biradicals are connected as in the drawing below corresponding to linker A:

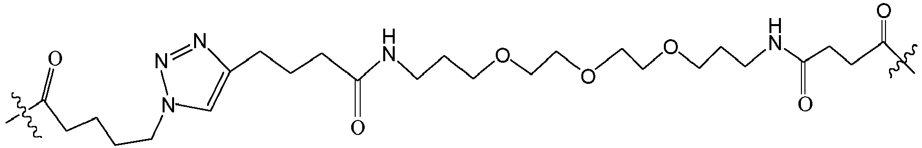

In another particular embodiment, the compound of formula (I) is compound of formula (Id) below or a pharmaceutically acceptable salt thereof, where in formula (I) P is MiniAp4=DapKAPETALD with an intrapeptide bond between Dap and D, Y= CONH₂, W=0, the linker is linker C below, and the SN38 is attached to the linker by the hydroxyl (a).

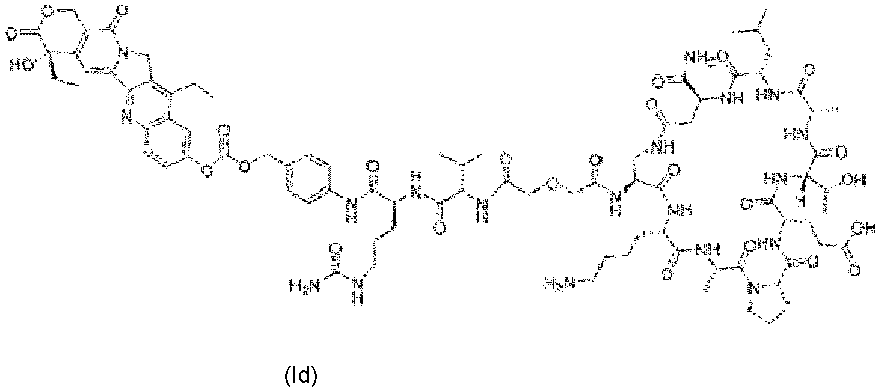

This compound is also named herein G2B-004. Linker C is formed by La': L₁₂ and Lc': L₁₃, and n=0.

In another particular embodiment, the compound of formula (I) is compound of formula (le) below or a pharmaceutically acceptable salt thereof, where in formula (I) P is MiniAp4=DapKAPETALD with an intrapeptide bond between Dap and D, Y= CONH₂, W=0, the linker is linker D below, and the SN38 is attached to the linker by the hydroxyl (a).

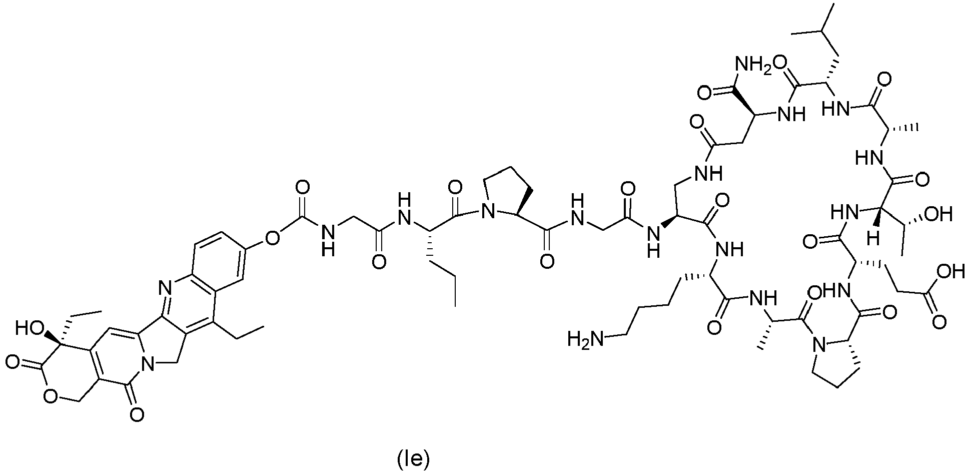

This compound is also named herein G2B-005. Linker D is formed by La': -C(=O)-NH-(CH₂)ᵣ-C(=O)- with r= 1, Lb': L₁₅ and n=0.

The compounds of formula (I) can be generated wholly or partly by chemical synthesis. The amino acids required for the preparation of compounds of formula (I) are commercially available. The compounds of formula (I) can be prepared easily, for example by synthesis in liquid-phase or, preferably, by solid-phase peptide synthesis, for which there are a number of procedures published (see M. Amblard, et al., "Methods and protocols of modern solid-phase peptide synthesis. Molecular Biotechnology 2006, Vol. 33, p. 239-254). The compounds of formula (I) can also be prepared by any combination of liquid-phase synthesis and/or solid-phase synthesis. For example, by synthesizing the body of the peptide P through solid-phase synthesis and, subsequently removing protecting groups in solution. The binding of SN-38 to the linker L and peptide P can be performed in solid-phase or in solution. The construction of the linker L can also be prepared by any combination of liquid-phase synthesis and/or solid-phase synthesis.

The peptides of the invention can also be obtained by generation of a DNA template and subcloning into an expression vector (see J.H. Lee et al. Eur. J. 30 Biochem. 2001. Vol. 268. Pp. 2004-2012).

Compound SN38-linker A-MiniAp4 of formula (Ia) can be prepared by a process comprising reacting a compound of formula (III) with a compound of formula (IV) as described above to yield a compound of formula (Ia)

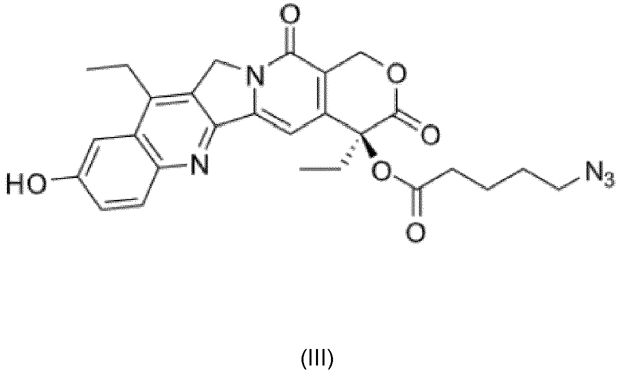

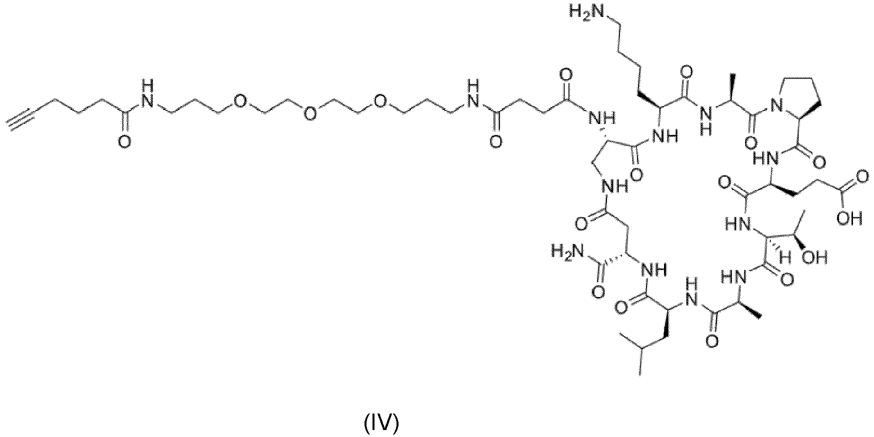

The preparation of pharmaceutically acceptable salts of the compounds of formula (I) can be carried out by methods known in the art. For instance, they can be prepared from the parent compound, which contains a basic or acidic moiety, by conventional chemical methods. Generally, such salts are, for example, prepared by reacting the free acid or base forms of these compounds with a stoichiometric amount of the appropriate pharmaceutically acceptable base or acid in water or in an organic solvent or in a mixture of them.

It is also part of the invention the pharmaceutical composition comprising a therapeutically effective amount of the compound of formula (I) as defined above, together with appropriate amounts of pharmaceutically acceptable carriers or excipients. The term "therapeutically effective amount" as used herein, refers to the amount of a compound that, when administered, is enough to prevent development of, or alleviate to some extent, one or more symptoms of the disease which is addressed. The particular dose of compound administered according to this invention will of course be determined by the particular circumstances surrounding the case, including the compound administered, the via of administration, the particular condition being treated, and similar considerations.

The term "pharmaceutical composition" refers to a mixture of a compound described herein with other chemical components, such as diluents or carriers. The pharmaceutical composition facilitates administration of the compound to an organism. The terms "pharmaceutically acceptable excipients or carriers" refers to pharmaceutically acceptable material, composition or vehicle. Each component must be pharmaceutically acceptable in the sense of being compatible with the other ingredients of the pharmaceutical composition. It must be also suitable for use in contact with tissues or organs of humans and animals without excessive toxicity, irritation, allergic response, immunogenicity, or other problems or complications commensurate with a benefit/risk ratio.

The compositions of the present invention may be administered in parenteral form suitable for injection, infusion or implantation into the body.

An important feature of the compounds of the present invention is their bioactivity inhibiting cell growth of the tested tumor cell lines. As it is illustrated in the Examples, the compounds of the present invention show antitumoral properties in several cancer cell lines.

As already explained, the comparative data provided in the experimental section show that, when the SN38 is conjugated through the same linker (linker A) to another peptide (THRre) of formula below part/all of the antitumoral activity is lost and the results are even worse if a different linker to that of the invention is also used (linker B). Linker B is -C(=O)-(CH₂)₂-C(=O)-. The THRre has the following formula: H-D-Pro-D-Trp-D-Val-D-Pro-D-Ser-D-Trp-D-Met-D-Pro-D-Pro-D-Arg-D-His-D-Thr-CONH₂. However, the exemplified compound of of the present invention unexpectedly maintains antitumoral activity against several cell lines from brain and extracranial cancers, similar to SN38, being much higher than irinotecan with good stability in human serum *in vitro.*

Thus, it is part of the invention the compounds of formula (I) or their pharmaceutically acceptable salts as defined above, for use as a medicament.

It is also part of the invention, the compounds of formula (I) or their pharmaceutically acceptable salts as defined above, for use in the treatment of cancer in a mammal, including a human as they are active in all the types of cancer where have been tested. This aspect can also be formulated as the use of compounds of formula (I) or their pharmaceutical acceptable salts as defined above for the preparation of a medicament for the treatment and/or prevention of a cancer in a mammal, including a human. The invention also relates to a method of treatment of a mammal, including a human, suffering from or being susceptible of suffering from cancer, said method comprising the administration to said patient of a therapeutically effective amount of a compound of formula (I) or their pharmaceutical acceptable salts as defined above, together with pharmaceutically acceptable excipients or carriers.

In a particular embodiment, the compounds of formula (I) are for use as defined above, where the cancer treatment comprises the treatment of a tumor selected from the group consisting of extracranial solid tumors, eye tumors and CNS tumors. In another particular embodiment, the compounds are for use as defined above, where the cancer is selected from the group consisting of adult glioma, pediatric gliomas, retinoblastoma, Ewing sarcoma, DIPG, neuroblastoma and rhabdomyosarcoma. In another particular embodiment, the compounds are for use as defined above, where the pediatric gliomas are selected from the group consisting of diffuse intrinsic pontine glioma (*DIPG*), and pediatric high-grade glioma. In another particular embodiment, the compounds are for use as defined above, where the cancer is diffuse intrinsic pontine glioma. In another particular embodiment, the compounds are for use as defined above, where the cancer is retinoblastoma. In another particular embodiment, the compounds are for use as defined above, where the cancer is Ewing Sarcoma. In another particular embodiment, the compounds are for use as defined above, where the cancer is neuroblastoma. In another particular embodiment, the compounds are for use as defined above, where the cancer is rhabdomyosarocma. In another particular embodiment, the compounds are for use as defined above, where the cancer is adult glioma.

In another particular embodiment, the compounds are for use as defined above, where the compounds are active against a cancer cell line selected from the group consisting of: A673, HSJD-DIPG-007, HSJD-DIPG-011, HSJD-GBM-001, Rd, RH4, HSJD-RBT-5, HSJD-RBT-7, HSJD-RBT-14, U373, U87, LAN-1, SK-N-JD, HSJD-NB-004, HSJD-NB-005, HSJD-NB-013, and HSJD-NB-016.

In another particular embodiment, the compounds are for use as defined above, wherein the compound of formula (I) is administered by intravenous bolus and/or intravenous infusion.

Compounds of the present invention can be used in the same manner as other known chemotherapeutic agents, i.e. in combination with other treatments, either simultaneously or sequentially, depending on the condition to be treated. They may be used alone or in combination with other suitable bioactive compounds. Thus, compounds of formula (I) of the present invention are for use in the treatment of cancer in a mammal, including a human in combination therapy with a chemotherapeutic agent. Also, compounds of formula (I) of the present invention are for use in the treatment of cancer in a mammal, including a human in combination with an ester stabilizer. Suitable ester stabilizers are carboxylesterase inhibitors. Examples of carboxylesterases inhibitors are found in M. J. Hatfield and P. M. Potter, Exp. Opin. Ther. Pat. 2011, vol. 21(8), pp. 1159-1171.

In a particular disclosure, the compound of formula (I) is to be administered in combination with a carboxylesterase inhibitor. In another particular disclosure, the compound of formula (I) is administered simultaneously with the carboxylesterase inhibitor. In another particular disclosure, the compound of formula (I) is administered separately, in any order, within a therapeutically effective interval.

In a particular disclosure, the compound of formula (I) is to be administered in combination with another chemotherapeutic agent. In another particular disclosure, the compound of formula (I) is administered simultaneously with another chemotherapeutic agent. In another particular disclosure, the compound of formula (I) is administered separately, in any order, within a therapeutically effective interval.

In another particular disclosure, the compound of formula (I) is to be administered in combination with another chemotherapeutic agent and with a carboxylesterase inhibitor. In another particular disclosure, the compound of formula (I) is administered simultaneously with another chemotherapeutic agent and with carboxylesterase inhibitor. In another particular disclosure, the compound of formula (I) is administered separately, in any order, within a therapeutically effective interval with another chemotherapeutic agent and with carboxyl esterase inhibitor.

Throughout the description and claims the word "comprise" and variations of the word, are not intended to exclude other technical features, additives, components, or steps. Furthermore, the word "comprise" encompasses the case of "consisting of". Additional objects, advantages of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

Protected amino acids, handles and resins were supplied by: Luxembourg Industries (Tel-Aviv, Israel), Neosystem (Strasbourg, France), CalbiochemNovabiochem AG (Laufelfingen, Switzerland), Bachem AG (Bubendorf, Switzerland) or Iris Biotech (Marktredwitz, Germany). Other reagents and solvents used are summarized in **Table 1**. **Table 1** Commercials suppliers and reagents used. DCM passed through an Al₂O₃ column. DMF is stored on molecular sieves 4Å and nitrogen is bubbled in order to eliminate volatile agents.

| Commercial supplier | | Reagents and solvents |
|---|---|---|
| Albatros Chem. Inc. | | *N*-Hydroxybenzotriazole (HOBt) |
| Aldrich | | Piperidine, piperazine, pyridine, 3-(4,5-dimethyl-2-thiazolyl)-2,5 -diphenyl-2H-tetrazolium bromide (MTT), 3-(4,5-dimethylthiazol-2-yl)-5-(3-carboxymethoxyphenyl)-2-(4-sulfophenyl)-2H-tetrazolium (MTS), |
| | | tetrakis(triphenylphosphine)palladium(0), phenyl silane, sodium diethyldithiocarbamate, |
| | | triisopropylsilane (TIS), 1,2- ethanedithiol (EDT) diisopropylcarbodiimide (DIC), |
| | | diisopropylethylamine (DIEA), O-(benzotriazol-1-yl)-*N,N,N',N'*-tetramethyluronium tetrafluoroborate, tris(3-hydroxypropyltriazolylmethyl)amine (THPTA), NaHCO₃, brine, |
| | | *N*-(3-dimethylaminopropyl)-N'-ethylcarbodiimide hydrochloride, mono-tert-butyl succinate, DBU, sodium ascorbate, 5-hexynoic acid, |
| | | Fmoc-Asp(OAI)-OH, Fmoc-Dap(Alloc)- OH, HCl, MgSO₄ |
| Applied GL Biochem Shangai | | 1-Hydroxy-7-azabenzotriazole (HOAt) |
| CEM | | Rink amide Protide resin, CI-TCP(CI) ProTide resin |
| Envigo | | Athymic nude mice |
| | Carbosynth | SN38 |
| | | |
| | IRIS Biotech | All aminoacids except otherwise specified, 5-azido-pentanoic acid (N₃-pen-OH), Fmoc-TTDS-OH, Di-*tert-*butyl dicarbonate, |
| | Kalichem | Trifluoro acetic acid (TFA) |
| | Merck | Copper (II) sulphate (CuSO₄) |
| | Novabiochem | Benzotriazol-1-yl-oxytripyrrolidinophosphonium hexaluorophosphate (PyBOP), Oxyma pure |
| | Scharlau | Dichloromethane (DCM), dimethylformamide, (DMF), methanol (MeOH), acetonitrile (AcCN), DMSO, tbutylmethyl ether (tBuMeO), NMP |
| | SDS | Acetone, toluene, dioxane |
| | PCAS Biomatrix Inc. | ChemMatrix^{®} resin |

General considerations about the manual synthesis: Solid-phase peptide elongation and other solid-phase manipulations were carried out manually in polypropylene syringes fitted with a polyethylene porous disk. Solvents and soluble reagents were removed by suction. Washings between different synthetical steps were carried out with dimethylformamide (DMF) (5 × 30 s) and dichloromethane (DCM) (5 × 30 s) using 10 mL of solvent/g of resin each time.

General considerations about the microwave assisted synthesis: Microwave assisted solid-phase peptide synthesis was carried out on a Liberty Blue Automated Microwave Peptide Synthesizer using H-Rink amide Protide resin (loading: 0.56 mmols/g). Linear peptide was synthesized on a 0.5 mmol scale using a 5 excess of Fmoc-aminoacid (0.2M) relative to the resin.

Identification tests:The test used for the identification and control of the synthesis was the following: A) Kaiser colorimetric assay for the detection of solid-phase bound primary amines (E. Kaiser et al., Anal. Biochem. 1970, vol. 34, pp. 595-598); B) p-nitro phenyl ester test for secondary amines bound to solid-phase (A. Madder et al., Eur. J. Org. Chem. 1999, pp. 2787-2791).

Protocols used during the manual synthesis of the compounds: The compounds were synthesized at a 100 µmοl scale using the following methods and protocols: The resin for the manual synthesis was selected depending on the group Y: If Y is a OH, the terminus will be COOH, 2-chlorotrytil chloride resin will be choosen among others available. If Y is a NH₂, the terminus will be CONH₂, Rink amide MBHA resin resin will be chosen among others available.

Resin initial conditioning: The resin was conditioned by washing with MeOH (5 × 30 s), DMF (5 × 30 s), DCM (5 × 30 s), 1% TFA in DCM (1 × 30 s and 2 × 10 min), DCM (5 × 30 s), DMF (5 × 30 s), DCM (5 × 30s), 5 % DIEA in DCM (1 × 30 s, 2 × 10 min), DCM (5 × 30 s), DMF (5 × 30 s).

Fmoc group removal: Removal of the 9-fluorenylmethyloxycarbonyl (Fmoc) protecting group was done with 20% (v/v) piperidine in DMF using a treatment of 30 s followed by two treatments of 10 minutes each. Two additional treatments with DBU, toluene, piperidine, DMF (5%, 5%, 20%, 70%) (2 × 5 min) were performed to ensure the removal of the Fmoc group from secondary amines (proline).

### Coupling methods described for 100 pmol scale:

Coupling method 1: The protected amino acid (4 eq., 400 µmols), TBTU (4 eq., 400 µmols, 128 mg) dissolved in DMF (1-3 mL/g resin) were added sequentially to the resin, subsequently DIEA was added (8 eq, 800 µmols, 136 µ!). The mixture was allowed to react with intermittent manual stirring for 1 h. The solvent was removed by suction and the resin washed with DMF (5 × 30 s) and DCM (5 × 30 s). The extent of coupling was checked by the Kaiser colorimetric assay. The Fmoc group was removed with a 20% solution of piperidine in DMF (v/v) using a 30 s treatment and two treatments of 10 minutes. If the amino acid to be deprotected was a proline, an additional tretament with DBU, toluene, piperidine, DMF (5%, 5 %, 20%, 70%) (2 × 5 min) was applied to ensure the removal of the Fmoc group.

Coupling method 2: The protected amino acid (4 eq. 400 µmols), PyBOP (4 eq., 400 µmols, 208 mg), HOAt (12 eq., 1.2 mmols, 163 mg) dissolved in DMF (1-3 mL/g resin) were added sequentially to the resin, subsequently DIEA was added (12 eq., 1.2 mmols, 204 µL). The mixture was allowed to react with intermittent manual stirring for 1 h. The solvent was removed by suction and the resin washed with DMF (5 × 30 s) and DCM (5 × 30 s). The coupling reaction was carried out twice under the same conditions. The extent of coupling was checked by the Kaiser colorimetric assay. The Fmoc group was removed with a 20% solution of piperidine in DMF (v/v) using a 30 s treatments and two treatments of 10 minutes. If the amino acid to be deprotected was a proline, an additional tretament with DBU, toluene, piperidine, DMF (5%, 5 %, 20%, 70%) (2 × 5 min) was applied to ensure the removal of the Fmoc group.

Coupling method 3: The protected amino acid (4 eq., 400 µmols), PyBOP (4 eq., 400 µmols, 208 mg), HOBt (12 eq., 1.2 mmols, 162 mg) dissolved in DMF (1-3 mL/g resin) were added sequentially to the resin, subsequently DIEA was added (12 eq., 1.2 mmols, 204 µ L). The mixture was allowed to react with intermittent manual stirring for 1 h. The solvent was removed by suction and the resin washed with DMF (5 × 30 s) and DCM (5 × 30 s). The coupling reaction was carried out twice under the same conditions. The extent of coupling was checked by the Kaiser colorimetric assay. The Fmoc group was removed with a 20% solution of piperidine in DMF (v/v) using a 30 s treatments and two treatments of 10 minutes. If the amino acid to be deprotected was a proline, an additional tretament with DBU, toluene, piperidine, DMF (5%, 5 %, 20%, 70%) (2 × 5 min) was applied to ensure the removal of the Fmoc group.

Coupling method 4, scale 100 µmols: The protected amino acid (3 eq., 300 µmols)), DIC (3 eq., 300 µmols, 46 µL) and Oxyma (3 eq., 300 µmols, 43 mg) in DCM/DMF (1:1). The mixture was allowed to react with intermittent manual stirring for 45 min. The solvent was removed by suction and the resin washed with DMF (5 × 30 s) and DCM (5 × 30 s). The extent of coupling was checked by the Kaiser colorimetric assay. The Fmoc group was removed with a 20% solution of piperidine in DMF (v/v) using a 30 s treatments and two treatments of 10 minutes. If the amino acid to be deprotected was a proline, an additional tretament with DBU, toluene, piperidine, DMF (5%, 5 %, 20%, 70%) (2 × 5 min) was applied to ensure the removal of the Fmoc group.

Coupling method 5, scale 100 µmols: The protected amino acid (3 eq., 300 µmols)), DIC (3 eq., 300 µmols, 46 µL) and HOBt (3 eq., 300 µmols, 41 mg) in DCM/DMF (1:1). The mixture was allowed to react with intermittent manual stirring for 45 min. The solvent was removed by suction and the resin washed with DMF (5 × 30 s) and DCM (5 × 30 s). The extent of coupling was checked by the Kaiser colorimetric assay. The Fmoc group was removed with a 20% solution of piperidine in DMF (v/v) using a 30 s treatments and two treatments of 10 minutes. If the amino acid to be deprotected was a proline, an additional tretament with DBU, toluene, piperidine, DMF (5%, 5 %, 20%, 70%) (2 × 5 min) was applied to ensure the removal of the Fmoc group.

Protocols used during the microwave assisted automated synthesis: The compounds were synthesized at a 500 µmol scale using the following methods and protocols:The resin for the microwave assisted automated synthesis was selected depending on the group Y: If Y is a OH, the terminus will be COOH, CI-TCP(CI) ProTide resin will be choosen among others available. If Y is a NH₂, the terminus will be CONH₂, Rink amide ProTide resin resin will be chosen among others available.

Resin initial conditioning: The resin was conditioned by washing with MeOH (5 × 30 s), DMF (5 × 30 s), DCM (5 × 30 s), 1% TFA in DCM (1 × 30 s and 2 × 10 min), DCM (5 × 30 s), DMF (5 × 30 s), DCM (5 × 30s), 5 % DIEA in DCM (1 × 30 s, 2 × 10 min), DCM (5 × 30 s), DMF (5 × 30 s).

### Coupling and deprotection conditions for the microwave assisted automated peptide synthesis:

### Coupling conditions:

| CEM preference | Reagents | Cycle | Microwave method | Exceptions |
|---|---|---|---|---|
| 1 | AA/DIC/Oxyma in DMF | Single coupling | Standard | His: Use singe 50°C 10 min coupling |
| | | | | Arg: double coupling |

| Method | Ramp time | | Total time | | Max temp | |
|---|---|---|---|---|---|---|
| Standard coupling | 20-30 s | | 1:05 | | 90 °C | |
| 50 °C MW | | 1. N/A | | 1. 2:00 | | 1. Room Temp |
| | | 2. 30-75s | | 2. 4.00 | | 2. 50 °C |
| Arg coupling | | 1. N/A | | 1. 25:00 | | 1. Room Temp |
| | | 2. 30-75s | | 2. 2:00 | | 2. 75 °C |

### Deprotection conditions:

| CEM preference | Deprotection coktail | Microwave method |
|---|---|---|
| 1 | 10% (w/v) Piperazine in 10:90 (EtOH/NMP) | Standard |
| 2 | 20% Piperidine (v/v) in DMF or NMP | Standard |

| Method | Ramp time | Total time | Max Temp |
|---|---|---|---|
| Standard deprotection | 20-30 s | 1:05 | 90 °C |

### Methods for the cyclization of the peptide sequence P:

Cyclization method 1: disulfide or diselenide bond: The cyclization was performed in solution after the cleavage from the resin or on resin after the selective deprotection of the Cys, Sec or Pen residues. The peptide was dissolved at a concentration of 100 µM in aqueous ammonium bicarbonate buffer 10 mM and pH 8.0. The solution was intensely stirred for 24 h at room temperature. After that, the product was acidified with TFA to pH 2-3, frozen and lyophilized.

Cyclization method 2: amide bond: The cyclization was performed on resin. The Fmoc group was removed with a 20% solution of piperidine in DMF (v/v) using a 30 s treatment and two treatments of 10 minutes. The *N*-terminal amine was protected with a Boc protecting group using Boc₂O (3 eq, 1000 µmol, 56 mg) and DIEA (30 eq, 3000 µmol, 240 µL). The OAI and Alloc groups were first deprotected by addition of tetrakis(triphenylphosphine)palladium(0) (0.1 eq, 10 µM, 12 mg), phenyl silane (10 eq, 1000 µmol, 123 mg) in DCM (3 × 15 min). The resin was washed with 0.02 M sodium diethylcarbamate in DCM (3 × 5 min). The coupling of the amino group of Dap and the carboxylate group of aspartic acid was then achieved by addition of PyBOP (4 eq, 400 µmols, 208 mg), HOAt (12 eq, 1.2 mmol, 163 mg), DMF (1-3 mL/g resin) and DIEA (12 eq, 1.2 mmol, 204 µ L). The coupling was left 1.5 h and repeated overnight.

Cyclization method 3: amide bond: The cyclization was performed on resin. The Fmoc group was removed with a 20% solution of piperidine in DMF (v/v) using a 30 s treatment and two treatments of 10 minutes. The *N*-terminal amine was protected with a Boc protecting group using Boc₂O (3 eq, 1000 µmol, 56 mg) and DIEA (30 eq, 3000 µmol, 240 µL). The OAI and Alloc groups were first deprotected by addition of tetrakis(triphenylphosphine)palladium(0) (0.1 eq, 10 µM, 12 mg), phenyl silane (10 eq, 1000 µmol, 123 mg) in DCM (3 × 15 min). The resin was washed with 0.02 M sodium diethyldithiocarbamate in DCM (3 × 5 min). The coupling of the amino group of Dap and the carboxylate group of aspartic acid was then achieved by_2 cycles of 30 min of 4 equivalents of Oxyma (400 µmols, 57 mg) and 4 of N,N'-Diisopropylcarbodiimide (DIC) (400 µmols, 61 µL).

Cyclization method 4: amide bond: The cyclization was performed on resin. The Fmoc group was removed with a 20% solution of piperidine in DMF (v/v) using a 30 s treatment and two treatments of 10 minutes. The N-terminal amine was protected with a Boc protecting group using Boc₂O (3 eq., 1000 µmols, 56 mg) and DIEA (30 eq., 3000 µmols, 240 µL). The OAI and Alloc groups were first deprotected by addition of tetrakis(triphenylphosphine)palladium(0) (0.1 eq., 10 µM, 12 mg), phenyl silane (10 eq., 1000 µmols, 123 mg) in DCM (3 × 15 min). The resin was washed with 0.02 M sodium diethyldithiocarbamate in DCM (3 × 5 min). The coupling of the amino group of Dap and the carboxylate group of aspartic acid was then achieved by_2 cycles of 1 hour of 4 equivalents of DIC (400 µmols, 61 µL) and 4 of HOBt (400 µmols, 54 mg).

### General methods for the construction of the linker L:

General method for the formation of disulfides: the disulfide bond can be accomplished by reaction of two thiols. The thiols are dissolved at a concentration of 100 µM in aqueous ammonium bicarbonate buffer 10 mM and pH 8.0 and the solution is intensely stirred for 24 h at room temperature. After that, the solution is acidified with TFA to pH 2-3, frozen and lyophilized.

General methods for the formation of thioethers:The thioether bond is accomplished by reaction of an N-terminal bromoacetyl group with a cysteine thiol as described in P.L. Barker et al. J. Med. Chem. 1992. Vol 35. Pp. 2040-2048.

General methods for the formation of ethers: Ether formation can be accomplished by reaction of an hydroxyl group with an halo alkyl compound, preferably under basic conditions as described in Greene's Protective Groups in Organic Synthesis, Fifth Edition. Peter G. M. Wuts. 2014 John Wiley & Sons, Inc. pages 26-29.

General methods for the formation of esters: Ester formation can be accomplished by reaction of an hydroxyl group and a carboxylic acid using typical esterification conditions, such as Fischer esterification in the presence of acid catalysis, or alterntively with the reaction of the hydroxyl group with the corresponding acid chloride, as describede in Greene's Protective Groups in Organic Synthesis, Fifth Edition. Peter G. M. Wuts. 2014 John Wiley & Sons, Inc. pages 271-279

General methods for the formation of thioesters:The thioester bond is accomplished by reaction of an thiol with a carboxilic acid as described in M. Kazemi et al., Journal of Sulfur Chemistry, 2015, vol. 36:6, pp. 613-623.

General methods for the formation of urethanes:The reaction of the hydroxyl group with an isocyanate can yield the corresponding urethane, as described here M. T. Nguyen et al., J. Org. Chem. 1998, 63, vol. 20, pp. 6878-6885.

General methods for the formation of silyl ethers: Reaction of an hydroxyl group with an halo trialkyl silyl yields the corresponding silyl ether. An acid scavening agent is usually needed, as described in Greene's Protective Groups in Organic Synthesis, Fifth Edition. Peter G. M. Wuts. 2014 John Wiley & Sons, Inc. pages 456-463.

General methods for the formation of sulphonates: Reaction of an hydroxyl group with an alkyl or aryl sulphonyl halide yields the corresponding sulfphonate ester as described in F. David et al., Org. Process Res. Dev. 2010, 14, 4, 999-1007.

General methods for the formation of phosphates:Reaction of an hydroxyl group with a dialkyl or diaryl phosphate ester having one hydroxy group under dehydrating condition or using Mitsunobu reaction conditions can form the corresponding phosphate ester where one of the substituents is the chain attached to the hydroxyl group.

General methods for the formation of ketals: Ketals can be formed by reaction of an hydroxyl group with an halomethylenoxy alkyl compound, or by addition of an hydroxyl group to a substituted dihydropyran or dihydrofuran under acidic conditions, as described in Greene's Protective Groups in Organic Synthesis, Fifth Edition. Peter G. M. Wuts. 2014 John Wiley & Sons, Inc. pages 69-77.

General methods for the formation of hemiketals:The contact of an hydroxyl group with an aldehyde can yield the formation of the corresponding hemiketal, as described here https://www.cliffsnotes.com/study-guides/chemistry/organic-chemistry-ii/aldehydes- and-ketones/reactions-of-aldehydes-and-ketones.

General methods for the formation of carbamates: Carbamates can be formed by reaction of an hydroxyl group with an haloformate or an isocyanate, as described in Greene's Protective Groups in Organic Synthesis, Fifth Edition. Peter G. M. Wuts. 2014 John Wiley & Sons, Inc.. pages 371-374.

General methods for the formation of carbonates: Carbonates can be formed by reaction of SN38 with PNPC, as described in Eur J Pharm Biopharm, 2017, vol. 115, pp.149-158 or triphosgene as described in J. Med. Chem. 2008, vol. 51, pp.6916-6926.

Coupling of Fmoc-TTDS-OH: The coupling of the Fmoc-TTDS-OH (2 equivalents), was achieved by either 2 cycles of 30 min of 4 equivalents of oxyma and 4 of N,N'-Diisopropylcarbodiimide (DIC) in DMF or 4 equivalents of DIC and 4 of HOBt in DCM during 2 h. Followed by removal of the 9-fluorenylmethyloxycarbonyl (Fmoc) protecting group with 20% (v/v) piperidine in DMF using a treatment of 30 s followed by two treatments of 10 minutes each.

Coupling of 5-hexynoic acid:The coupling of the 5-hexynoic acid (2 eq., 200 µmols, 23 mg), was achieved by either 2 cycles of 30 min of 4 equivalents of Oxyma (400 µmols, 57 mg) and 4 of N,N'-diisopropylcarbodiimide (DIC) (400 µmols, 61 µL) in DMF:DCM (1:1) or 4 equivalents of DIC (400 µmols, 61 µL) and 4 of HOBt (400 µmols, 54 mg) in DMF:DCM 1:1 in DCM during 4 h or 2 equivalents of PyBOP (400 µmols, 208 mg ) in DMF:DCM 1:1, 6 equivalents of HOAt (600 µmols, 81.5 mg) and 6 equivalents of DIEA (600 µmols, 102 µL) for 1.5 h in DMF. The solvent was removed by suction, the resin washed with DMF (5 × 30 s) and DCM (5 × 30 s). The coupling was repeated under the same conditions. The extent of coupling was monitored using the Kaiser colorimetric assay.

Coupling diglycolic anhidride: The coupling of diglycolic anhidride (10 eq., 1000 µmols, 116 mg), was achieved by_2 cycles of 60 min of 10 equivalents of DIEA (1000 µmols, 174 µL) in DMF. The solvent was removed by suction, the resin washed with DMF (5 × 30 s) and DCM (5 × 30 s). The coupling was repeated under the same conditions. The extent of coupling was monitored using the Kaiser colorimetric assay.

General methods for the cleavage from the resin: Final cleavage of the resin and side-chain deprotection: It was carried out by treating resin with TFA (95%), H₂O (2.5%) and TIS (2.5%) (2 h). Tert-butyl methyl ether was added to the obtained product and the mixture was centrifuged (3 × 8 min). The supernatant was discarded and the pellet was resuspended in a mixture of H₂O, MeCN and TFA (1000:1000:1). The product was filtered out and frozen.

General methods for the characterization of the compounds: The compounds were characterized by UPLC (Acquity high-class system (PDA detector, sample manager FNT and Quaternary solvent manager, Acquity BEH C18 (50 × 2 mm × 1.7 µm) column, 0.61 mL/min and MeCN (0.036% TFA) and H₂O (0.045% TFA) were used as solvents. In all cases, 2-min linear gradients were used) and UPLC-MS spectrometry (Waters high class (PDA detector, sample manager FNT and Quaternary solvent manager) coupled to an electrospray ion source ESI-MS Micromass ZQ and using the MassLynx 4.1 software (Waters, Milford, MA). Using a BEH C18 column (50 × 2.1 mm × 1.7 µm, Waters). The flow rate was 0.6 mL/min, and MeCN (0.07% formic acid) and H₂O (0.1% formic acid) were used as solvents. Samples were analysed with positive ionisation: the ion spay voltage was 30 V and the capillary temperature was 1 kV). The exact mass was obtained by Mass Spectrometer: LTQ-FT Ultra (Thermo Scientific) with sample introduction in Direct infusion (Automated Nanoelectrospray). The NanoMate (Advion BioSciences, Ithaca, NY, USA) aspirated the samples from a 384-well plate (protein Lobind) with disposable, conductive pipette tips, and infused the samples through the nanoESI Chip (which consists of 400 nozzles in a 20 × 20 array) towards the mass spectrometer. Spray voltage was 1.70 kV and delivery pressure was 0.50 psi; the ionization was NanoESI, positive ionization.

NMR experiments were carried out on a Bruker Avance III 600 MHz spectrometer equipped with a TCI cryoprobe. Samples were prepared by dissolving compounds in 90% H₂O/10% D₂O at 3-4 mM and pH was adjusted to 2-3. Chemical shifts were referenced to internal sodium-3-(trimethylsilyl)propanesulfonate (DSS). Suppression of the water signal was achieved by excitation sculpting. Residue specific assignments were obtained from 2D total correlated spectroscopy (TOCSY) and correlation spectroscopy (COSY) experiments, while 2D nuclear Overhauser effect spectroscopy (NOESY) permitted sequence specific assignments. 13C resonances were assigned from 2D 1H13C HSQC spectra. All experiments were performed at 298 K except NOESY spectra that were acquired at 278 K. Amide proton temperature coefficients were determined from a series of onedimensional spectra acquired between 278 and 308K. The TOCSY and NOESY mixing times were 70 and 250 ms, respectively.

### Example 1: Preparation of (S)-tert-butyl (4,11-diethyl-4-hydroxy-3,14-dioxo-3,4,12,14-tetrahydro-1H-pyranor3',4':6,7]indolizino[1,2-b]quinolin-9-yl) carbonate

To a 250 ml flask, 1.5 g of 7-ethyl-10-hydroxy-camptothecin (SN38), 1.3 equivalents of di-tert-butyl dicarbonate (1.15 mL) and excess of dry pyridine (9mL) were added in 150 ml of dry DCM. The mixture was stirred at room temperature overnight. Afterwards the reaction mixture was washed with HCl (0,5 N)×3, saturated NaHCOsx1 and brine. The organic layer was dried with MgSO₄ and the solvents were removed under vacuum. No further purification was performed. Reverse phase UPLC-PDA: linear gradient from 0 to 100% MeCN in H₂O in 2 minutes using a Acquity BEH C18 (50 × 2 mm × 1.7 µm) column, 0.61 mL/min and MeCN (0.036% TFA) and H₂O (0.045% TFA) were used as solvents; Retention time: 1.89 min. Yield: 96%, [M+H]ₑₓₚ⁺: 493.5 Da. 1H NMR (400MHz, choroform-*d)* δ 7.81 (d, *J*= 9.2 Hz, 1H), 7.45 (d, *J*= 2.5 Hz, 1H), 7.25-7.20 (m, 1H), 6.91- 6.85 (m, 1H), 5.32 (d, *J*= 16.3, 1H), 4.86 (d, *J*= 1Hz, 6H), 3.85 (s, 1H), 2.73 (q, *J*= 7.7 Hz, 2H), 1.60 (s, 1H), 1.57-1.41 (m, 3H), 1,21 (s, 10H), 0.99 (t, *J*=7.7 Hz, 3H), 0.61 (t, *J*= 7.3 Hz, 3H).

### Example 2: Preparation of (S)-9-((tert-butoxycarbonyl)oxy)-4,11-diethyl-3,14-dioxo-3,4,12,14-tetrahydro-1H-pyrano[3',4':6,7]indolizino[2-b]quinolin-4-yl 5-azidopentanoate

In a round bottom flask, 400 mg of (S)-tert-butyl (4,11-diethyl-4-hydroxy-3,14-dioxo-3,4,12,14-tetrahydro-1H-pyrano[3',4':6,7]indolizino[1,2-b]quinolin-9-yl) carbonate (Example 1), 1.6 equivalents of 5-azido-pentanoic acid (193 mg) were purged with N₂ before being dissolved in dry DCM. The mixture was cooled at 0°C. 1.4 equivalents of N-(3-Dimethylaminopropyl)-N'-ethylcarbodiimide hydrochloride (218 mg) was then added and the mixture was stirred at 0°C for 1 hour and at r.t. Overnight. The mixture was washed with NaHCOs saturated x3, HCl(0,1N)x2 and brine. Then organic layer was dried with MgSO₄ and removed under vacuum. The compound was used without further purification. [M+H]ₑₓₚ⁺: 618.36 Da.

### Example 3: Preparation of (S)-4,11-diethyl-9-hydroxy-3,14-dioxo-3,4,12,14-tetrahydro-1H-pyrano[3',4':6,7]indolizino[1,2-b]quinolin-4-yl 5-azidopentanoate (modification of SN38 with an azide as a result of Examples 1-3)

In a round bottom flask, 1.5 g of (S)-9-((*tert*-butoxycarbonyl)oxy)-4,11-diethyl-3,14-dioxo-3,4,12,14-tetrahydro-1*H*-pyrano[3',4':6,7]indolizino[1,2-*b*]quinolin-4-yl 5-azidopentanoate (Example 2) was stirred in 50 ml HCl (4N in dioxane) during 2 hours at r.t. Afterwards, the solvent was removed under vacuum and the crude mixture was purified with with silica using (DCM/MeOH (10%)) with a purity higher than 95%. Total yield from example 1: 8%, [M+H]⁺: 518,58 Da. Reverse phase UPLC-PDA: linear gradient from 0 to 100% MeCN in H₂O in 2 min using a Acquity BEH C18 (50 × 2 mm × 1.7 µm) column, 0.61 mL/min and MeCN (0.036% TFA) and H₂O (0.045% TFA) were used as solvents; Retention time: 1.819 min. [M+H]ₑₓₚ⁺: 518.58 Da. ¹H-NMR (400MHz, chloroform-d) δ 1.00 (t, 3H), 1.38 (t, 3H), 1.65 (m, 2H), 1.69 (m, 2H), 2.20 (m, 2H), 2.56 (m, 2H), 3.13 (q, 3H), 3.27 (td, 2H), 5.20 (s, 2H), 5.39-5.72 (dd, 2H), 7.42 (d, 1H), 7.48 (s, 1H), 7.62 (dd, 1H), 8.45 (d, 1H).

### Example 4: Preparation of (S)-9-((tert-butoxycarbonyl)oxy)-4,11-diethyl-3,14-dioxo-3,4,12,14-tetrahydro-1H-pyrano[3',4':6,7]indolizino[1,2-b]quinolin-4-yl tert-butyl succinate

1 mmol of (*S*)-*tert*-butyl (4,11-diethyl-4-hydroxy-3,14-dioxo-3,4,12,14-tetrahydro-1*H-*pyrano[3',4':6,7]indolizino[1,2-*b*]quinolin-9-yl) from Example 1 and 1.5 equivalents of mono-tert-Butyl succinate were added to a round bottom flask and purged with N₂. The reagents were dissolved in 20 ml of dry DCM and cooled at 0°C. N-(3-Dimethylaminopropyl)-N'-ethylcarbodiimide hydrochloride (EDC·HCl) was then added and the mixture was stirred at 0°C during 1 hour and overnight at room temperature. The reaction mixture was washed with NaHCO₃ saturated x3, HCl(0,1N)x2 and brine. Then it was dried with MgSO₄, evaporated the volatiles and dried. The compound was used without further purification. Yield 72%. ¹H-NMR (400MHz, chloroform-d) δ 0.92 (t, 3H), 1.30 (s, 9H), 1.33 (m, 3H), 1.38 (q, 2H) ,1.42 (s, 2H), 1.55 (s, 9H), 2.14 (ddq, 2H), 2.49 (td, 2H), 2.69 (m, 2H), 2.86 (dd, 2H), 3.09 (m, 2H), 5.20 (s, 2H), 5.32 (d, 2H), 5.61 (d, 2H).

### Example 5: Preparation of (S)-4-((4,11-diethyl-9-hydroxy-3,14-dioxo-3,4,12,14-tetrahydro-1H-pyrano[3',4':6,7]indolizino[1,2-b]auinolin-4-yl)oxy)-4-oxobutanoic acid (modification of SN38 with succinic acid as a result of examples 4 and 5)

In a round bottom flask, (S)-9-((*tert*-butoxycarbonyl)oxy)-4,11-diethyl-3,14-dioxo-3,4,12,14-tetrahydro-1*H*-pyrano[3',4':6,7]indolizino[1,2-*b*]quinolin-4-yl *tert*-butyl succinate (Example 4) was stirred in 20 ml HCl (4N in dioxane) for 2 hours at r.t. Afterwards, the solvent was evaporated and the crude mixture was purified with the Teledyne-ISCO, gradient 0-50. It was obtained the desired compound with a yield of 32% and purity higher than 95%. Yield 32%. Reverse phase UPLC-PDA: linear gradient from 0 to 100% MeCN in H₂O in 2 minutes using a Acquity BEH C18 (50 × 2 mm × 1.7 µm) column, 0.61 mL/min and MeCN (0.036% TFA) and H₂O (0.045% TFA) were used as solvents; Retention time: 1.498 min. [M+H]ₑₓₚ⁺: 491.27Da.¹H-NMR (400MHz, chloroform-*d)* δ 1.02 (t, 3H), 1.39 (t, 3H), 2.22 (m, 2H), 2.61 (t, 2H), 2.84 (m, 2H), 3.16 (q, 2H), 5.27 (s, 2H), 5.45 (d, 2H), 5.60 (d, 2H), 7.33 (s, 1H), 7.42 (dq, 1H), 8.03 (d, 1H).

Methods for the alkyne-azide cycloaddition:The alkyne-azide cycloaddition (Click reaction) coupling was performed in solution using the protocol described in S.F.M. van Dongen et al.; Bioconjugate Chem. 2009, vol. 20, pp. 20-23.

This reaction was done without microwave following the procedure descrived in lumiprobe (https://www.lumiprobe.com/protocols/click-chemistry-dna-labeling) However, this reaction took around 2 days to be completed. The Cu was used with the ligant THTPA. As SN38-N₃ was not soluble in H₂O, it was made the reaction only using DMF instead of buffer and DMSO because it is not soluble in water or even in the mixture (water/DMSO).

Alternatively the alkyne-azide cycloaddition (Click reaction) of alkyne-TTDS-DapKAPETALD with SN38-N₃ with was done using microwaves:To a microwave vial of 10 ml SN38-N₃ (1.5 eq.), alkyne-TTDS-DapKAPETALD (1 eq.), CuTHTPA (0.15 eq.) and sodium ascorbate (0.3 eq.) were added and dissolved in 3 ml of DMF. The mixture was stirred from 2 to 4 hours with MW (discover SP MW) assisted at 30°C during all reaction. The crude mixture was purified with HPLC semipreparative (C18).

Preparation of reagents for alkyne-azide cycloaddition (Click chemistry): 100 mM Copper (II)-THPTA Stock in 55% DMSO: 50 mg of copper (II) sulfate pentahydrate were dissolved in 1 mL of distilled water and 116 mg of tris(3-hydroxypropyltriazolylmethyl)amine (THPTA) ligand were dissolved in 1.1 mL of DMSO. Then, the two solutions were mixed. 5 mM Ascorbic Acid Stock: 18 mg of ascorbic acid were dissolved in 20 mL of distilled water.

General method for purification and characterization of products: The crude were purified by RP-HPLC at semi-preparative scale and characterized by UPLC (Acquity high-class system (PDA detector, sample manager FNT and Quaternary solvent manager, Acquity BEH C18 (50 × 2 mm × 1.7 µm) column, 0.61 mL/min and MeCN (0.036% TFA) and H2O (0.045% TFA) were used as solvents. In all cases, 2-min linear gradients were used) and UPLC-MS spectrometry (Waters high class (PDA detector, sample manager FNT and Quaternary solvent manager) coupled to an electrospray ion source ESI-MS Micromass ZQ and using the MassLynx 4.1 software (Waters, Milford, MA). Using a BEH C18 column (50 × 2.1 mm × 1.7 µm, Waters). The flow rate was 0.6 mL/min, and MeCN (0.07% formic acid) and H₂O (0.1% formic acid) were used as solvents. Samples were analysed with positive ionisation: the ion spay voltage was 30 V and the capillary temperature was 1 kV). The exact mass was obtained by Mass Spectrometer: LTQ-FT Ultra (Thermo Scientific) with sample introduction in Direct infusion (Automated Nanoelectrospray). The NanoMate (Advion BioSciences, Ithaca, NY, USA) aspirated the samples from a 384-well plate (protein Lobind) with disposable, conductive pipette tips, and infused the samples through the nanoESI Chip (which consists of 400 nozzles in a 20 × 20 array) towards the mass spectrometer. Spray voltage was 1.70 kV and delivery pressure was 0.50 psi; the ionization was NanoESI, positive ionization. All peptides were obtained with a purity higher than 95%.

### Example 6 : Preparation of Hexynoic-TTDS-Dap-Lys-Ala-Pro-Glu-Thr-Ala-Leu-Asp-NH₂ (hexynoic-TTDS-SEQ ID NO: 7) with a amide bond between Dap side-chain amino group and Asp side-chain carboxilic acid.

For the manual coupling of the first protected amino acid to the resin, the coupling method 4 was applied using Fmoc-Asp(OAI)-OH (118.5 mg). The subsequent amino acids were coupled sequentially as follows using coupling method 4:

| **Add. order** | **Code** | **Amino acid** | **MW** | **m (mg)** | **eq** |
|---|---|---|---|---|---|
| 1 | D | Fmoc-L-Asp(OAI) -OH | 395.4 | 118.5 | 3 |
| 2 | L | Fmoc-L-Leu-OH | 353.4 | 105.9 | 3 |
| 3 | A | Fmoc-Ala-OH H₂O | 329.3 | 98.7 | 3 |
| 4 | T | Fmoc-L-Thr(tBu)-OH | 397.5 | 119.1 | 3 |
| 5 | E | Fmoc-Glu(OtBu)-OH H₂O | 443.5 | 132.9 | 3 |
| 6 | P | Fmoc-L-Pro-OH H₂O | 355.4 | 106.5 | 3 |
| 7 | A | Fmoc-Ala-OH H₂O | 329.3 | 98.7 | 3 |
| 8 | K | Fmoc-Lys(Boc)-OH | 468.5 | 140.4 | 3 |
| 9 | Dap | Fmoc-L-Dap(Alloc)-OH | 410.4 | 123 | 3 |

Using 46 µL DIC and 43 mg Oxyma in DMF/DCM (1:1). The mixture was allowed to react with intermittent manual stirring for 45 min. After each coupling removal of the 9-fluorenylmethyloxycarbonyl (Fmoc) protecting group was done with 20% (v/v) piperidine in DMF using a treatment of 30 s followed by two treatments of 10 minutes each. Two additional treatments with DBU, toluene, piperidine, DMF (5%, 5%, 20%, 70%) (2 × 5 min) were performed to ensure the removal of the Fmoc group from secondary amines (proline). The cyclization was performed on resin following cyclization method 2:The Fmoc group was removed with a 20% solution of piperidine in DMF (v/v) using a 30 s treatment and two treatments of 10 minutes. The *N*-terminal amine was protected with a Boc protecting group using Boc₂O (3 eq., 1000 µmols, 56 mg) and DIEA (30 eq., 3000 µmols, 240 µL). The OAI and Alloc groups were first deprotected by addition of tetrakis(triphenylphosphine)palladium(0) (0.1 eq., 10 µM, 12 mg), phenyl silane (10 eq., 1000 µmols, 123 mg) in DCM (3 × 15 min). The resin was washed with 0.02 M sodium diethylcarbamate in DCM (3 × 5 min). The coupling of the amino group of Dap and the carboxylate group of aspartic acid was then achieved by addition of PyBOP (4 eq., 400 µmols, 208 mg), HOAt (12 eq., 1.2 mmols, 163 mg), DMF (1-3 mL/g resin) and DIEA (12 eq., 1.2 mmols, 204 µ L). The coupling was left 1.5 h and repeated overnight.

Coupling of Fmoc-TTDS-OH:The coupling of the Fmoc-TTDS-OH (2 equivalents, 200 µmols, 108.53 mg), was achieved by_4 equivalents of DIC (400 µmols, 61 µL) and 4 of HOBt (400 µmols, 54 mg) in DCM during 2 h._Followed by removal of the 9-fluorenylmethyloxycarbonyl (Fmoc) protecting group with 20% (v/v) piperidine in DMF using a treatment of 30 s followed by two treatments of 10 minutes each.

Coupling of 5-hexynoic acid: For the coupling of the 5-hexynoic acid to the peptide anchored onto the resin the following protocol was used: hexynoic acid (4 eq., 400 µmols, 45 mg) in DMF (1-3 mL/g resin), PyBOP (4 eq., 400 µmols, 208 mg) and HOAt (12 eq., 1.2 mmols, 163 mg) were sequentially added to the resin followed by the addition of 12 eq. of DIEA (1.2 mmols, 204 µL). The mixture was allowed to react with intermittent manual stirring for 1.5 h. The solvent was removed by suction, the resin washed with DMF (5 × 30 s) and DCM (5 × 30 s). The coupling was repeated under the same conditions. The extent of coupling was monitored using the Kaiser colorimetric assay.

The peptide was then cleaved and liophilized. Product characterization. Reverse phase UPLC: linear gradient from 20 to 60% MeCN in H₂O in 2 min using a Acquity BEH C18 (50 × 2 mm × 1.7 µm) column, 0.61 mL/min and MeCN (0.036% TFA) and H₂O (0.045% TFA) were used as solvents; Retention time: 0.939 min. UPLC-MS [M + H]ₑₓₚ⁺: 1308.15 Da; Yield (synthesis and purification): 7.5 %.

### Example 7: Preparation of compound of formula (Ia), SN38-linker A -MiniAp4

Starting from Hexynoic-TTDS-MiniAp4 prepared as in example 6, and using SN38-N₃ prepared as in example 3, the following protocol was follow to achieve compound of formula (Ia) also named SN38-linker A -MiniAp4:
To a microwave vial of 10 ml 1.5 eq. of *(S)-4,11-diethyl-9-hydroxy-3,14-dioxo-3,4,12,14-tetrahydro-1H-pyrano[3',4':6,7]indolizino[1,2-b]quinolin-4-yl 5-azidopentanoate,* 1 eq. of Alkyne-TTDS-DapKAPETALD, 0.15 eq. of CuTHPTA and 0.3 eq. of sodium ascorbate were added and dissolved in 3 ml of DMF. The mixture was stirred from 2 to 4 hours with MW (CEM discover SP MW) assisted at 30°C during all reaction. The crude was purified by RP-HPLC at semi-preparative scale. It was obtained the compound with a purity higher than 95%. Product characterization: Reverse phase UPLC-PDA: linear gradient from 0 to 100% MeCN in H₂O in 2 minutes using a Acquity BEH C18 (50 × 2 mm × 1.7 µm) column, 0.61 mL/min and MeCN (0.036% TFA) and H₂O (0.045% TFA) were used as solvents; Retention time: 1.462 min. UPLC-MS [M + H]ₑₓₚ⁺: 1824.76 Da; Yield (synthesis and purification): 30%.

### Comparative Example 1: SN38-linker A-THRre

In this comparative example, all the amino acids used are D-amino acids. For the manual coupling of the first protected amino acid to the resin, the coupling method 4 was applied using Fmoc-D-Thr(tBu)-OH (159 mg). The subsequent amino acids were coupled sequentially as follows using coupling method 4:

| **Add. order** | **Code** | **Amino acid** | **MW** | **m (mg)** | **eq** |
|---|---|---|---|---|---|
| 1 | t | Fmoc-D-Thr(tBu)-OH | 397.5 | 159 | 3 |
| 2 | h | Fmoc-D-His(Trt)-OH | 619.7 | 248 | 3 |
| 3 | r | Fmoc-D-Arg(Pbf)-OH | 648.8 | 260 | 3 |
| 4 | p | Fmoc-D-Pro-OH H₂O | 355.4 | 142 | 3 |
| 5 | P | Fmoc-D-Pro-OH H₂O | 355.4 | 142 | 3 |
| 6 | m | Fmoc-D-Met-OH | 371.5 | 149 | 3 |
| 7 | w | Fmoc-D-Trp-OH | 426.5 | 171 | 3 |
| 8 | s | Fmoc-D-Ser(tBu)-OH | 383.4 | 153 | 3 |
| 9 | p | Fmoc-D-Pro-OH H₂O | 355.4 | 142 | 3 |
| 10 | v | Fmoc-D-Val-OH | 339.4 | 136 | 3 |
| 11 | w | Fmoc-D-Trp-OH | 426.5 | 171 | 3 |
| 12 | P | Fmoc-D-Pro-OH H₂O | 355.4 | 142 | 3 |

Using 46 µL DIC and 43 mg Oxyma in DMF/DCM (1:1). The mixture was allowed to react with intermittent manual stirring for 45 min. After each coupling removal of the 9-fluorenylmethyloxycarbonyl (Fmoc) protecting group was done with 20% (v/v) piperidine in DMF using a treatment of 30 s followed by two treatments of 10 minutes each. Two additional treatments with DBU, toluene, piperidine, DMF (5%, 5%, 20%, 70%) (2 × 5 min) were performed to ensure the removal of the Fmoc group from secondary amines (proline).

Coupling of Fmoc-TTDS-OH: The coupling of the Fmoc-TTDS-OH (2 eq., 200 µmols, 108.53 mg), was achieved by 4 eq. of DIC (400 µmols, 61 µL) and 4 of HOBt (400 µmols, 54 mg) in DCM during 2 h._Followed by removal of the 9-fluorenylmethyloxycarbonyl (Fmoc) protecting group with 20% (v/v) piperidine in DMF using a treatment of 30 s followed by two treatments of 10 minutes each.

Coupling of 5-hexynoic acid: For the coupling of the 5-hexynoic acid to the peptide anchored onto the resin the following protocol was used: hexynoic acid (4 eq., 400 µmols, 45 mg) in DMF (1-3 mL/g resin), PyBOP (4 eq., 400 µmols, 208 mg) and HOAt (12 eq., 1.2 mmols, 163 mg) were sequentially added to the resin followed by the addition of 12 eq. of DIEA (1.2 mmols, 204 µL). The mixture was allowed to react with intermittent manual stirring for 1.5 h. The solvent was removed by suction, the resin washed with DMF (5 × 30 s) and DCM (5 × 30 s). The coupling was repeated under the same conditions. The extent of coupling was monitored using the Kaiser colorimetric assay. The peptide was then cleaved and liophilized.

With this Hexynoic-TTDS-THRre and using SN38-N₃ prepared as in example 3, the following protocol was follow to achieve SN38-linker A-THRre:To a microwave vial of 10 ml 1.5 equivalents of *(S)-4,11-diethyl-9-hydroxy-3,14-dioxo-3,4,12,14-tetrahydro-1H-pyrano[3',4':6,7]indolizino[1,2-b]quinolin-4-yl 5-azidopentanoate,* 1 equivalent of Alkyne-TTDS-THRre, 0,15 equivalents of CuTHPTA and 0,3 equivalent of sodium ascorbate were added and dissolved in 3 ml of DMF. The mixture was stirred from 2 to 4 hours with MW (CEM discover SP MW) assisted at 30°C during all reaction. The crude was purified by RP-HPLC at semi-preparative scale. It was obtained the compound with a purity higher than 95%. Product characterization: Reverse phase UPLC-PDA: linear gradient from 0 to 100% MeCN in H₂O in 2 minutes using a Acquity BEH C18 (50 × 2 mm × 1.7 µm) column, 0.61 mL/min and MeCN (0.036% TFA) and H₂O (0.045% TFA) were used as solvents; Retention time: 1.597 min. UPLC-MS [M + H]ₑₓₚ⁺: 2403.86 Da.

### Comparative Example 2: SN38- Linker B-THRre

In this comparative example, all the amino acids used are D-amino acids. For the manual coupling of the first protected amino acid to the resin, the coupling method 4 was applied using Fmoc-D-Thr(tBu)-OH (159 mg). The subsequent amino acids were coupled sequentially as follows using coupling method 4:

| **Add. order** | **Code** | | **MW** | **m (mg)** | **eq** |
|---|---|---|---|---|---|
| 1 | t | Fmoc-D-Thr(tBu)-OH | 397.5 | 159 | 3 |
| 2 | h | Fmoc-D-His(Trt)-OH | 619.7 | 248 | 3 |
| 3 | r | Fmoc-D-Arg(Pbf)-OH | 648.8 | 260 | 3 |
| 4 | p | Fmoc-D-Pro-OH H₂O | 355.4 | 142 | 3 |
| 5 | p | Fmoc-D-Pro-OH H₂O | 355.4 | 142 | 3 |
| 6 | m | Fmoc-D-Met-OH | 371.5 | 149 | 3 |
| 7 | w | Fmoc-D-Trp-OH | 426.5 | 171 | 3 |
| 8 | s | Fmoc-D-Ser(tBu)-OH | 383.4 | 153 | 3 |
| 9 | p | Fmoc-D-Pro-OH H₂O | 355.4 | 142 | 3 |
| 10 | v | Fmoc-D-Val-OH | 339.4 | 136 | 3 |
| 11 | w | Fmoc-D-Trp-OH | 426.5 | 171 | 3 |
| 12 | p | Fmoc-D-Pro-OH H₂O | 355.4 | 142 | 3 |

Using 46 µL DIC and 43 mg Oxyma in DMF/DCM (1:1). The mixture was allowed to react with intermittent manual stirring for 45 min. After each coupling removal of the 9-fluorenylmethyloxycarbonyl (Fmoc) protecting group was done with 20% (v/v) piperidine in DMF using a treatment of 30 s followed by two treatments of 10 minutes each. Two additional treatments with DBU, toluene, piperidine, DMF (5%, 5%, 20%, 70%) (2 × 5 min) were performed to ensure the removal of the Fmoc group from secondary amines (proline).

The peptide was then cleaved and liophilized. With this THRre and using SN38-succinic prepared as in example 5 , the following protocol was follow to achieve SN38- linker B-THRre:_In a round bottom flask, it was added 1.5 eq. of *(S)-4-((4,11-diethyl-9-hydroxy-3,14-dioxo-3,4,12,14-tetrahydro-1H-pyrano[3',4':6,7]indolizino[1,2-b[quinolin-4-yl)oxy)-4-oxobutanoic acid,* 1.2 eq. of N-(3-dimethylaminopropyl)-N'-ethylcarbodiimide hydrochloride (EDC·HCl) and 1.2 eq. of *N*-hydroxybenzotriazole (HOBt) in N₂ atmosphere and 0°C, then it was added dry DMF, and stirred for 10 min. Afterwards, it was added THRre and reacted ON at room temperature. The crude was purified by RP-HPLC at semi-preparative scale. It was obtained the compound with a purity higher than 95%. Product characterization: Reverse phase UPLC-PDA: linear gradient from 0 to 100% MeCN in H₂O in 2 minutes using a Acquity BEH C18 (50 × 2 mm × 1.7 µm) column, 0.61 mL/min and MeCN (0.036% TFA) and H₂O (0.045% TFA) were used as solvents; Retention time: 1.543 min. UPLC-MS [M + 2H]ₑₓₚ⁺²: 982.32Da.

### Example 8a: Evaluation of solubility in water.

25 mg of SN38-linker A-MiniAp4 were disolved in H₂O, showing a completely transparent solution. This means that SN38-linker A-MiniAp4 has a solubility in water higher than 5 mg/mL, more than 400-fold the solubility in water described for SN38 (Mol.Pharmaceutics, 2016, 13, 379-390).

G2B-001 lineal (Ib), G2B-003 (Ic), G2B-004 (Id) and G2B-005 (le) show good solubility in water at 1 mg/mL.

### Example 8b: Evaluation of the turbidimetric Solubility of SN38-linker A-MiniAp4.

SN38-linker A-MiniAp4 compound (10 mM in DMSO) was serially diluted to give solutions of 0.1, 0.3, 1 and 3 mM in DMSO. Each test compound concentration was then further diluted 1 in 100 in buffer (0.01 M phosphate buffered saline pH 7.4) so that the final DMSO concentration was 1 % and the final test compound concentrations were 1, 3, 10, 30 and 100 µM. The experiment was performed at 37 °C and each concentration was incubated in 7 replicate wells. The plates were incubated for 2 hr at 37 °C before the absorbance was measured at 620 nm. Nicardipine and pyrene were included as control compounds. The solubility of nicardipine is pH dependent whereas the solubility of pyrene is pH independent. The solubility was estimated from the concentration of test compound that produces an increase in absorbance above vehicle control (1 % DMSO in buffer). See **Table 2**.

**Table 2. Turbidimetry aqueous solubility data.**

| Compound | Turbidity aqueous solubility | | |
|---|---|---|---|
| | Estimated precipitation range (µm) | | |
| | Lower bond | Upper bond | Calculated mid-range |
| SN38-linker A-MiniAp4 | 30 | 100 | 65 |
| Nicardipine | 3 | 20 | 11.5 |
| Pyrene | 3 | 10 | 6.5 |

No solubility problems found up to 100 micromolar of G2B-001 as expected.

### Example 9: Antitumoral activity of the compound (Ia): SN38 - linker A- MiniAp4 (also named G2B-001) against cancer cell lines: diffuse intrinsic pontine gliomas, adult gliomas and pediatric solid tumors retinoblastoma, Ewing sarcoma, rhabdomyosarcoma and neuroblastoma.

The present inventors compared the activity of the new compound (Ia) SN38- linker A-MlniAp4 (also named G2B-001) with the one of the closely related drugs, SN38 and irinotecan.

Cancer cell lines were obtained from the repository maintained at Hospital Sant Joan de Deu (Barcelona, Spain). Cancer cell lines used in these experiment included cancer types of adult glioma (U373 and U87 cell lines), pediatric gliomas including diffuse intrinsic pontine glioma (HSJD-DIPG-007, HSJD-DIPG-011), and pediatric high-grade glioma (HSJD-GBM-001), retinoblastoma (HSJD-RBT-5, HSJD-RBT-7 and HSJD-RBT-14), Ewing sarcoma (A673), rhabdomyosarcoma (Rd and RH4) and neuroblastoma (LAN-1 and SK-N-JD cell lines and HSJD-NB-004, HSJD-NB-005 and HSJD-NB-016 patient-derived cell models). Briefly, between 3000 and 30,000 cancer cells were cultured in 96 well plates and 24 h later the cells were exposed to compound SN38- linker A-MiniAp4 (Ia) (concentration range 1-0.00000001 µM), SN38 (concentration range 1-0.00000001 µM), or irinotecan (concentration range 100-0,000001 µM). To add the drugs to the cells in culture, SN38- linker A-MiniAp4 (Ia) and irinotecan were prepared in culture medium from stock solutions at concentration 1 mg/mL in water. SN38 was prepared in culture medium from a stock solution at concentration 1 mg/mL in DMSO.The tetrazolium compound [3-(4,5-dimethylthiazol-2-yl)-5-(3-carboxymethoxyphenyl)-2-(4-sulfophenyl)-2H-tetrazolium] (MTS assay; Promega, Fitchburg, Wl) was used to determine cell viability after 72 h incubation with the drugs. The concentrations of drug required to cause a reduction of 50% in cell proliferation (IC50 and 95% confidence intervals) were calculated with Graphpad Prism 8 software (La Jolla, CA). Activity curves were built for adult glioma cell lines U87 (FIG. 1) and U373 (FIG. 2), pediatric gliomas including DIPG models HSJD-DIPG-007 (FIG. 3) and HSJD-DIPG-011 (FIG. 4), and pediatric high-grade glioma model HSJD-GBM-001 (FIG. 5), retinoblastoma models HSJD-RBT-5 (FIG. 6), HSJD-RBT-7 (FIG. 7) and HSJD-RBT-14 (FIG. 8), Ewing sarcoma cell line A673 (FIG. 9), rhabdomyosarcoma cell lines Rd (FIG. 10) and RH4 (FIG. 11), neuroblastoma cell lines LAN-1 (FIG. 12) and SK-N-JD (FIG. 13) and neuroblastoma patient-derived cell models HSJD-NB-004 (FIG. 14), HSJD-NB-005 (FIG. 15) and HSJD-NB-016 (FIG. 16).

The established IC50 values are in **Table 3**.

**Table 3. IC50 values of compounds SN38- linker A-MiniAp4 (Ia), SN38 and irinotecan against cancer cell lines.**

| **Cell line** | **SN38-Linker A-MiniAp4 (Ia)** | **SN38** | **Irinotecan** |
|---|---|---|---|
| A673 | 0.003802 | 0.0004617 | 1.442 |
| HSJD-DIPG-007 | 0.03416 | 0.001125 | 4.748 |
| HSJD-DIPG-011 | 0.07288 | 0.001122 | 1.204 |
| U373 | 0.006647 | 0.0008685 | 3.719 |
| HSJD-GBM-001 | 0.07026 | 0.002611 | 9.573 |
| Rd | 0.006945 | 0.001574 | 5.957 |
| RH4 | 0.08149 | 0.01475 | 10.83 |
| HSJD-RBT-5 | 0.09208 | 0.01031 | 0.9106 |
| HSJD-RBT-7 | 0.0151 | 0.0003349 | 1.12 |
| HSJD-RBT-14 | 0.03353 | 0.001079 | 2.604 |
| U87 | 0.744 | 0.004042 | 9.747 |
| LAN-1 | 0.02161 | 0.001962 | 6.996 |
| SK-N-JD | 0.0139 | 0.00755 | 7.76 |
| HSJD-NB-004 | 0.07685 | 0.2307 | 9.84 |
| HSJD-NB-005 | 0.05145 | 0.01251 | 8.196 |
| HSJD-NB-016 | 0.0256 | 0.01232 | 0.6594 |

The values of IC50 obtained for SN38- linker A- MiniAp4 (Ia) were significantly lower than drug irinotecan (*P* < 0.0001; paired ANOVA test) as shown in FIG. 17.

### Example 10: In vivo tolerability of the SN38- linker A- MiniAp4 (Ia) in single and repeated intravenous injections.

To evaluate the toxicity of irinotecan and SN38- linker A- MiniAp4 (Ia) (also named G2B-001) in athymic nude mice (10 week old; 8 mice per group) first the maximum tolerated dose (MTD) of SN38- linker A- MiniAp4 (Ia) was studied, by intravenous injection. Three mice were injected at dose levels 200, 500 and 1000 mg/kg, and observed for symptoms including death or weight lost during the following 14 days. The MTD was established at 200 mg/kg. At this dose level, mice survived and did not show immediate symptoms of distress or significant weight lost during the following 14 days, compared to untreated control animals (FIG. 18).

Upon the establishement of the MTD of SN38- linker A- MiniAp4 (Ia) at single i.v, we compared mean weight of mice treated with irinotecan at a clinically relevant multi-dose regimen, and SN38- linker A- MiniAp4 (Ia)at an equimolar dose. Briefly, mice were treated with either irinotecan 10 mg/kg (15 doses, on days 1-5, 8-12 and 29-33), by the intraperitoneal (i.p.) route, or with a SN38- linker A- MiniAp4 (Ia) 30 mg/kg (9 doses, on days 1, 3, 5, 8, 10, 12, 29, 31, 33), intravenously (i.v.). This regimen was considered the maximum feasible number of i.v. injections in mice. Control mice were treated with saline i.p. on days 1-5, 8-12 and 29-33.

Mouse weight was measured until day 40 and mean and SD values are represented for each group in Fig. 19. Mice did not lose significant weight, compared to controls, as a result of SN38- linker A- MiniAp4 (Ia)treatments. Weight of SN38- linker A- MiniAp4 (Ia) (Ia) treated mice was similar to that of mice treated with clinically relevant dosages of irinotecan.

### Example 11: Comparative activity of conjugates of SN38, SN38 with MiniAp4 (SN38-linker A-MiniAp4, example 7) and SN38-linker B-THRre (comparative example 2).

The present inventors compared the activity of the new compound SN38 conjugated with MiniAp4 (SN38-linkerA-MiniAp4, example 7 also named G2B-001) and with SN38-linker B-THRre (comparative example 2). Original compound SN38 was used as reference. Cancer cell lines used in these experiments were pediatric gliomas including diffuse intrinsic pontine glioma HSJD-DIPG-007, and pediatric high-grade glioma HSJD-GBM-001.Briefly, 3000 cancer cells were cultured in 96 well plates and 24 h later the cells were exposed to compound compound (Ia) (concentration range 1-0.00000001 µM), SN38-Linker B-THRre (concentration range 1-0.00000001 µM), and SN38 (concentration range 1-0.00000001 µM). To add the drugs to the cells in culture, SN38-Linker A-MiniAp4 (Ia) and SN38-Linker B-THRre were prepared in culture medium from stock solutions at concentration 1 mg/mL in water. SN38 was prepared in culture medium from a stock solution at concentration 1 mg/mL in DMSO. The MTS assay was used to determine cell viability after 72 h incubation with the drugs. The concentrations of drug required to cause a reduction of 50% in cell proliferation (IC50 and 95% confidence intervals) were calculated with Graphpad Prism 8 software (La Jolla, CA). Activity curves were built for cells HSJD-DIPG-007 (Fig. 20) and HSJD-GBM-001 (Fig. 21).The established IC50 values are in **Table 4**. As it can be observed, SN38 conjugation to THRre resulted in less activity than conjugation to MiniAp4.

**Table 4. IC50 values of compounds**

| **Cell line** | **SN38** | **SN38-LinkerA-MiniAp4 (Ia)** | **Comparative example 2** |
|---|---|---|---|
| HSJD-DIPG-007 | 0.001505 | 0.01071 | 0.1039 |
| HSJD-GBM-001 | 0.006306 | 0.006387 | 0.06063 |

### Example 12. Comparative activity of compound SN38-Linker A-MiniAp4 (Ia), comparative example 1 (SN38-Linker A-THRre) and comparative example 2 (SN38-Linker B-THRre) (Effect of the peptide and of both peptide and linker).

Because in previous Example 11 compound (Ia) and comparative example 2 (SN38-Linker B-THRre) were composed of linkers with different size, the present inventors compared the activity of both compounds with the one of comparative example 1 (SN38-Linker A-THRre), having compounds (Ia) and comparative example 1 the same linker.

Cancer cell lines used in these experiments were pediatric gliomas including diffuse intrinsic pontine glioma HSJD-DIPG-007, and pediatric high-grade glioma HSJD-GBM-001. Briefly, 3000 cancer cells were cultured in 96 well plates and 24 h later the cells were exposed to compounds (Ia), comparative example 1 and comparative example 2 (concentration range 1-0.001 µM for each of them). All compounds were prepared in culture medium from stock solutions at concentration 1 mg/mL in water.The MTS assay was used to determine cell viability after 72 h incubation with the drugs. The concentrations of drug required to cause a reduction of 50% in cell proliferation (IC50 and 95% confidence intervals) were calculated with Graphpad Prism 8 software (La Jolla, CA). Activity curves were built for cells HSJD-DIPG-007 (FIG. 22) and HSJD-GBM-001 (FIG. 23). Comparative example 1 (SN38-linker A-THRre) and comparative example 2 (SN38-linker B-THRre), presented less activity than compound (Ia). Thus, the surprising activity of compound (Ia) was due to the peptide MiniAp4, but also due to the space linker, compared to comparative example. 1 and comparative example 2 The established IC50 values are in **Table 5.**

**Table 5. IC50 values (µM) of compounds (Ia), Comp. Ex.3 and Comp. Ex. 4 against cancer cell lines.**

| **Cell line** | **SN38-LinkerA-MiniAp4 (Ia)** | **Comparative Example 1** | **Comparative Example 2** |
|---|---|---|---|
| HSJD-DIPG-007 | 0.02032 | 0.07509 | 0.1485 |
| HSJD-GBM-001 | 0.02165 | 0.06734 | 0.1008 |

### Example 13: Stability of SN38-linker A-MiniAp4 in human serum at 37°C in vitro versus time.

Regarding to the stability in human serum of the SN38-linker A-MiniAp4 (Ia) (example 7), it was incubated at a concentration of 200 µM in buffer HBSS at 37 °C in the presence of 90% human serum. At a range of times, 100 µL aliquots were collected to which 400 µL cold methanol was added in order to precipitate the serum proteins. The samples were centrifuged at 3000 rpm at 4°C during 30 min, filtered and analyzed by UPLC-PDA and UPLC-MS to determine the degree of degradation of SN38-Linker A-MiniAp4 (Ia). No significant degradation was observed in 24 h, which implies that the half-life is longer than this timeframe (FIG 24).

### Example 14: Stability of SN38-linker A-MiniAp4 (G2B-001) (Ia) in rat, mouse, dog and human plasma at 37°C in vitro versus time.

To assess the stability of the SN38-linker A-MiniAp4 (Ia) (example 7) in human, mouse, dog and rat plasma at 37°C, the compound was prepared at 200 µM in plasma samples in triplicates at each timepoint (0h, 1h, 4h, 8h and 24h) for each species. Once the incubation was finalised, plasma proteins were immediately precipitated by adding chilly methanol (ratio 4:1, methanol:plasma) and tubes were shacked for few seconds (vortex). Samples were centrifuged at 3,000 rpm for 30 minutes at 4°C, supernatants were collected, transferred to the injection plate and injected to LC-MS system.

Compound recovery was determined by comparing the analyte response in a biological sample that is spiked with the analyte and processed, with the response in a methanol sample that is spiked with the analyte and processed.

Plasma stability assay was performed using blank plasma from untreated volunteers / animals. K2-EDTA was used as anticoagulant. Human blank plasma used was obtained from Hospital Sant Pau, mouse blank plasma used was obtained from Janvier, dog blank plasma used was obtained from Isoquimen and rat blank plasma used were obtained from Draconis Pharma.

Half-life of G2B-001 at 200µM in human, mouse, dog and rat plasma was higher than 24 hours, except for compound G2B-001 in mouse and dog plasma that half-life was 8 - 24h. **(****FIG. 25****).**

**Table 6. Percentage of recovery of G2B-001 after 24 h in human, mouse, dog and rat plasma.**

| **G2B-001 (Ia)** | **% Recovery** |
|---|---|
| Human | 65.1 |
| Dog | 68.3 |
| Rat | 61.2 |
| Mouse | 57.1 |

### Example 15: Preparation of Hexynoic-TTDS-Dap-Lys-Ala-Pro-Glu-Thr-Ala-Leu-Asp-NH₂ (hexynoic-TTDS-SEQ ID NO: 14) lineal without an amide bond between Dap side-chain amino group and Asp side-chain carboxilic acid. DapKAPETALD (SEQ ID NO:14), i.e linear peptide.

For the manual coupling of the first protected amino acid to the resin, the coupling method 4 was applied using Fmoc-Asp(OAI)-OH (118.5 mg). The subsequent amino acids were coupled sequentially as follows using coupling method 4:

| **Add. order** | **Code** | **Amino acid** | **MW** | **m (mg)** | **eq** |
|---|---|---|---|---|---|
| 1 | D | Fmoc-L-Asp(OAI) -OH | 395.4 | 118.5 | 3 |
| 2 | L | Fmoc-L-Leu-OH | 353.4 | 105.9 | 3 |
| 3 | A | Fmoc-Ala-OH H₂O | 329.3 | 98.7 | 3 |
| 4 | T | Fmoc-L-Thr(tBu)-OH | 397.5 | 119.1 | 3 |
| 5 | E | Fmoc-Glu(OtBu)-OH H₂O | 443.5 | 132.9 | 3 |
| 6 | P | Fmoc-L-Pro-OH H₂O | 355.4 | 106.5 | 3 |
| 7 | A | Fmoc-Ala-OH H₂O | 329.3 | 98.7 | 3 |
| 8 | K | Fmoc-Lys(Boc)-OH | 468.5 | 140.4 | 3 |
| : 9 | Dap | Fmoc-L-Dap(Alloc)-OH | 410.4 | 123 | 3 |

Using 46 µL DIC and 43 mg Oxyma in DMF/DCM (1:1). The mixture was allowed to react with intermittent manual stirring for 45 min. After each coupling removal of the 9-fluorenylmethyloxycarbonyl (Fmoc) protecting group was done with 20% (v/v) piperidine in DMF using a treatment of 30 s followed by two treatments of 10 minutes each. Two additional treatments with DBU, toluene, piperidine, DMF (5%, 5%, 20%, 70%) (2 × 5 min) were performed to ensure the removal of the Fmoc group from secondary amines (proline).

Coupling of Fmoc-TTDS-OH:The coupling of the Fmoc-TTDS-OH (2 equivalents, 200 µmols, 108.53 mg), was achieved by_4 equivalents of DIC (400 µmols, 61 µL) and 4 of HOBt (400 µmols, 54 mg) in DCM during 2 h. Followed by removal of the 9-fluorenylmethyloxycarbonyl (Fmoc) protecting group with 20% (v/v) piperidine in DMF using a treatment of 30 s followed by two treatments of 10 minutes each.

Coupling of 5-hexynoic acid: For the coupling of the 5-hexynoic acid to the peptide anchored onto the resin the following protocol was used: hexynoic acid (4 eq., 400 µmols, 45 mg) in DMF (1-3 mL/g resin), PyBOP (4 eq., 400 µmols, 208 mg) and HOAt (12 eq., 1.2 mmols, 163 mg) were sequentially added to the resin followed by the addition of 12 eq. of DIEA (1.2 mmols, 204 µL). The mixture was allowed to react with intermittent manual stirring for 1.5 h. The solvent was removed by suction, the resin washed with DMF (5 × 30 s) and DCM (5 × 30 s). The coupling was repeated under the same conditions. The extent of coupling was monitored using the Kaiser colorimetric assay.

The OAI and Alloc groups were deprotected by addition of tetrakis(triphenylphosphine)palladium(0) (0.1 eq., 10 µM, 12 mg), phenyl silane (10 eq., 1000 µmols, 123 mg) in DCM (3 × 15 min). The resin was washed with 0.02 M sodium diethylcarbamate in DCM (3 × 5 min).

The peptide was then cleaved and liophilized. Product characterization. Reverse phase HPLC: linear gradient from 10 to 60% MeCN in H₂O in 30 min using a Xbridge 25 cm 3.5 µm column, 1 mL/min and MeCN (0.1% TFA) and H₂O (0.1% TFA) were used as solvents; Retention time: 11.526 min. Yield (synthesis and purification): 9%.

### Example 16: Preparation of compound of formula (Ib), G2B-001 lineal without an amide bond between Dap side-chain amino group and Asp side-chain carboxilic acid.

Starting from Hexynoic-TTDS-MiniAp4 lineal prepared as in example 15 and using SN38-N₃ prepared as in example 3, the following protocol was follow to achieve compound of formula (Ib) also named G2B-001 lineal:
To a microwave vial of 10 ml 1.5 eq. of *(S)-4, 11-diethyl-9-hydroxy-3,14-dioxo-3,4, 12,14-tetrahydro-1H-pyrano[3',4':6,7]indolizino[1,2-b]quinolin-4-yl 5-azidopentanoate,* 1 eq. of Alkyne-TTDS-DapKAPETALD lineal, 0.15 eq. of CuTHPTA and 0.3 eq. of sodium ascorbate were added and dissolved in 3 ml of DMF. The mixture was stirred from 2 to 4 hours with MW (CEM discover SP MW) assisted at 30°C during all reaction. The crude was purified by RP-HPLC at semi-preparative scale. It was obtained the compound with a purity higher than 95%. Product characterization: Reverse phase HPLC-MS: linear gradient from 0 to 80% MeCN in H₂O in 7 minutes using a Luna 3 µm C18 (2) 100A 50 × 2.1 mm column, 0.85 mL/min and MeCN (0.1% Formic acid) and H₂O (0.1% Formic acid) were used as solvents; Retention time: 5.08 min. UPLC-MS [M + H]ₑₓₚ⁺: 1845.10 Da; Yield (synthesis and purification): 22%.

### Example 17: Preparation of N₃-Pen-SN38

In a two neck round bottom flask, 1 eq. of SN-38 (600 mg) was added and dissolved in 100 ml per gram dry DCM (60ml). Turbid solution was observed. This reaction mixture was cooled to 0-2°C and 6 eq. of DIEA (2 ml) were added and stir for 15minutes. After 15 min, portion wise 3 eq. of N₃-Pen-Cl (700 mg) were added [dissolved in 500 uL dry DCM]. It was cooled for 15 mins and then stirring was continued at room temperature. Reaction progress was checked by HPLC. When starting material was below 3% the reaction was taken for work up.

Initial volume of reaction mixture ~45 ml which was further made-up to 300 ml using dry DCM. It was extracted thrice with distilled water (20% volume of final volume of reaction mixture) 3 X 3min X 60ml. DCM layer was dried using sodium sulphate (Na₂SO₄) for 30 mins and evaporate on rotavapour. The solid obtained was treated with 80:20 Hex: Ether twice and kept for drying in desiccator. Yield after work up, 730 mg. Product characterization. Reverse phase HPLC: linear gradient from 10 to 90% MeCN in H₂O in 30 min using a SS column 250×4.6mm packed with C-18 silica gel for chromatography R (5µm), 1 mL/min and MeCN (0.1% TFA) and H₂O (0.1% TFA) were used as solvents; Retention time: 19.730 min. Mass observed = 518.20 Da, Mass calculated= 517.19Da.

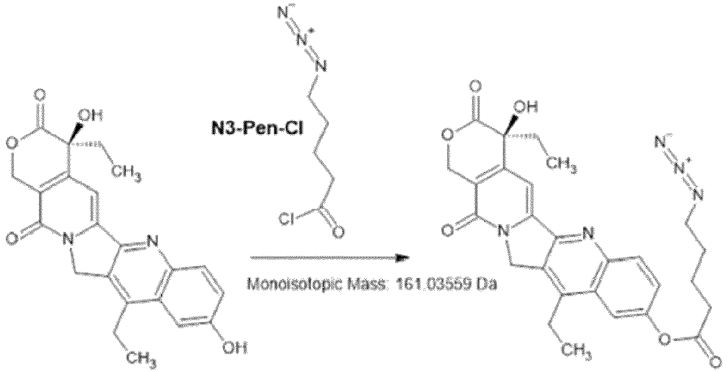

### Example 18: Preparation of compound of formula (Ic), G2B-003

Starting from Hexynoic-TTDS-MiniAp4 prepared as in example 6, and using N₃-Pen-SN38 prepared as in example 17, the following protocol was follow to achieve compound of formula (Ic) also named G2B-003.

To a vial of 10 ml 1.5 eq. of N3-Pen-SN38, 1 eq. of Hexynoic-TTDS-MiniAp4, 0.15 eq. of CuTHPTA and 0.3 eq. of sodium ascorbate were added and dissolved in 3 ml of DMF.

The mixture was stirred from 2 to 4 hours with Microwave (CEM discover SP MW) assisted at 30°C during all reaction. The crude was purified by RP-HPLC at semi-preparative scale. It was obtained the compound with a purity higher than 95%. Product characterization: Reverse phase HPLC: linear gradient from 20 to 70% MeCN in H₂O in 30 minutes using a using a Xbridge 25 cm 3.5 µm column, 1 mL/min and MeCN (0.1% TFA) and H₂O (0.1% TFA) were used as solvents; Retention time: 12.00 min. UPLC-MS [M + H]ₑₓₚ⁺: 1826.15 Da; Yield (synthesis and purification): 17%.

### Example 19: Preparation of SN38-O-CO-NH-Gly-COOH

A solution of glycine-COOBu^{t} (1 eq) and DMAP (2 eq) in dry DCM (15 mL) was added dropwise to a solution of bis(4-nitrophenyl) carbonate (1.3 eq.) in dry DCM (15 mL) and the resulting solution was stirred at 50 °C overnight. The reaction mixture was then diluted in DCM (150 mL) and washed with 0.5 N HCl (100 mL). The aqueous layer was washed with DCM (5 × 100 mL) and all the organic fractions were collected, dried over MgSO₄ and filtered. The solvent was evaporated under reduced pressure and the residue was purified by flash chromatography (Hexane:DCM:Et₂O; 5:4:1).

The resulting PNP-Gly-COOBu^{t} (1 eq) was reacted with SN38 (1.2 eq) in the presence of DMAP (2 eq) in dry DCM. The reaction was was stirred at 50 °C overnight. The solvent was evaporated under reduced pressure and the residue was purified by flash chromatography.

The t-butyl ester group was removed by treating SN38-O-CO-NH-Gly-COOBu^{t} with TFA:DCM:TIS (40:40:20) during 6 h. Afterwards the solvent was evaporated. Toluene was added twice to remove the traces of TFA. No further purification was performed.

### Example 20: Preparation of Boc-Val-Cit-PAB-SN38

In a round bottom flask, 1 eq of 7-ethyl-10-hydroxy-camptothecin (SN38), 1 eq of Boc-Val-Cit-PAB-PNP (from iris biotech), 2 eq of DIEA and catalitic DMAP were stirred in dry DMF for 16 h at r.t. Afterwards, the reaction mixture was diluted with AcOEt and washed with HCl (0.5 M) (x3), and brine (x4). Then it was dried with MgSO₄, evaporated the volatiles the crude mixture was purified by silica cromatography (AcOEt: MeOH; 20:1; 15:1: 9:1). The desired compound was obtained with a yield of 52% and purity higher than 90%. Yield 52%. Reverse phase UPLC-PDA: linear gradient from 0 to 100% MeCN in H₂O in 2 minutes using a Acquity BEH C18 (50 × 2 mm × 1.7 µm) column, 0.61 mL/min and MeCN (0.036% TFA) and H₂O (0.045% TFA) were used as solvents; Retention time: 1.42 min. [M+H]ₑₓₚ⁺: 898.4 Da.

### Example 21: Preparation of H₂N-Val-Cit.PAB-SN38

In a round bottom flask, 1 eq of Boc-Val-Cit- PAB-SN38 prepared as in example 20 was stirred in a mixture of DCM:TFA:H₂0 (44.75:49.75:0.5) during 5 minutes. Afterwards the solvent was evaporated. Toluene was added twice to remove the traces of TFA. No further purification was performed.

### Example 22: Preparation of G2B-004 (Id)

G2B-004 was prepared starting from diglycolic-MiniAp4 (prepared using standard methods described above, coupling method 4) and 2 eq H₂N-Val-Cit-PAB-SN38 (prepared as in example 21) using PyBOP (4 eq), HOAt (12 eq) dissolved in DMF (1-3 mL/g resin) followed by addition of DIEA (12 eq). The mixture was allowed to react with intermittent manual stirring for 1 h. The solvent was removed by suction and the resin washed with DMF (5 × 30 s) and DCM (5 × 30 s). The coupling reaction was carried out twice (1h and overnight).

G2B-004 was then cleaved and liophilized. Product characterization. Reverse phase UPLC: linear gradient from 0 to 100% MeCN in H₂O in 2 min using a Acquity BEH C18 (50 × 2 mm × 1.7 µm) column, 0.61 mL/min and MeCN (0.036% TFA) and H₂O (0.045% TFA) were used as solvents; Retention time: 1.17 min. UPLC-MS [M + H]ₑₓₚ⁺: 1807.84 Da. Yield (synthesis and purification): 21%.

### Example 23: Preparation of G2B-005 (le)

Nval-Pro-Gly-MiniAp4 was prepared using standard methods described above (coupling method 4).

G2B-005 was prepared starting from Nval-Pro-Gly-MiniAp4 and 2 eq SN38-O-CO-NH-Gly-OH prepared as in example 19 using PyBOP (4 eq), HOAt (12 eq) dissolved in DMF (1-3 mL/g resin) followed by addition of DIEA (12 eq). The mixture was allowed to react with intermittent manual stirring for 1 h. The solvent was removed by suction and the resin washed with DMF (5 × 30 s) and DCM (5 × 30 s). The coupling reaction was carried out twice (1h and overnight).

G2B-005 was then cleaved and liophilized. Product characterization. Reverse phase UPLC: linear gradient from 0 to 100% MeCN in H₂O in 2 min using a Acquity BEH C18 (50 × 2 mm × 1.7 µm) column, 0.61 mL/min and MeCN (0.036% TFA) and H₂O (0.045% TFA) were used as solvents; Retention time: 1.51 min. UPLC-MS [M + H]ₑₓₚ⁺: 1640.77 Da. Yield (synthesis and purification): 18%.

### Example 24: Stability of G2B-001 lineal (Ib) in human plasma at 37°C in vitro versus time

To assess the stability of the G2B-001 lineal (lb) (example 16) in human plasma at 37°C, the compound was prepared at 200 µM in plasma samples in triplicates at each timepoint (0h, 0.5h, 1h, 2h, 4h, 8h and 24h). Once the incubation was finalised, plasma proteins were immediately precipitated by adding chilly methanol (ratio 4:1, methanol: plasma) and tubes were shacked for few seconds (vortex). Samples were centrifuged at 3,000 rpm for 30 minutes at 4°C, supernatants were collected, transferred to the injection plate and injected to LC-MS system.Compound recovery was determined by comparing the analyte response in a biological sample that is spiked with the analyte and processed, with the response in a methanol sample that is spiked with the analyte and processed.

Plasma stability assay was performed using blank plasma from untreated volunteers. K2-EDTA was used as anticoagulant. Human blank plasma used was obtained from Hospital Sant Pau.

Half-life of G2B-001 lineal (lb) at 200µM in human was higher than 24 hours. **(****FIG. 26****).**

**Table 7. Percentage of recovery of G2B-001 lineal (lb) after 24 h in human plasma.**

| **G2B001 lineal** | **% Recovery** |
|---|---|
| Human | 69.9 |

### Example 25: Antitumoral activity of the new compound (lb) named G2B-001 lineal against cancer cell lines: diffuse intrinsic pontine glioma

The present inventors compared the activity of the new compound (lb) G2B-001 lineal (example 16) with the one of the closely related drugs, G2B-001 (Ia) and SN38.

Cancer cell line used in these experiment was HSJD-DIPG-007. Briefly, 3000 cancer cells were cultured in 96 well plates and 24 h later the cells were exposed to compounds G2B-001 (Ia), compound G2B-001 lineal (Ib), and SN-38 (concentration range 1-0.001 µM for each of them). The MTS assay was used to determine cell viability after 72 h incubation with the drugs. G2B-001 (Ia) and G2B-001 lineal (lb) showed similar activity (Figure 27). Thus, the in vitro activity of compound G2B-001 (Ia) was maintained after chemical modification to compound G2B-001 (Ib). As an internal reference of the experiment, SN-38 showed similar activity to the one reported in Example 9.The established IC50 values are in **Table 8.**

**Table 8. IC50 values (µM) of compounds G2B-001 (Ia), G2B-001 lineal (Ib), and SN38.**

| **Cell line** | **G2B-001 (Ia)** | **G2B-001 (Ib)** | **SN-38** |
|---|---|---|---|
| HSJD-DIPG-007 | 0.0278 | 0.0368 | 0.000739 |

### Example 26: Comparative antitumor activity of G2B-003 (Ic) and irinotecan in a patient-derived xenograft of neuroblastoma. Tumor model was HSJD-NB-013.

_We inserted subcutaneously freshly excised tumors (obtained from one mouse) in both flanks of three athymic nude mice. Upon engraftment (tumor volumes ranging 100-500 mm³), mice were treated with irinotecan, G2B-003 (Ib) or saline solution (control), all intravenous. One mouse received 10 doses of 10 mg/kg irinotecan at days 1, 2, 3, 4, 5, 8, 9, 10, 11 and 12. One mouse received 6 doses of 100 mg/kg G2B-003 (Ib) at days 1, 2, 3, 4, 5 and 8. One mouse received saline using the same regimen as irinotecan. Tumor volume was measured until day 44 or volume higher than 1500 mm³. Irinotecan and G2B-003 treatments achieved a measurable and long lasting antitumor response (Figure 28), while control tumors grew faster. Thus, in vivo activity of irinotecan and G2B-003 (Ic) were similar in this tumor model.

### Citation List

### Patent Literature

US8299089B2
WO2015/051307A1
WO2017/113687A2
WO2018/064683A1
WO2015/001015A1

### Non Patent Literature

F. Koizumi et al in "Novel SN38-incorporating polymeric micelles, NK012, eradicate vascular endothelial growth factor-secreting bulky tumors", Cancer Res 2006, vol. 66(20) pp. 10048-56
F Meyer-Losic et al in "DTS-108, A novel Peptidic profurg of DN38: In vivo Efficacy and Toxicokinetic Studies", Clinical Cancer Research 2008, vol.14, issue 7, pp. 2145-2153,
B. Oller et al., "MiniAp-4: A Venom-Inspired Peptidomimetic for Brain Delivery"; Angew Chem Int Ed 2016, vol.55, pp.572-575
M. Amblard, et al., "Methods and protocols of modern solid-phase peptide synthesis"; Molecular Biotechnology 2006, Vol. 33, pp. 239-254
J.H. Lee et al. Eur. J. Biochem. 2001. vol. 268. pp. 2004-2012
M. J. Hatfield and P. M. Potter, Exp. Opin. Ther. Pat. 2011, 21(8): 1159-1171.
P.L. Barker et al. J. Med. Chem. 1992. vol 35. pp. 2040-2048
E. Kaiser et al., Anal. Biochem. 1970, vol. 34, pp. 595-598
A. Madder et al., Eur. J. Org. Chem. 1999, pp. 2787-2791
Greene's Protective Groups in Organic Synthesis, Fifth Edition. Peter G. M. Wuts. 2014 John Wiley & Sons, Inc. Published 2014 by John Wiley & Sons, Inc. pp.26-29, 271-279, 456-463, 69-77, 371-374
M. Kazemi & L. Shiri, Journal of Sulfur Chemistry, 2015, 36:6, 613-623. here J. Org. Chem. 1998, 63, vol. 20, pp. 6878-6885.
M. T. Nguyen et al., J. Org. Chem. 1998, 63, vol. 20, pp. 6878-6885.
F. David et al., Org. Process Res. Dev. 2010, 14, 4, 999-1007.
https://www.cliffsnotes.com/study-guides/chemistry/organic-chemistry-ii/aldehydes-and-ketones/reactions-of-aldehydes-and-ketones
S.F.M. van Dongen et al.; Bioconjugate Chem. 2009, vol. 20, pp. 20-23.
https://www.lumiprobe.com/protocols/click-chemistry-dna-labeling
Mol. Pharmaceutics, 2016, 13, 379-390
M. Azzolini et al., Eur J Pharm Biopharm, 2017, vol. 115, pp.149-158
S. - J. et al. J. Med. Chem. 2008,vol. 51, pp. 6916-6926.

## Claims

1. A compound of formula (I) or a pharmaceutically acceptable salt thereof,
(Z)-(L)-P-(W)ₛ-(Y) (I)
wherein:
Z is a radical of the pharmaceutical active ingredient SN38 or a pharmaceutically acceptable salt thereof, wherein the pharmaceutical active ingredient SN38 has formula (II), and wherein Z is attached to a linker L independently by only one of the two hydroxyl groups (a) or (b) of the pharmaceutical active ingredient; 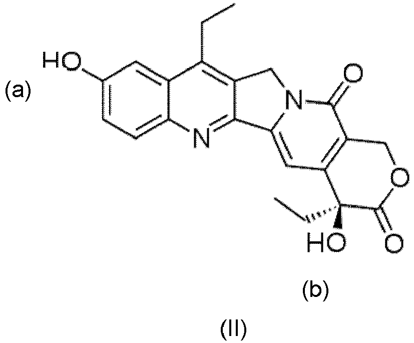
L is a linker which is a biradical composed from 2 to 8 biradicals L' and has the formula: -L'ₐ-(L'_{b})ₙ-L'_{c}-;
Lₐ' is a biradical selected from the group consisting of: -C(=O)-(CH₂)ᵣ-C(=O)-; -C(=O)-(CH₂)ᵣ-NH-; -C(=O)-(CH₂)ᵣ-S-; -C(=O)-(CH₂)ᵣ-O-; -C(=O)-NH-(CH₂)ᵣ-C(=O)-; -C(=O)-NH-(CH₂)ᵣNH-;-C(=O)-NH-(CH₂)ᵣ-S-; -C(=O)-NH-(CH₂)ᵣ-O-; -(CH₂)ᵣ-C(=O)-; -(CH₂)ᵣ-NH-; -(CH₂)ᵣ-S-; -(CH₂)ᵣ-O-; -Si(R₁)(R₂)-(CH₂)ᵣ-NH-; -Si(R₁)(R₂)-(CH₂)ᵣ-C(=O)-; -Si(R₁)(R₂)-(CH₂)ᵣ-O-; -Si(R₁)(R₂)-(CH₂)ᵣ-S-; -SO₂-(CH₂)ᵣ-NH-; -SO₂-(CH₂)ᵣ-C(=O)-; -SO₂-(CH₂)ᵣ-O-; -SO₂-(CH₂)ᵣ-S-; -P(=O)(OR₁)-O-(CH₂)ᵣ-NH-; -P(=O)(OR₁)-O-(CH₂)ᵣ-C(=O)-; -P(=O)(OR₁)-O-(CH₂)ᵣ-O-; -P(=O)(OR₁)-O-(CH₂)ᵣ-S-; -CH(OH)-(CH₂)ᵣ-NH-; -CH(OH)-(CH₂)ᵣ-C(=O)-; -CH(OH)-(CH₂)ᵣ-O-; -CH(OH)-(CH₂)ᵣ-S-; 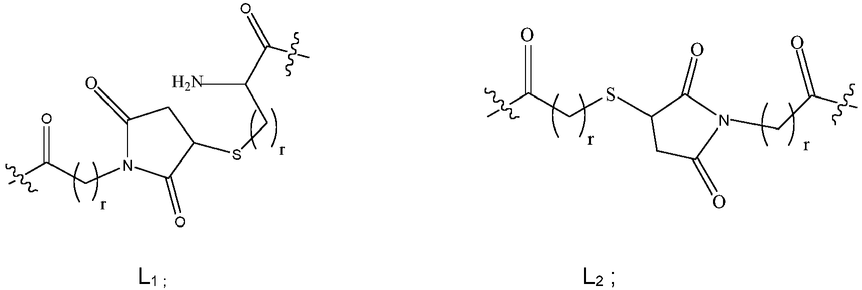 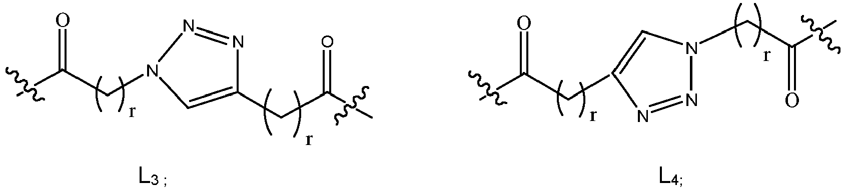 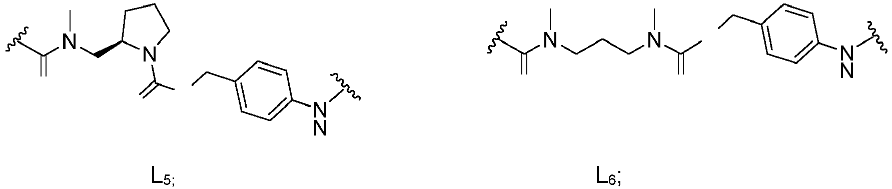 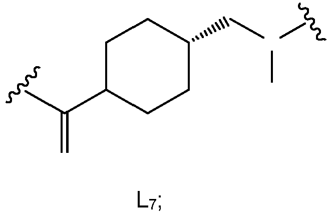 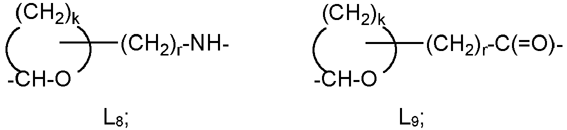 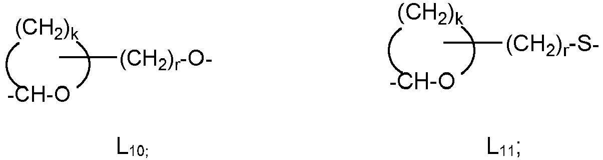
the substituent in any of L₈-L₁₁ can be in any position of the cycles; 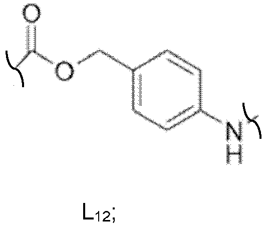
L_{b}' is a biradical independently selected from the group consisting of: -NH-(CH₂)ᵣ-C(=O)-; -C(=O)-(CH₂)ᵣ-C(=O)-; -S-(CH₂)ᵣ-C(=O)-; -O-(CH₂)ᵣ-C(=O)-; -NH-(CH₂)ᵣ; -C(=O)-(CH₂)ᵣ-; -S-(CH₂)ᵣ-;, -O-(CH₂)ᵣ; -NH-CH-((CH₂)ᵣNH₂)-C(=O)-; -S-CH₂-CH(NH₂)-C(=O)-; -(CH₂)ᵣ-C(=O)-; -(CH₂)ᵣ-O-; -(CH₂)ᵣ-NH-; -(CH₂)ᵣ-S-; -C(=O)-(CH₂)ᵣ-NH-; -C(=O)-(CH₂)ᵣ-O-; -C(=O)-(CH₂)ᵣ-S-; -NH-(CH₂)ᵣ-O-; -NH-(CH₂)ᵣ-NH-; -NH-(CH₂)ᵣ-S-; and combinations thereof; 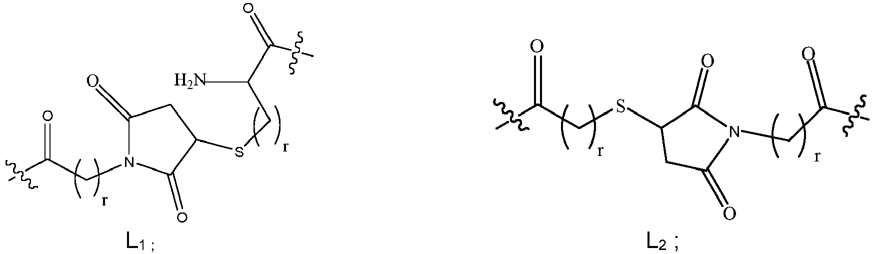 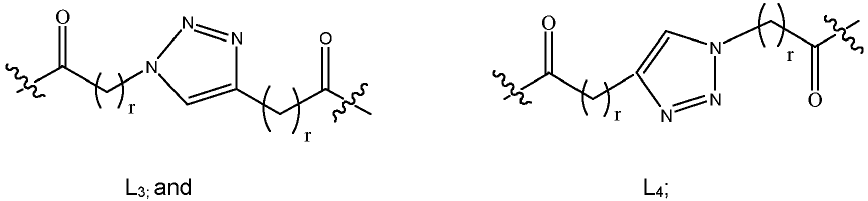
L_{c}' is a biradical selected from the group consisting of: -NH-(CH₂)ᵣ-C(=O)-; -NH-CH-((CH₂)ᵣNH₂)-C(=O)-; -C(=O)-(CH₂)ᵣ-C(=O)-; -S-(CH₂)ᵣ-C(=O)-; -S-CH₂-CH(NH₂)-C(=O)-; -O-(CH₂)ᵣ-C(=O)-, -(CH₂)ᵣ-C(=O)-; 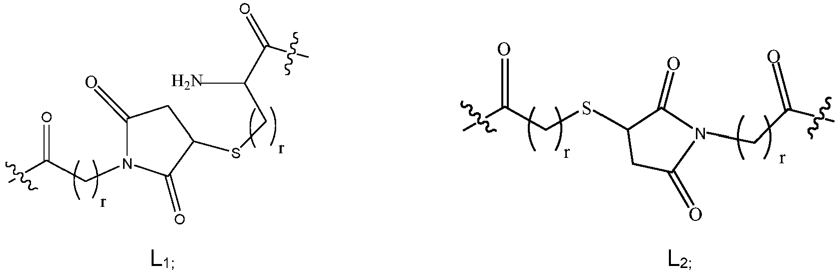 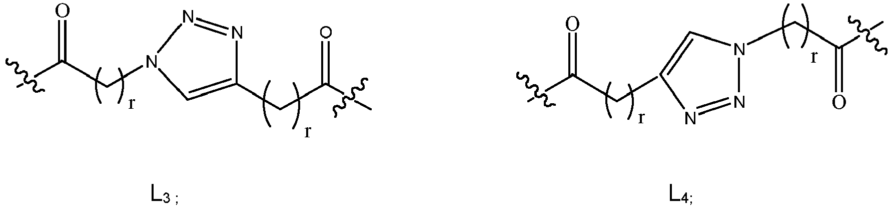 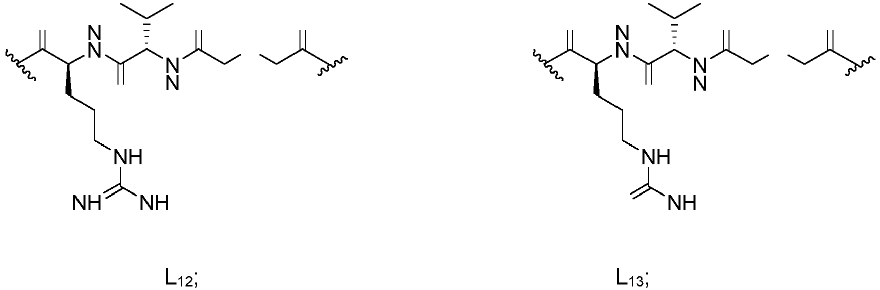 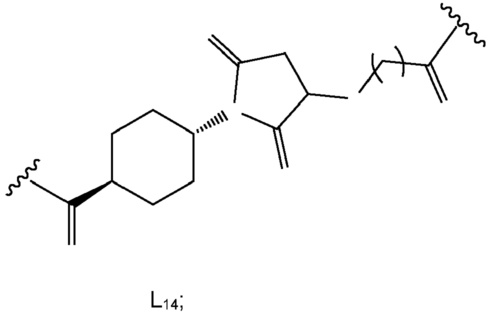 and 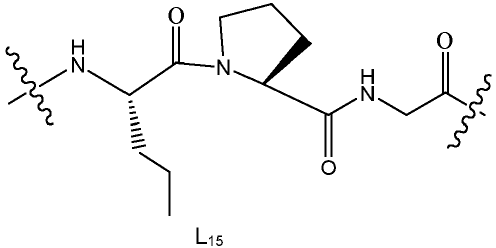
P is a biradical of a peptide selected from the group consisting of:
(a) a peptide which comprises the amino acid sequence X₁KAPETALX₂ with an intrapeptide bond between the X₁ and X₂ which is an amide bond; wherein X₁ is selected from the group consisting of Dap and Dab; and X₂ is selected from the group consisting of D (aspartic acid) and E (glutamic acid); i.e. 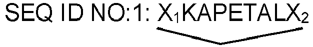
SEQ ID NO:1: X1KAPETALX2
(b) a peptide having 12-20 amino acids residues in length having at least an intrapeptide bond which is a disulfide or diselenide bond, and comprises an amino acid sequence which is: X₃KAPETALX₄AAA; having at least an intrapeptide disulfide or diselenide bond between X₃ and X₄, wherein X₃ and X₄ are equal and are selected from the group consisting of C (cysteines), Sec (selenocysteines), and Pen (penicillamines); 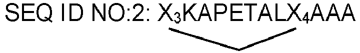
SEQ ID NO:2: X3KAPETALX4AAA
(c) a peptide having 9-11 amino acids residues in length having at least an intrapeptide bond which is a disulfide or diselenide bond and consists of an amino acid sequence selected from the group consisting of X₅KAPETALX₆; X₅KAPETALX₆A; and X₅KAPETALX₆AA having at least an intrapeptide disulfide or diselenide bond between X₅ and X₆; wherein X₅ and X₆ are equal and are selected from the group consisting of C (cysteines), Sec (selenocysteines), and Pen (penicillamines), i.e. 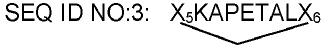
SEQ ID NO:3: X5KAPETALX6
 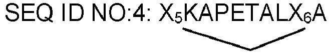
SEQ ID NO:4: X5KAPETALX6A
 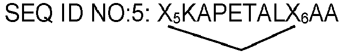
SEQ ID NO:5: X5KAPETALX6AA
(d) a peptide which has 16 amino acid residues and comprises the amino acid sequence X₇NX₈KAPETALX₉AAAX₁₀H with an intrapeptide disulfide or diselenide bond between the X₇ and X₉, and between X₈ and X₁₀; wherein X₇-X₁₀ are independently selected from the group consisting of C (cysteines), Sec (selenocysteines), and Pen (penicillamines); provided that X₇ and X₉ are equal, and X₈-X₁₀ are equal, i.e. 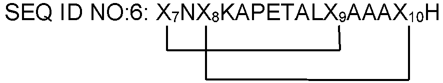
SEQ ID NO:6: X7NX8KAPETALX9AAAX10H
and (e) peptide which comprises the amino acid sequence X₁KAPETALX₂ wherein X₁ is selected from the group consisting of Dap and Dab; and X₂ is selected from the group consisting of D (aspartic acid) and E (glutamic acid) (SEQ ID NO:7);
W is a biradical selected from the group consisting of -NH-(CH₂)ᵣ₋C(=O)-, and -NH-CH((CH₂)ᵣNH₂)-C(=O)-;
Y is a radical is selected from the group consisting of -NH₂, -OH, -OR₃, and -NHR₃;
s is an integer independently selected from 0 to 1;
n is an integer from 0 to 6;
r is an integer independently selected from 1 to 5;
k is an integer from 5 to 8;
R₁ and R₂ are independently selected from an (C₁-C₆)-alkyl;
R₃ is a radical selected from the group consisting of (C₁-C₆)-alkyl;
Lₐ' is attached to the radical Z through a bond which is selected from the group consisting of an ester, ether, urethane, silyl ether, sulphonate, phosphate, ketal, hemiketal, carbonate, and carbamate bond, the bond being formed between the C=O, SO₂, Si, P, CH or CH₂ groups on the left side of the draw Lₐ' formulas and one of the hydroxyl groups of the SN38;
when n=0, Lₐ' is attached to the radical L_{c}' through a chemically feasible bond which is selected from the group consisting of amine, amide, ether, thioether, disulfide, ester, and thioester, the bond being formed between the functional groups on the right side of the draw Lₐ' formulas and the functional groups of the left side of the L_{c}' formulas;
when n=1, Lₐ' is attached to the radical L_{b}' through a chemically feasible bond which is selected from the group consisting of amine, amide, ether, thioether, disulfide ester, and thioester, the bond being formed between the functional groups on the right side of the draw Lₐ' formulas and the functional groups on the left side of the L_{b}' formulas; and L_{b}' is attached to the radical L_{c}' through a chemically feasible bond which is selected from the group consisting of amine, amide, ether, thioether, disulfide, ester, and thioester, the bond being formed between the functional groups on the right side of the draw L_{b}' formulas and the functional groups on the left side of the draw L_{c}' formulas;
when n is higher than 1, L_{b}' are equal or different and are attached among them through a chemically feasible bond selected from the group consisting of amine, amide, ether, thioether, disulfide, ester, and thioester; being one L_{b}' terminal attached to Lₐ' through a chemically feasible bond which is selected from the group consisting of amine, amide, ether, thioether, disulfide, ester, and thioester, the bond being formed between the functional groups on the right side of the draw Lₐ' formulas and the functional groups of the left side of the draw L_{b}' formulas; and being another L_{b}' terminal attached to L_{c}' through a chemically feasible bond which is selected from the group consisting of amine, amide, ether, thioether, disulfide, ester, and thioester, the bond being formed between the functional group on the right side of the draw L_{b}' formulas and the functional group on the left side of the draw L_{c}' formulas;
L_{c}' is attached to the to the biradical P through an amide bond formed with the carbonyl group on the right side of the draw L_{c}' formulas and an amino group of the first amino acid of the peptide sequence P;
when s=0, P is directly attached to Y through an amide, carboxylic acid or ester bond, the bond being formed between the C=O of the C-terminal of the last amino acid of the sequence P, and the radical Y which is -NH₂, -OH, -OR₃, or -NHR₃; and
when s=1 P is attached to a radical W through an amide bond formed with a C=O of the C-terminal of the last amino acid of the sequence P, the bond being formed between the functional groups on the left side of the draw W formulas and the functional groups (C=O) of the C-terminal of the last amino acid of the sequence P on the right side of the draw sequence; and W is attached to Y as follows: -C(=O)-NH-(CH₂)ᵣ₋C(=O)-Y, or -C(=O)-NH-CH((CH₂)ᵣNH₂)-C(=O)-Y.

2. The compound of formula (I) according to claim 1, wherein Z is attached to a linker L by the hydroxyl group (b) of the pharmaceutical active ingredient.

3. The compound of formula (I) according to claim 1, wherein Z is attached to a linker L by the hydroxyl group (a) of the pharmaceutical active ingredient.

4. The compound of formula (I) according to any of the claims 1-3, wherein P is a biradical of a peptide selected from the group consisting of:
(a) a peptide which comprises the amino acid sequence DapKAPETALD with an intrapeptide bond between the Dap and D which is an amide bond, that is SEQ ID NO:8: 
DapKAPETALD
NHCO
(b) a peptide having 9-20 amino acids residues in length having at least an intrapeptide bond which is a disulfide bond, and comprises an amino acid sequence which is:
CKAPETALCAAA having at least an intrapeptide disulfide bond between cysteines 1 and 9, that is 
SEQ ID NO:9: CKAPETALCAAA
S-S
(c) a peptide having 9-11 amino acids residues in length having at least an intrapeptide bond which is a disulfide bond and consists of an amino acid sequence selected from the group consisting of CKAPETALC; CKAPETALCA; and CKAPETALCAA having at least an intrapeptide disulfide bond between cysteines 1 and 9, that are 
SEQ ID NO:10: CKAPETALC
S-S
 
SEQ ID NO:11 CKAPETALCA
S-S
 
SEQ ID NO:12) CKAPETALCAA;
S-S
 and
(d) a peptide which has 16 amino acid residues and comprises the amino acid sequence CNCKAPETALCAAACH with an intrapeptide disulfide bond between the first and third cysteine which are cysteines 1 and 11, and between the second and the fourth cysteine which are cysteine 3 and 15, that is, 
SEQ ID NO:13: CNCKAPETALCAAACH
S-S
S-S
(e) a peptide which comprises the amino acid sequence DapKAPETALD (SEQ ID NO:14).

5. The compound of formula (I) according to claim 4, wherein, P is a biradical of a peptide selected from the group consisting of:
(a) the peptide having the amino acid sequence DapKAPETALD with an intrapeptide bond between the Dap and D which is an amide bond (SEQ ID NO:7);
(b) the peptide having the amino acid sequence CKAPETALC having at least an intrapeptide disulfide bond between cysteines in position 1 and 9 (SEQ ID NO:10;
(c) the peptide having the amino acid sequence DapKAPETALD (SEQ ID NO:14).

6. The compound of formula (I) according to any of the claims 1-5, wherein
Lₐ' is a biradical selected from the group consisting of: -C(=O)-(CH₂)ᵣ-C(=O)-, -C(=O)-(CH₂)ᵣ-NH-, -C(=O)-(CH₂)ᵣ-S-; -C(=O)-(CH₂)ᵣ-O-; -C(=O)-NH-(CH₂)ᵣ-C(=O)-; L₁, L₂, L₃, L₄, L₅, L₆, L₇ and L₁₂.

7. The compound according to any of the claims 1-6, wherein L is a linker which is a biradical composed from 3 to 8 biradicals and n is an integer from 1 to 6.

8. The compound according to claim 7, wherein Lₐ' is L₃ of formula below and L_{c}' is -C(=O)-(CH₂)ᵣ-C(=O)-,

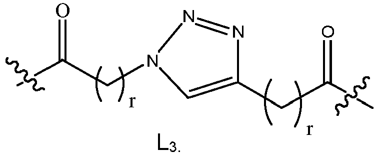

9. The compound according to claim 8, wherein L_{b}' is selected from the group consisting of -NH-(CH₂)ᵣ-O-, -(CH₂)ᵣ-O-; and -(CH₂)ᵣ-NH-, and combinations thereof.

10. The compound of formula (I) according to any of the claims 1-5, wherein:
a) L is a linker which is a biradical composed from 2 biradicals, n=0, Lₐ' is L₁₂, and L_{c}' is L₁₃; or alternatively,
b) L is a linker which is a biradical composed from 2 biradicals, n=0, Lₐ' is -C(=O)-NH-(CH₂)ᵣ-C(=O)-, and L_{c}' is L₁₅.

11. The compound according to claim 1, which is selected from the following list: 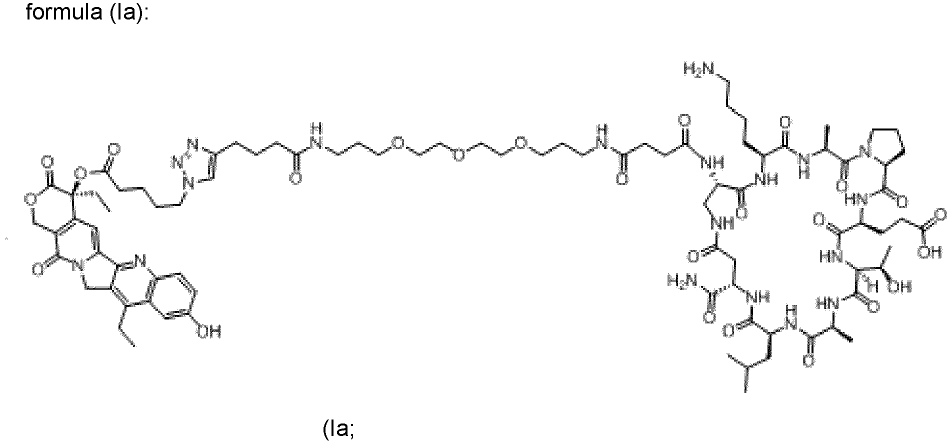 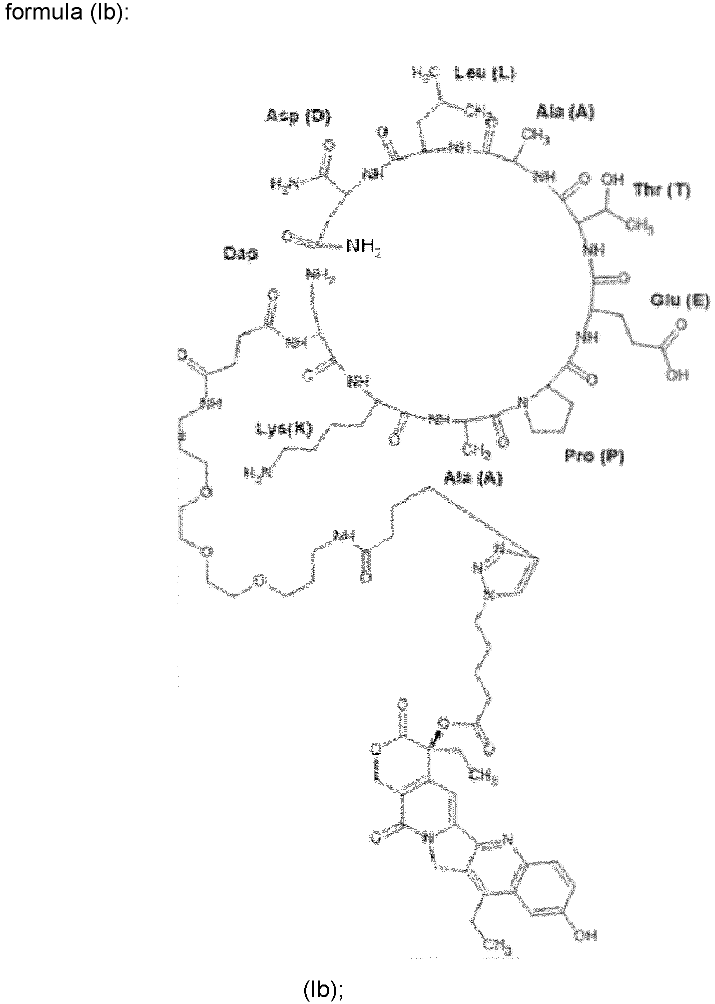 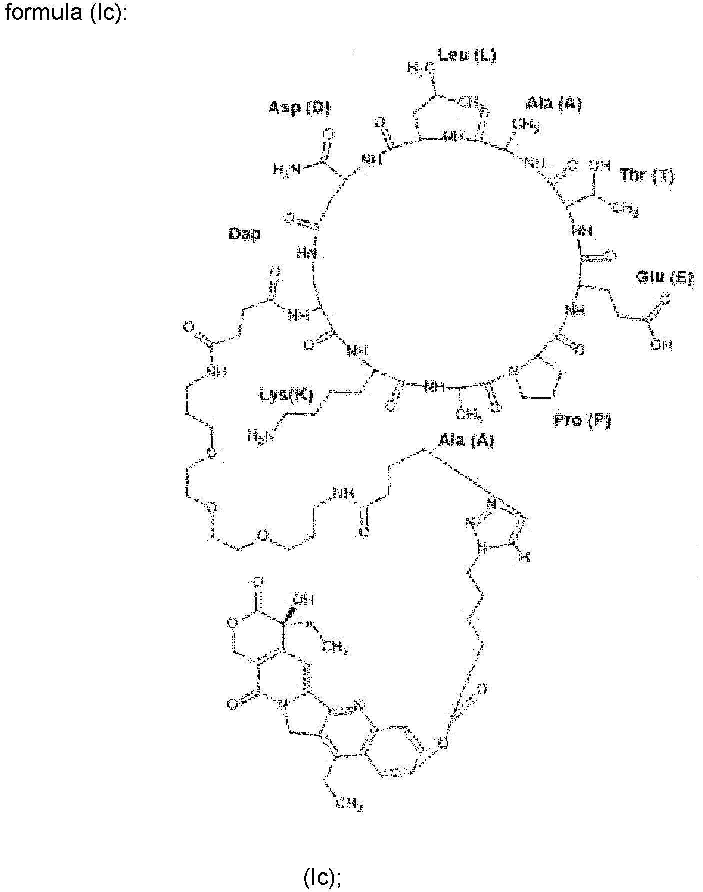 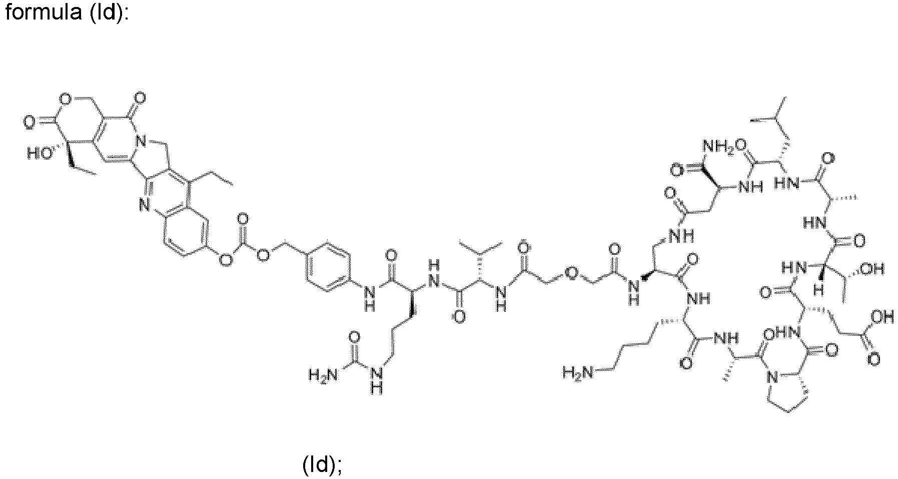 and

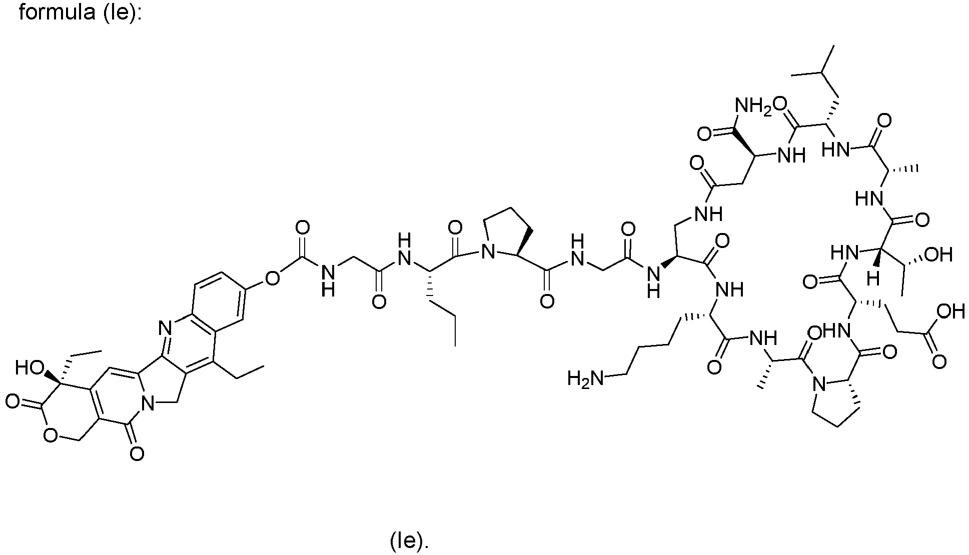

12. A pharmaceutical composition comprising a therapeutically effective amount of the compound as defined in any of the claims 1-11, together with appropriate amounts of pharmaceutically acceptable carriers or excipients.

13. A compound as defined in any of the claims 1-11, for use as a medicament.

14. A compound as defined in any of the claims 1-11, for use in the treatment of cancer in a mammal, including a human.

15. The compound for use according to claim 14, wherein the cancer treatment comprises the treatment of either a tumor selected from the group consisting of extracranial solid tumors, eye tumors, and CNS tumors; or a cancer selected from the group consisting of adult glioma, pediatric gliomas, retinoblastoma, Ewing sarcoma, DIPG, neuroblastoma, and rhabdomyosarcoma.

## Patentansprüche

1. Eine Verbindung der Formel (I) oder ein pharmazeutisch akzeptables Salz davon,
(Z)-(L)-P-(W)ₛ-(Y) (I)
wobei:
Z ein Rest des pharmazeutischen Wirkstoffs SN38 oder eines pharmazeutisch akzeptablen Salzes davon ist, wobei der pharmazeutische Wirkstoff SN38 die Formel (II) hat und wobei Z unabhängig über nur eine der beiden Hydroxylgruppen (a) oder (b) des pharmazeutischen Wirkstoffs an einen Linker L gebunden ist; 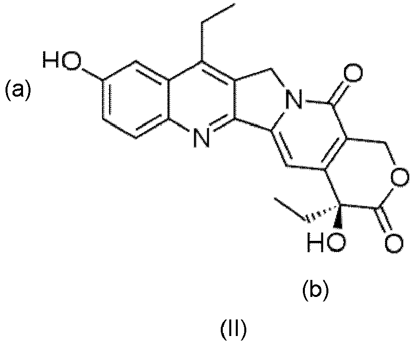
L ein Linker ist, welcher ein Biradikal ist, das aus 2 bis 8 Biradikalen L' zusammengesetzt ist und folgende Formel hat:
-L'ₐ-(L'_{b})ₙ-L'_{c}-;
Lₐ' ein Biradikal ist, das ausgewählt ist aus der Gruppe bestehend aus: -C(=O)-(CH₂)ᵣ-C(=O)-; -C(=O)-(CH₂)ᵣ-NH-; -C(=O)-(CH₂)ᵣ-S-; -C(=O)-(CH₂)ᵣ-O-; -C(=O)-NH-(CH₂)ᵣ-C(=O)-; -C(=O)-NH-(CH₂)ᵣNH-;-C(=O)-NH-(CH₂)ᵣ-S-; -C(=O)-NH-(CH₂)ᵣ-O-; -(CH₂)ᵣ-C(=O)-; -(CH₂)ᵣ-NH-; -(CH₂)ᵣ-S-; -(CH₂)ᵣ-O-; -Si(R₁)(R₂)-(CH₂)ᵣ-NH-; -Si(R₁)(R₂)-(CH₂)ᵣ-C(=O)-; -Si(R₁)(R₂)-(CH₂)ᵣ-O-; -Si(R₁)(R₂)-(CH₂)ᵣ-S-; -SO₂-(CH₂)ᵣ-NH-; -SO₂-(CH₂)ᵣ-C(=O)-; -SO₂-(CH₂)ᵣ-O-; -SO₂-(CH₂)ᵣ-S-; -P(=O)(OR₁)-O-(CH₂)ᵣ-NH-; -P(=O)(OR₁)-O-(CH₂)ᵣ-C(=O)-; -P(=O)(OR₁)-O-(CH₂)ᵣ-O-; -P(=O)(OR₁)-O-(CH₂)ᵣ-S-; -CH(OH)-(CH₂)ᵣ-NH-; -CH(OH)-(CH₂)ᵣ-C(=O)-; -CH(OH)-(CH₂)ᵣ-O-; -CH(OH)-(CH₂)ᵣ-S-; 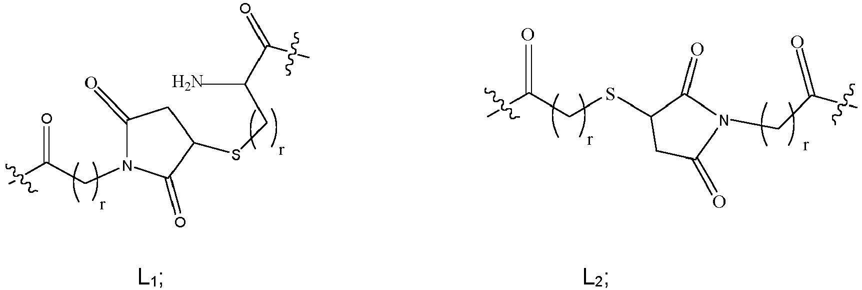 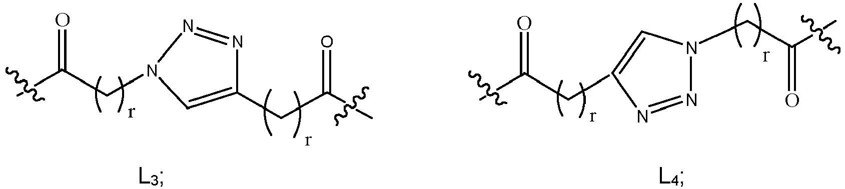 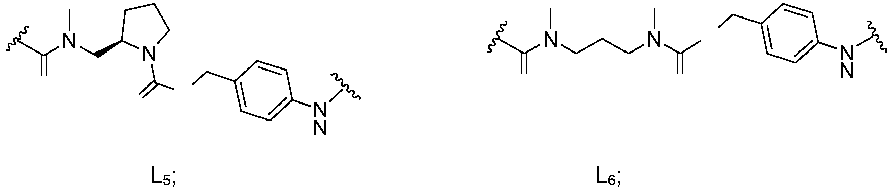 und 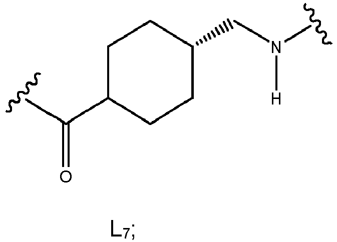 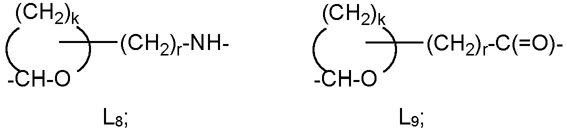 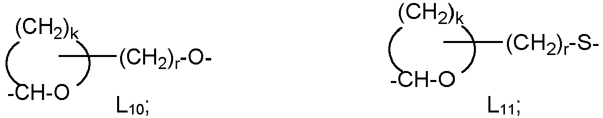
wobei der Substituent in jedem von L₈-L₁₁ sich in einer beliebigen Position der Zyklen befinden kann; 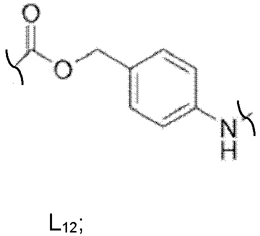
L_{b}' ein Biradikal ist, das unabhängig ausgewählt ist aus der Gruppe bestehend aus: -NH-(CH₂)ᵣ-C(=O)-; -C(=O)-(CH₂)ᵣ-C(=O)-; -S-(CH₂)ᵣ-C(=O)-; -O-(CH₂)ᵣ-C(=O)-; -NH-(CH₂)ᵣ-; - C(=O)-(CH₂)ᵣ-; -S-(CH₂)ᵣ-;, -O-(CH₂)ᵣ-; -NH-CH-((CH₂)ᵣNH₂)-C(=O)-; -S-CH₂-CH(NH₂)-C(=O)-; -(CH₂)ᵣ-C(=O)-; -(CH₂)ᵣ-O-; -(CH₂)ᵣ-NH-; -(CH₂)ᵣ-S-; -C(=O)-(CH₂)ᵣ-NH-; -C(=O)-(CH₂)ᵣ-O-; -C(=O)-(CH₂)ᵣ-S-; -NH-(CH₂)ᵣ-O-; -NH-(CH₂)ᵣ-NH-; -NH-(CH₂)ᵣ-S-; und Kombinationen davon; 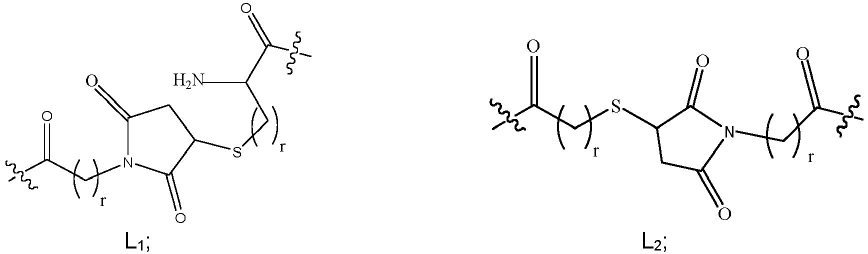 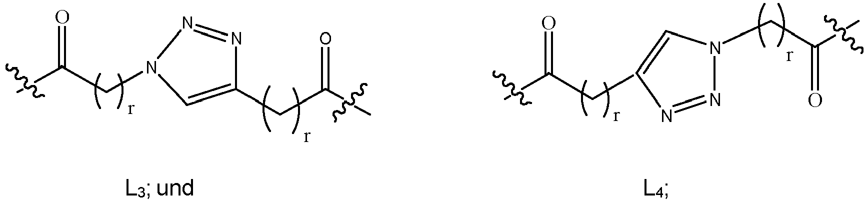
L_{c}' ein Biradikal ist, das ausgewählt ist aus der Gruppe bestehend aus: -NH-(CH₂)ᵣ-C(=O)-; -NH-CH-((CH₂)ᵣNH₂)-C(=O)-; -C(=O)-(CH₂)ᵣ-C(=O)-; -S-(CH₂)ᵣ-C(=O)-; -S-CH₂-CH(NH₂)-C(=O)-; -O-(CH₂)ᵣ-C(=O)-, -(CH₂)ᵣ-C(=O)-; 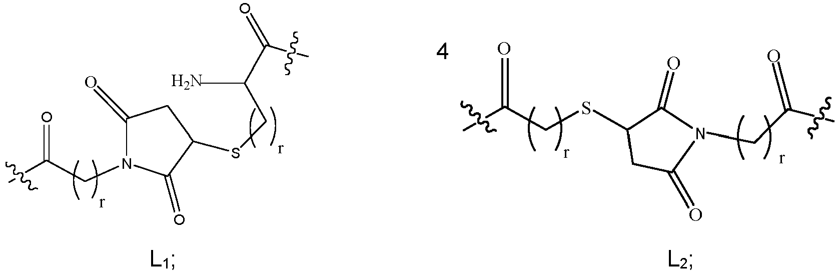 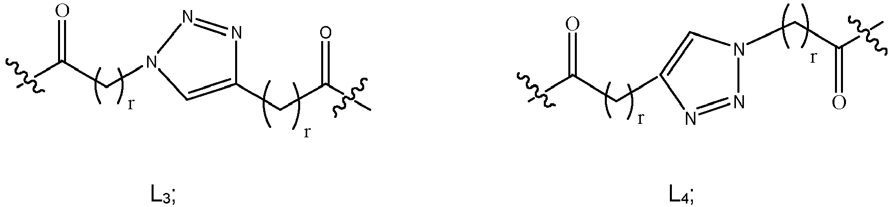 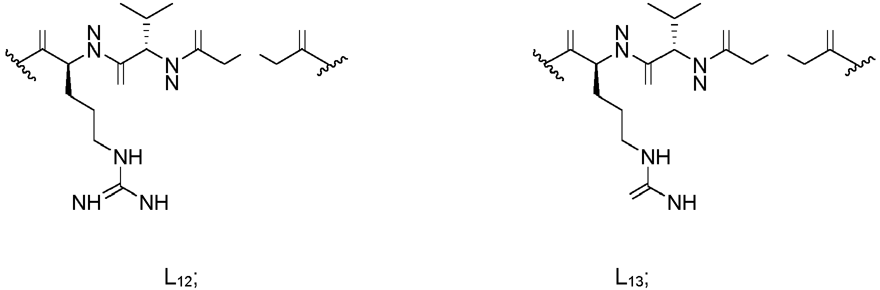 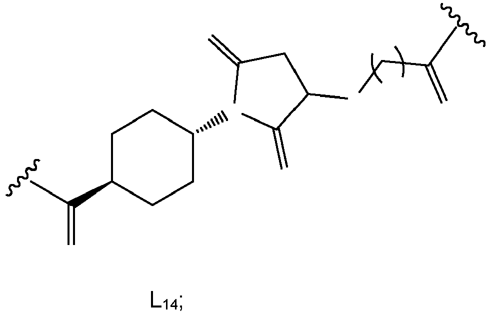 und 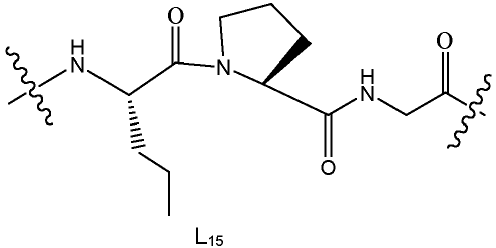
P ein Biradikal eines Peptids ist, das ausgewählt ist aus der Gruppe bestehend aus:
(a) einem Peptid, welches die Aminosäuresequenz X₁KAPETALX₂ mit einer intrapeptidischen Bindung zwischen X₁ und X₂ umfasst, welche eine Amidbindung ist; wobei X₁ ausgewählt ist aus der Gruppe bestehend aus Dap und Dab; und X₂ ausgewählt ist aus der Gruppe bestehend aus D (Asparaginsäure) und E (Glutaminsäure); d.h. 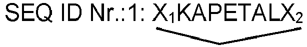
SEQ ID Nr.:1: X1KAPETALX2
(b) einem Peptid mit einer Länge von 12-20 Aminosäureresten, das mindestens eine intrapeptidische Bindung hat, welche eine Disulfid- oder Diselenidbindung ist, und eine Aminosäuresequenz umfasst, welche die Folgende ist: X₃KAPETALX₄AAA; mit mindestens einer intrapeptidischen Disulfid- oder Diselenidbindung zwischen X₃ und X₄, wobei X₃ und X₄ gleich sind und aus der Gruppe ausgewählt sind, die aus C (Cysteinen), Sec (Selenocysteinen) und Pen (Penicillaminen) besteht; 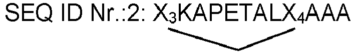
SEQ ID Nr.:2: X3KAPETALX4AAA
(c) einem Peptid mit einer Länge von 9-11 Aminosäureresten mit mindestens einer intrapeptidischen Bindung, welche eine Disulfid- oder eine Diselenidbindung ist und aus einer Aminosäuresequenz besteht, die ausgewählt ist aus der Gruppe bestehend aus X₅KAPETALX₆; X₅KAPETALX₆A; und X₅ KAPETALX₆AA mit mindestens einer intrapeptidischen Disulfid- oder Diselenidbindung zwischen X₅ und X₆; wobei X₅ und X₆ gleich sind und ausgewählt sind aus der Gruppe bestehend aus C (Cysteinen), Sec (Selenocysteinen) und Pen (Penicillaminen), d. h. 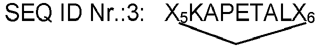
SEQ ID Nr.:3: X5KAPETALX6
 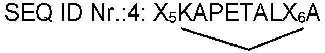
SEQ ID Nr.:4: X5KAPETALX6A
 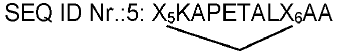
SEQ ID Nr.:5: X5KAPETALX6AA
(d) einem Peptid, das 16 Aminosäurereste hat und die Aminosäuresequenz X₇NX₈KAPETALX₉AAAX₁₀H mit einer intrapeptidischen Disulfid- oder -diselenidbindung zwischen X₇ und X₉, sowie zwischen X₈ und X₁₀ umfasst; wobei X₇-X₁₀ unabhängig voneinander aus der Gruppe ausgewählt sind, die aus C (Cysteinen), Sec (Selenocysteinen) und Pen (Penicillaminen) besteht; vorausgesetzt, dass X₇ und X₉ gleich sind und X₈-X₁₀ gleich sind, d. h. 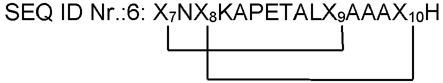
SEQ ID Nr.:6: X7NX8KAPETALX9AAAX10H
und (e) Peptid, welches die Aminosäuresequenz X₁KAPETALX₂ umfasst, wobei X₁ ausgewählt ist aus der Gruppe bestehend aus Dap und Dab; und X₂ ausgewählt ist aus der Gruppe bestehend aus D (Asparaginsäure) und E (Glutaminsäure) (SEQ ID Nr.:7);
W ein Biradikal ist, das ausgewählt ist aus der Gruppe bestehend aus -NH-(CH₂)ᵣ₋C(=O)-, und -NH-CH((CH₂)ᵣNH₂)-C(=O)-;
Y ein Rest ist, der ausgewählt ist aus der Gruppe bestehend aus -NH₂, -OH, -OR₃ und - NHR₃;
s eine ganze Zahl ist, die unabhängig von 0 bis 1 ausgewählt ist;
n eine ganze Zahl von 0 bis 6 ist;
r eine ganze Zahl ist, die unabhängig von 1 bis 5 ausgewählt ist;
k eine ganze Zahl von 5 bis 8 ist;
R₁ und R₂ unabhängig voneinander aus einem (C₁-C₆)-Alkyl ausgewählt sind;
R₃ ein Rest ist, der ausgewählt ist aus der Gruppe bestehend aus (C₁-C₆)-Alkyl;
Lₐ' an den Rest Z über eine Bindung gebunden ist, welche ausgewählt ist aus der Gruppe bestehend aus einer Ester-, Ether-, Urethan-, Silylether-, Sulfonat-, Phosphat-, Ketal-, Hemiketal-, Carbonat- und Carbamatbindung, wobei die Bindung zwischen den Gruppen C=O, SO₂, Si, P, CH oder CH₂ auf der linken Seite der gezeichneten Lₐ'-Formeln und einer der Hydroxylgruppen des SN38 entsteht;
wenn n=0, ist Lₐ' an den Rest L_{c}' über eine chemisch mögliche Bindung gebunden, welche aus der Gruppe ausgewählt ist, welche aus Amin, Amid, Ether, Thioether, Disulfid, Ester und Thioester besteht, wobei die Bindung zwischen den funktionellen Gruppen auf der rechten Seite der gezeichneten Lₐ'-Formeln und den funktionellen Gruppen der linken Seite der L_{c}'-Formeln entsteht;
wenn n=1, ist Lₐ' an den Rest L_{b}' über eine chemisch mögliche Bindung gebunden, welche aus der Gruppe ausgewählt ist, die aus Amin, Amid, Ether, Thioether, Disulfidester und Thioester besteht, wobei die Bindung zwischen den funktionellen Gruppen auf der rechten Seite der gezeichneten Lₐ'-Formeln und den funktionellen Gruppen auf der linken Seite der L_{b}'-Formeln entsteht; und L_{b}' ist an den Rest L_{c}' über eine chemisch mögliche Bindung gebunden, welche aus der Gruppe ausgewählt ist, die aus Amin, Amid, Ether, Thioether, Disulfid, Ester und Thioester besteht, wobei die Bindung zwischen den funktionellen Gruppen auf der rechten Seite der gezeichneten L_{b}'-Formeln und den funktionellen Gruppen auf der linken Seite der gezeichneten L_{c}'-Formeln entsteht;
wenn n größer als 1 ist, sind L_{b}' gleich oder verschieden und unter ihnen durch eine chemisch mögliche Bindung gebunden sind, die aus der Gruppe ausgewählt ist, die aus Amin, Amid, Ether, Thioether, Disulfid, Ester und Thioester besteht; wobei ein L_{b}'-Ende an Lₐ' durch eine chemisch mögliche Bindung gebunden ist, welche aus der Gruppe ausgewählt ist, die aus Amin, Amid, Ether, Thioether, Disulfide, Ester und Thioester besteht, wobei die Bindung zwischen den funktionellen Gruppen auf der rechten Seite der gezeichneten Lₐ'-Formeln und den funktionellen Gruppen auf der linken Seite der gezeichneten L_{b}'-Formeln entsteht; und ein anderes L_{b}'-Ende an L_{c}' durch eine chemisch mögliche Bindung gebunden ist, welche aus der Gruppe ausgewählt ist, die aus Amin, Amid, Ether, Thioether, Disulfid, Ester und Thioester besteht, wobei die Bindung zwischen der funktionellen Gruppe auf der rechten Seite der gezeichneten L_{b}'-Formeln und der funktionellen Gruppe auf der linken Seite der gezeichneten L_{c}'-Formeln entsteht;
L_{c}' an das Biradikal P über eine Amidbindung gebunden ist, die mit der Carbonylgruppe auf der rechten Seite der gezeichneten L_{c}'-Formeln und einer Aminogruppe der ersten Aminosäure der Peptidsequenz P entsteht;
wenn s=0, ist P direkt über eine Amid-, Carbonsäure- oder Esterbindung an Y gebunden, wobei die Bindung zwischen dem C=O des C-Endes der letzten Aminosäure der Sequenz P und dem Rest Y, welcher-NH₂, -OH, -OR₃ oder -NHR₃ ist, entsteht; und
wenn s=1, ist P über eine Amidbindung, die mit einem C=O des C-Endes der letzten Aminosäure der Sequenz P gebildet wird, an einen Rest W gebunden, wobei die Bindung zwischen den funktionellen Gruppen auf der linken Seite der gezeichneten Formeln W und den funktionellen Gruppen (C=O) des C-Endes der letzten Aminosäure der Sequenz P auf der rechten Seite der gezeichneten Sequenz entsteht; und W wie folgt an Y gebunden ist: -C(=O)-NH-(CH₂)ᵣ₋C(=O)-Y, oder -C(=O)-NH-CH((CH₂)ᵣNH₂)-C(=O)-Y.

2. Die Verbindung der Formel (I) nach Anspruch 1, wobei Z durch die Hydroxylgruppe (b) des pharmazeutischen Wirkstoffs an einen Linker L gebunden ist.

3. Die Verbindung der Formel (I) nach Anspruch 1, wobei Z durch die Hydroxylgruppe (a) des pharmazeutischen Wirkstoffs an einen Linker L gebunden ist.

4. Die Verbindung der Formel (I) nach einem der Ansprüche 1 bis 3, wobei P ein Biradikal eines Peptids ist, das ausgewählt ist aus der Gruppe bestehend aus:
(a) einem Peptid, welches die Aminosäuresequenz DapKAPETALD mit einer intrapeptidischen Bindung zwischen dem Dap und D umfasst, welche eine Amidbindung ist, das folgende Sequenz ist 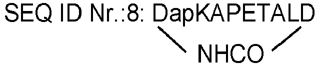
SEQ ID Nr.:8: DapKAPETALD
NHCO
(b) einem Peptid mit einer Länge von 9-20 Aminosäureresten, das mindestens eine intrapeptidische Bindung hat, welche eine Disulfidbindung ist, und eine Aminosäuresequenz umfasst, welche wie folgt ist: CKAPETALCAAA mit mindestens einer intrapeptidischen Disulfidbindung zwischen den Cysteinen 1 und 9, das folgende Sequenz ist 
SEQ ID Nr.:9: CKAPETALCAAA
S-S
(c) einem Peptid mit einer Länge von 9-11 Aminosäureresten mit mindestens einer intrapeptidischen Bindung, welche eine Disulfidbindung ist und aus einer Aminosäuresequenz besteht, die ausgewählt ist aus der Gruppe bestehend aus CKAPETALC; CKAPETALCA; und CKAPETALCAA mit mindestens einer intrapeptidischen Disulfidbindung zwischen den Cysteinen 1 und 9, das folgende Sequenzen sind 
SEQ ID Nr.:10: CKAPETALC
S-S
 
SEQ ID Nr.:11 CKAPETALCA
S-S
 
SEQ ID Nr.:12) CKAPETALCAA;
S-S
 und
(d) einem Peptid, das 16 Aminosäurereste hat und die Aminosäuresequenz CNCKAPETALCAAACH mit einer intrapeptidischen Disulfidbindung zwischen dem ersten und dem dritten Cystein, welche die Cysteine 1 und 11 sind, und zwischen dem zweiten und vierten Cystein, welche die Cystein 3 und 15 sind, umfasst, d. h. 
SEQ ID Nr.:13: CNCKAPETALCAAACH
S-S
S-S
(e) einem Peptid, welches die Aminosäuresequenz DapKAPETALD (SEQ ID Nr.:14) umfasst.

5. Die Verbindung der Formel (I) nach Anspruch 4, wobei P ein Biradikal eines Peptids ist, das ausgewählt ist aus der Gruppe bestehend aus:
(a) dem Peptid mit der Aminosäuresequenz DapKAPETALD mit einer intrapeptidischen Bindung zwischen der Dap und D, welche eine Amidbindung (SEQ ID Nr.:7) ist;
(b) dem Peptid mit der Aminosäuresequenz CKAPETALC mit mindestens einer intrapeptidischen Disulfidbindung zwischen den Cysteinen in Position 1 und 9 (SEQ ID Nr.:10;
(c) dem Peptid mit der Aminosäuresequenz DapKAPETALD (SEQ ID Nr.:14).

6. Die Verbindung der Formel (I) nach einem der Ansprüche 1 bis 5, wobei
Lₐ' ein Biradikal ist, das ausgewählt ist aus der Gruppe bestehend aus: -C(=O)-(CH₂)ᵣ-C(=O)-; -C(=O)-(CH₂)ᵣ-NH-, -C(=O)-(CH₂)ᵣ-S-; -C(=O)-(CH₂)ᵣ-O-; -C(=O)-NH-(CH₂)ᵣ-C(=O)-; L₁, L₂, L₃, L₄, L₅, L₆, L₇ und L₁₂.

7. Die Verbindung nach einem der Ansprüche 1 bis 6, wobei L ein Linker ist, welcher ein Biradikal ist, das aus 3 bis 8 Biradikalen zusammengesetzt ist, und n eine ganze Zahl von 1 bis 6 ist.

8. Die Verbindung nach Anspruch 7, wobei Lₐ' L₃ gemäß der untenstehenden Formel ist und L_{c}' -C(=O)-(CH₂)ᵣ-C(=O)- ist,

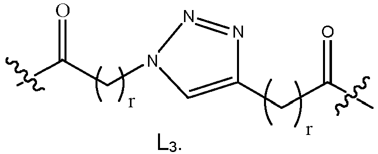

9. Die Verbindung nach Anspruch 8, wobei L_{b}' ausgewählt ist aus der Gruppe bestehend aus -NH-(CH₂)ᵣ-O-, -(CH₂)ᵣ-O-; und -(CH₂)ᵣ-NH- und Kombinationen davon.

10. Die Verbindung der Formel (I) nach einem der Ansprüche 1 bis 5, wobei
a) L ein Linker ist, welcher ein Biradikal ist, das aus 2 Biradikalen zusammengesetzt ist, n = 0, Lₐ' = L₁₂ ist und L_{c}' = L₁₃ ist; oder alternativ,
b) L ein Linker ist, welcher ein Biradikal ist, das aus 2 Biradikalen zusammengesetzt ist, n = 0, Lₐ' -C(=O)-NH-(CH₂)ᵣ-C(=O)- ist und L_{c}' = L₁₅ ist.

11. Die Verbindung nach Anspruch 1, welche ausgewählt ist aus der folgenden Liste:

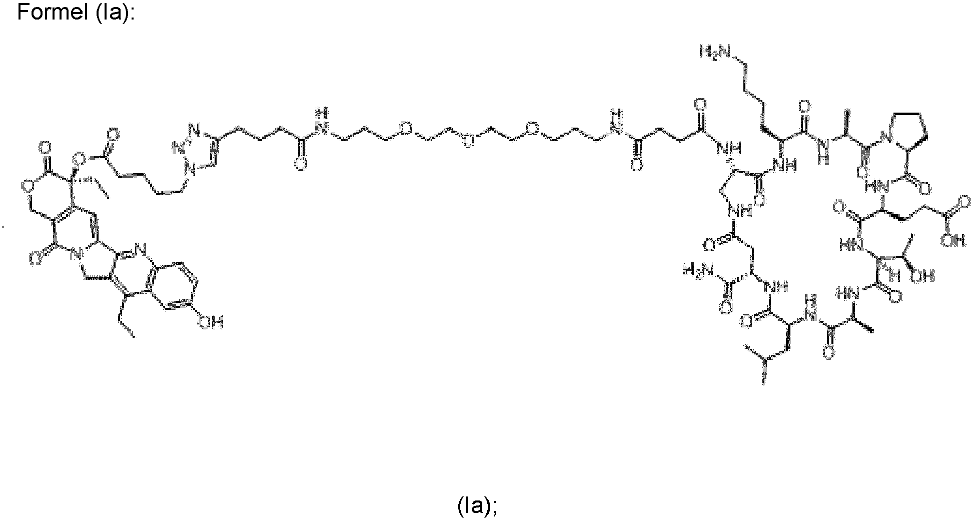

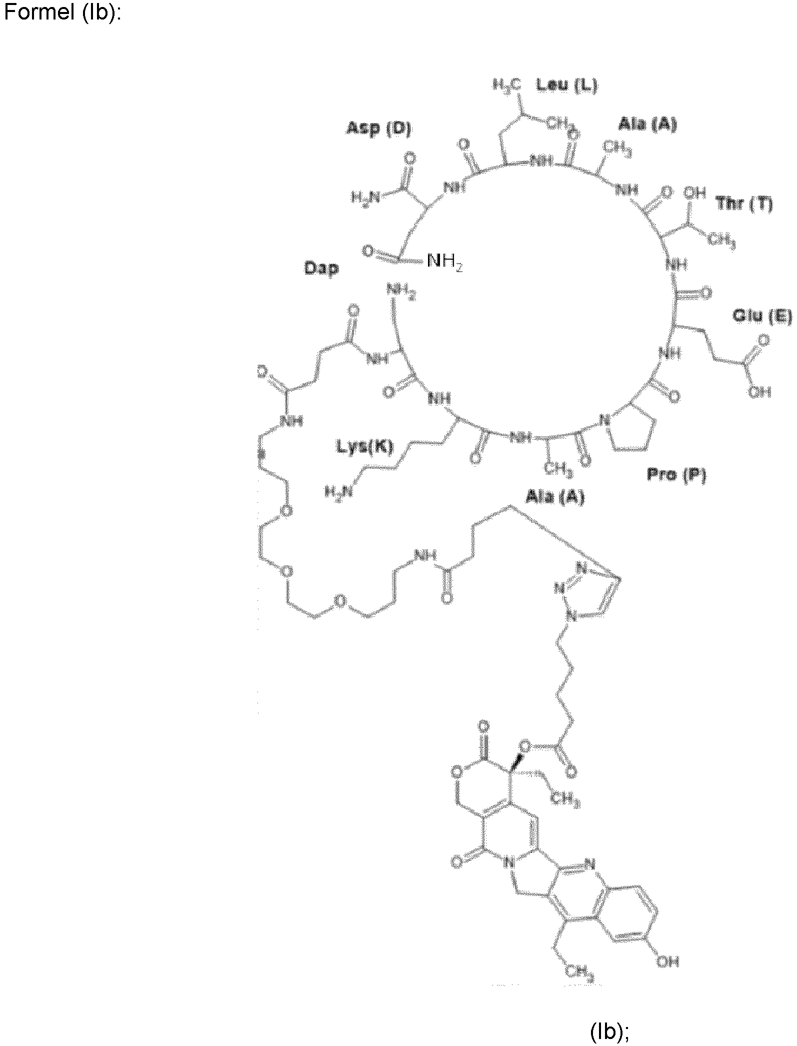

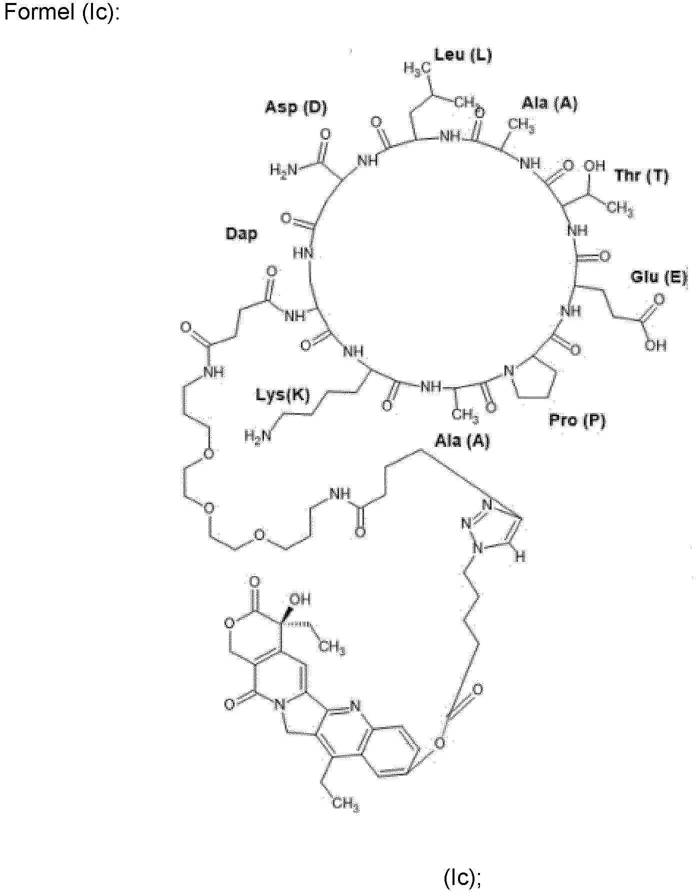

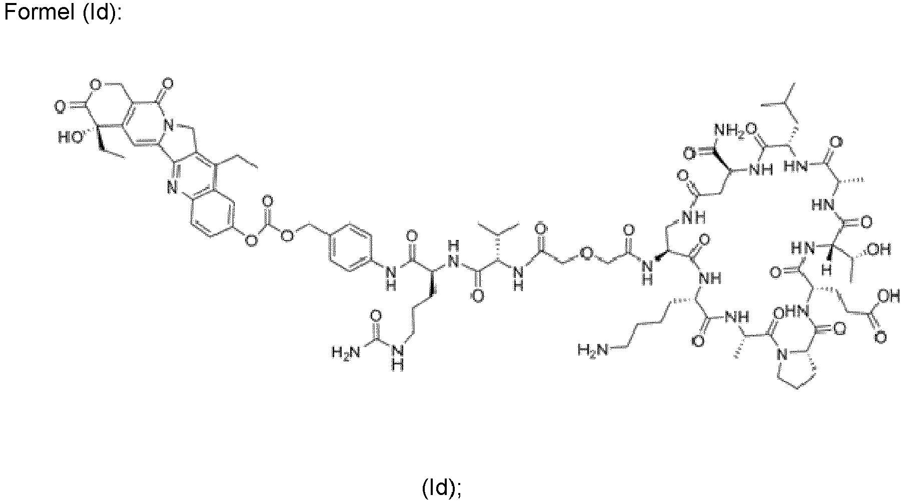

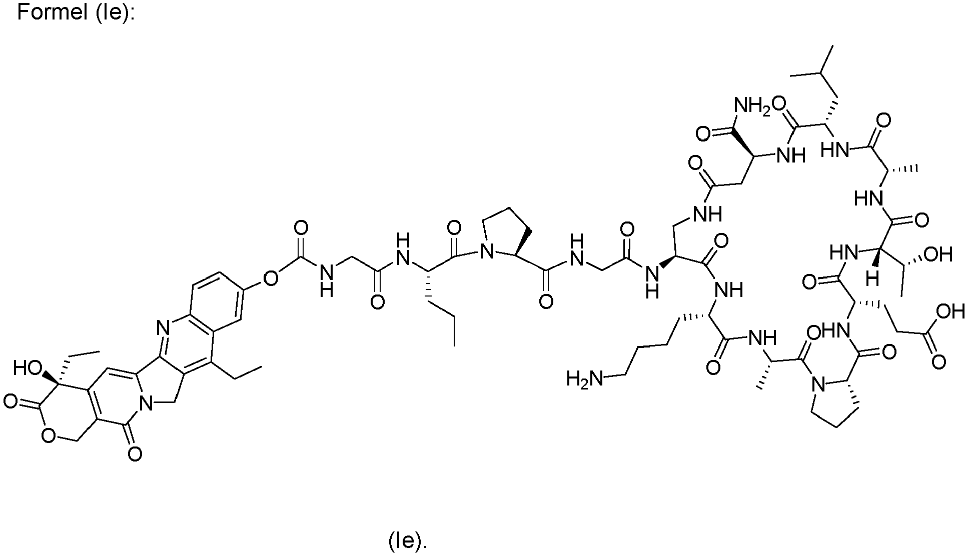

12. Eine pharmazeutische Zusammensetzung umfassend eine therapeutisch wirksame Menge der Verbindung wie in einem der Ansprüche 1 bis 11 definiert, zusammen mit geeigneten Mengen pharmazeutisch akzeptabler Trägersubstanzen oder Hilfsstoffe.

13. Eine Verbindung wie in einem der Ansprüche 1 bis 11 definiert, zur Verwendung als Arzneimittel.

14. Eine Verbindung wie in einem der Ansprüche 1 bis 11 definiert, zur Verwendung bei der Behandlung von Krebs bei einem Säugetier, einschließlich eines Menschen.

15. Die Verbindung zur Verwendung nach Anspruch 14, wobei die Krebsbehandlung die Behandlung von entweder einem Tumor, der ausgewählt ist aus der Gruppe bestehend aus extrakraniellen soliden Tumoren, Augentumoren und ZNS-Tumoren; oder einem Krebs, der ausgewählt ist aus der Gruppe bestehend aus Erwachsenengliom, pädiatrischen Gliomen, Retinoblastom, Ewing-Sarkom, DIPG, Neuroblastom und Rhabdomyosarkom, umfasst.

## Revendications

1. Un composé de formule (I) ou un sel pharmaceutiquement acceptable de celui-ci,
(Z)-(L)-P-(W)ₛ-(Y) (I)
dans laquelle :
Z est un radical de l'ingrédient actif pharmaceutique SN38 ou un sel pharmaceutiquement acceptable de celui-ci, dans lequel l'ingrédient actif pharmaceutique SN38 a la formule (II), et dans lequel Z est attaché à un lieur L indépendamment par un seul des deux groupes hydroxyle (a) ou (b) de l'ingrédient actif pharmaceutique ; 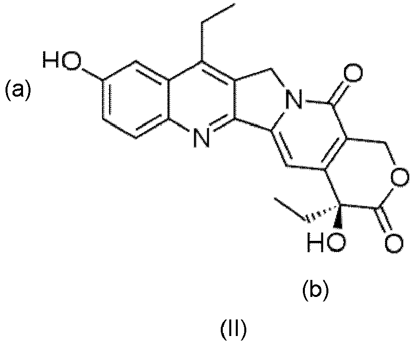
L est un lieur qui est un biradical composé de 2 à 8 biradicaux L' et qui a la formule :
-L'ₐ-(L'_{b})ₙ-L'_{c}-;
Lₐ' est un biradical choisi dans le groupe constitué de : -C(=O)-(CH₂)ᵣ-C(=O)-; -C(=O)-(CH₂)ᵣ-NH-; -C(=O)-(CH₂)ᵣ-S-; -C(=O)-(CH₂)ᵣ-O-; -C(=O)-NH-(CH₂)ᵣ-C(=O)-; -C(=O)-NH-(CH₂)ᵣNH-;-C(=O)-NH-(CH₂)ᵣS-; -C(=O)-NH-(CH₂)ᵣ-O-; -(CH₂)ᵣ-C(=O)-; -(CH₂)ᵣ-NH-; -(CH₂)ᵣ-S-; -(CH₂)ᵣ-O-; -Si(R₁)(R₂)-(CH₂)ᵣ-NH-; -Si(R₁)(R₂)-(CH₂)ᵣ-C(=O)-; -Si(R₁)(R₂)-(CH₂)ᵣ-O-; -Si(R₁)(R₂)-(CH₂)ᵣ-S-; -SO₂-(CH₂)ᵣ-NH-; -SO₂-(CH₂)ᵣ-C(=O)-; -SO₂-(CH₂)ᵣ-O-; -SO₂-(CH₂)ᵣ-S-; -P(=O)(OR₁)-O-(CH₂)ᵣ-NH-; -P(=O)(OR₁)-O-(CH₂)ᵣ-C(=O)-; -P(=O)(OR₁)-O-(CH₂)ᵣ-O-; -P(=O)(OR₁)-O-(CH₂)ᵣ-S-; -CH(OH)-(CH₂)ᵣ-NH-; -CH(OH)-(CH₂)ᵣ-C(=O)-; -CH(OH)-(CH₂)ᵣ-O-; -CH(OH)-(CH₂)ᵣ-S-; 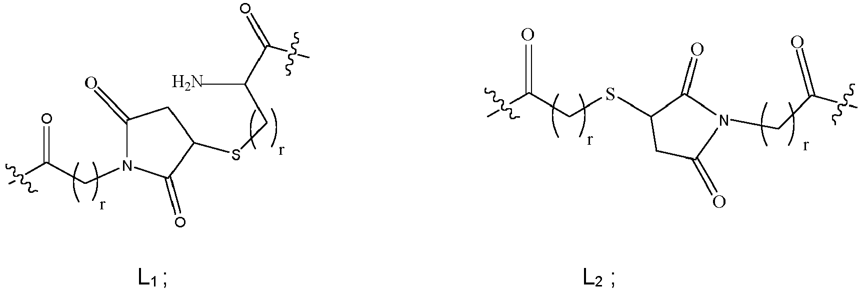 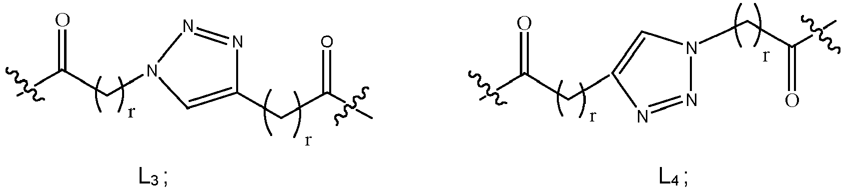 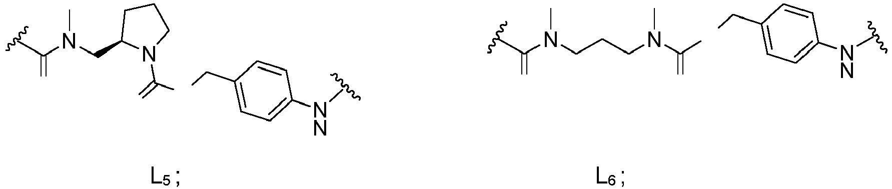 et 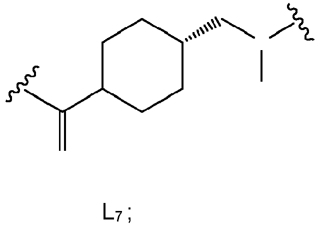 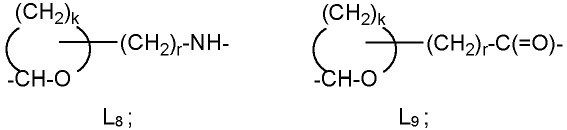 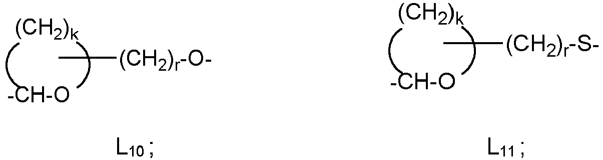
le substituant dans l'un quelconque des L₈ à L₁₁ peut être dans n'importe quelle position des cycles ; 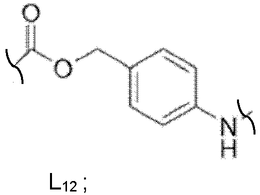
L_{b}' est un biradical choisi indépendamment dans le groupe constitué de : -NH-(CH₂)ᵣ-C(=O)- ; -C(=O)-(CH₂)ᵣ-C(=O)- ; -S-(CH₂)ᵣ-C(=O)- ; -O-(CH₂)ᵣ-C(=O)-; -NH-(CH₂)ᵣ- ; - C(=O)-(CH₂)ᵣ- ; -S-(CH₂)ᵣ- ;, -O-(CH₂)ᵣ- ; -NH-CH-((CH₂)ᵣNH₂)-C(=O)- ; -S-CH₂-CH(NH₂)-C(=O)- ; -(CH₂)ᵣ-C(=O)- ; -(CH₂)ᵣ-O- ; -(CH₂)ᵣ-NH- ; -(CH₂)ᵣ-S- ; -C(=O)-(CH₂)ᵣ-NH- ; - C(=O)-(CH₂)ᵣ-O- ; -C(=O)-(CH₂)ᵣ-S- ; -NH-(CH₂)ᵣ-O- ; -NH-(CH₂)ᵣ-NH- ; -NH-(CH₂)ᵣ-S- ; et des combinaisons de ceux-ci ; 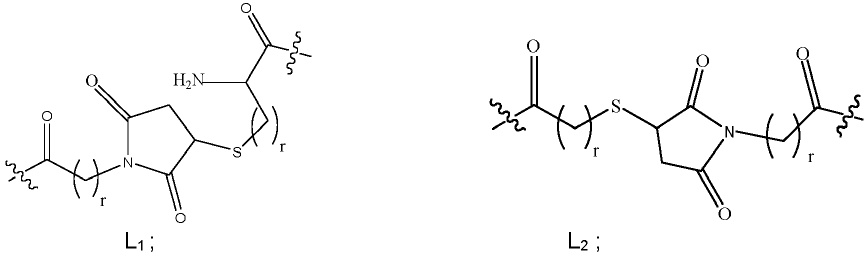 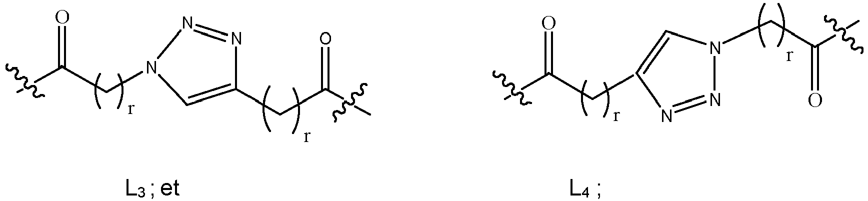
L'_{c} est un biradical choisi dans le groupe constitué de : -NH-(CH₂)ᵣ-C(=O)- ; -NH-CH-((CH₂)ᵣNH₂)-C(=O)- ; -C(=O)-(CH₂)ᵣ-C(=O)- ; -S-(CH₂)ᵣ-C(=O)- ; -S-CH₂-CH(NH₂)-C(=O)- ; -O-(CH₂)ᵣ-C(=O)-, -(CH₂)ᵣ-C(=O)- ; 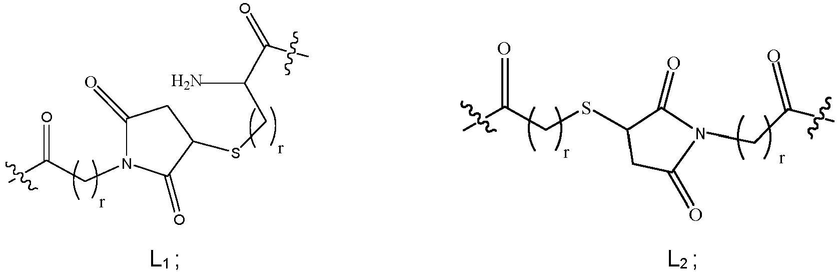 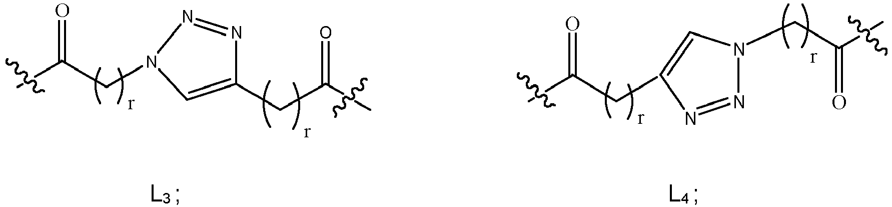 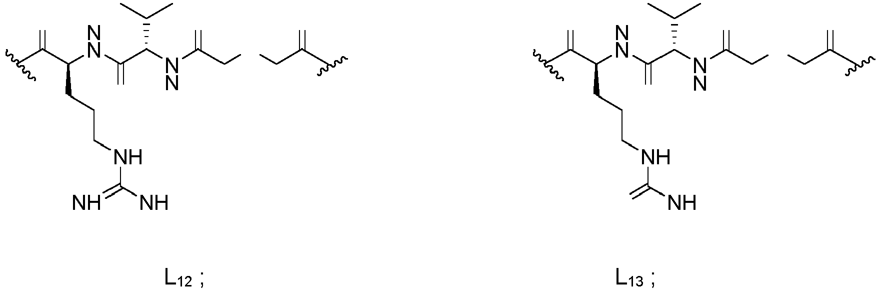 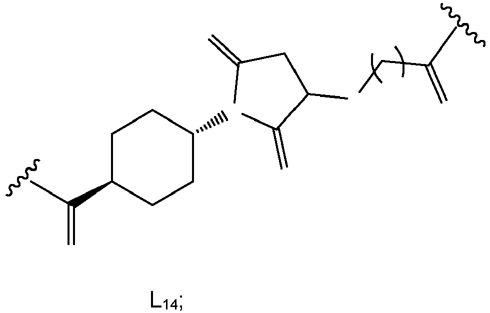 et 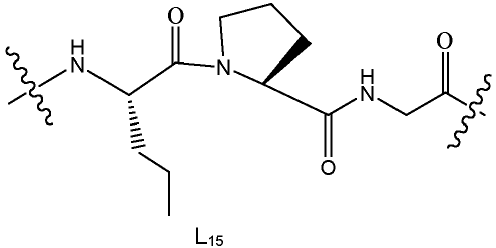
P est un biradical d'un peptide choisi dans le groupe constitué de :
(a) un peptide qui comprend la séquence d'acides aminés X₁KAPETALX₂ avec une liaison intrapeptidique entre X₁ et X₂ qui est une liaison amide ; où X₁ est choisi dans le groupe constitué de Dap et Dab ; et X₂ est choisi dans le groupe constitué de D (acide aspartique) et E (acide glutamique) ; c'est-à-dire 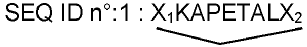
SEQ ID n°:1 : X1KAPETALX2
(b) un peptide ayant une longueur de 12 à 20 résidus acides aminés ayant au moins une liaison intrapeptidique qui est une liaison disulfure ou disélénure, et qui comprend une séquence d'acides aminés qui est : X₃KAPETALX₄AAA ; ayant au moins une liaison intrapeptidique disulfure ou disélénure entre X₃ et X₄, dans laquelle X₃ et X₄ sont égaux et sont choisis dans le groupe constitué de C (cystéines), Sec (sélénocystéines) et Pen (pénicillamines) ; 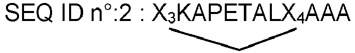
SEQ ID n°:2 : X3KAPETALX4AAA
(c) un peptide ayant une longueur de 9 à 11 résidus acides aminés ayant au moins une liaison intrapeptidique qui est une liaison disulfure ou disélénure et qui est constitué d'une séquence d'acides aminés choisie dans le groupe constitué de X₅KAPETALX₆ ; X₅KAPETALX₆A ; et X₅KAPETALX₆AA ayant au moins une liaison disulfure ou disélénure intrapeptidique entre X₅ et X₆ ; où X₅ et X₆ sont égaux et sont choisis dans le groupe constitué de C (cystéines), Sec (sélénocystéines) et Pen (pénicillamines), c'est-à-dire 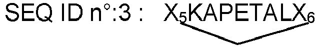
SEQ ID n°:3 : X5KAPETALX6
 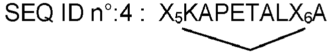
SEQ ID n°:4 : X5KAPETALX6A
 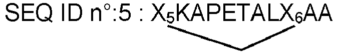
SEQ ID n°:5 : X5KAPETALX6AA
(d) un peptide qui a 16 résidus acides aminés et comprend la séquence d'acides aminés X₇NX₈KAPETALX₉AAAX₁₀H avec une liaison disulfure ou disélénure intrapeptidique entre X₇ et X₉, et entre X₈ et X₁₀ ; où X₇-X₁₀ sont choisis indépendamment dans le groupe constitué de C (cystéines), Sec (sélénocystéines) et Pen (pénicillamines) ; à condition que X₇ et X₉ soient égaux et que X₈-X₁₀ soient égaux, c'est-à-dire 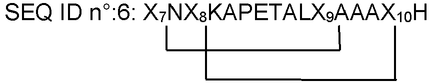
SEQ ID n°:6: X7NX8KAPETALX9AAAX10H
et (e) un peptide qui comprend la séquence d'acides aminés X₁KAPETALX₂ dans laquelle X₁ est choisi dans le groupe constitué de Dap et Dab ; et X₂ est choisi dans le groupe constitué de D (acide aspartique) et E (acide glutamique) (SEQ ID n°:7) ;
W est un biradical choisi dans le groupe constitué de -NH-(CH₂)ᵣ₋C(=O)-, et -NH-CH((CH₂)ᵣNH₂)-C(=O)- ;
Y est un radical choisi dans le groupe constitué de -NH₂, -OH, -OR₃ et -NHR₃ ;
s est un nombre entier choisi indépendamment de 0 à 1 ;
n est un nombre entier de 0 à 6 ;
r est un nombre entier choisi indépendamment de 1 à 5 ;
k est un nombre entier allant de 5 à 8;
R₁ et R₂ sont indépendamment choisis parmi un alkyle en (C₁-C₆) ;
R₃ est un radical choisi dans le groupe constitué d'alkyle en (C₁-C₆) ;
Lₐ' est attaché au radical Z à travers une liaison qui est choisie dans le groupe constitué d'une liaison ester, éther, uréthane, éther silylique, sulfonate, phosphate, cétal, hémikétal, carbonate et carbamate, la liaison étant formée entre les groupes C=O, SO₂, Si, P, CH ou CH₂ sur le côté gauche des formules dessinées Lₐ' et l'un des groupes hydroxyle du SN38 ;
lorsque n=0, Lₐ' est attaché au radical L_{c}' à travers une liaison chimiquement réalisable qui est choisie dans le groupe constitué d'amine, amide, éther, thioéther, disulfure, ester et thioester, la liaison étant formée entre les groupes fonctionnels sur le côté droit des formules dessinées Lₐ' et les groupes fonctionnels du côté gauche des formules L'_{c};
lorsque n=1, Lₐ' est lié au radical L_{b}' à travers une liaison chimiquement réalisable qui est choisie dans le groupe constitué d'amine, amide, éther, thioéther, ester de disulfure et thioester, la liaison étant formée entre les groupes fonctionnels sur le côté droit des formules dessinées Lₐ' et les groupes fonctionnels sur le côté gauche des formules L_{b}' ; et L_{b}' est lié au radical L_{c}' à travers une liaison chimiquement réalisable qui est choisie dans le groupe constitué d'amine, amide, éther, thioéther, disulfure, ester et thioester, la liaison étant formée entre les groupes fonctionnels sur le côté droit des formules dessinées L_{b}' et les groupes fonctionnels sur le côté gauche des formules dessinées L_{c}' ;
lorsque n est supérieur à 1, L_{b}' sont égaux ou différents et sont attachés entre eux à travers une liaison chimiquement réalisable choisie dans le groupe constitué d'amine, amide, éther, thioéther, disulfure, ester et thioester ; étant une extrémité L_{b}' attachée à Lₐ' à travers une liaison chimiquement réalisable qui est choisie dans le groupe constitué d'amine, amide, éther, thioéther, disulfure, ester et thioester, la liaison étant formée entre les groupes fonctionnels sur le côté droit des formules dessinées Lₐ' et les groupes fonctionnels du côté gauche des formules dessinées L_{b}' ; et étant une autre extrémité L_{b}' attachée à L_{c}' à travers une liaison chimiquement réalisable qui est choisie dans le groupe constitué d'amine, amide, éther, thioéther, disulfure, ester et thioester, la liaison étant formée entre le groupe fonctionnel sur le côté droit des formules dessinées L_{b}' et le groupe fonctionnel sur le côté gauche des formules dessinées L_{c}' ;
L_{c}' est attaché au biradical P à travers une liaison amide formée avec le groupe carbonyle sur le côté droit des formules dessinées L_{c}' et un groupe amino du premier acide aminé de la séquence peptidique P ;
lorsque s=0, P est directement lié à Y à travers une liaison amide, acide carboxylique ou ester, la liaison étant formée entre le C=O de l'extrémité C du dernier acide aminé de la séquence P, et le radical Y qui est -NH₂, -OH, -OR₃ ou -NHR₃ ; et
lorsque s=1, P est attaché à un radical W à travers une liaison amide formée avec un C=O de l'extrémité C du dernier acide aminé de la séquence P, la liaison étant formée entre les groupes fonctionnels sur le côté gauche des formules dessinées W et les groupes fonctionnels (C=O) de l'extrémité C du dernier acide aminé de la séquence P sur le côté droit de la séquence dessinée ; et W est attaché à Y comme suit : -C(=O)-NH-(CH₂)ᵣ₋C(=O)-Y, ou -C(=O)-NH-CH((CH₂)ᵣNH₂)-C(=O)-Y.

2. Le composé de formule (I) selon la revendication 1, dans lequel Z est attaché à un lieur L par le groupe hydroxyle (b) de l'ingrédient actif pharmaceutique.

3. Le composé de formule (I) selon la revendication 1, dans lequel Z est attaché à un lieur L par le groupe hydroxyle (a) de l'ingrédient actif pharmaceutique.

4. Le composé de formule (I) selon l'une quelconque des revendications 1 à 3, dans lequel P est un biradical d'un peptide choisi dans le groupe constitué par :
(a) un peptide qui comprend la séquence d'acides aminés DapKAPETALD avec une liaison intrapeptide entre le Dap et le D qui est une liaison amide, c'est-à-dire la SEQ ID 
n°:8 : DapKAPETALD
NHCO
(b) un peptide ayant une longueur de 9 à 20 résidus acides aminés ayant au moins une liaison intrapeptidique qui est une liaison disulfure, et qui comprend une séquence d'acides aminés qui est :
CKAPETALCAAA ayant au moins une liaison disulfure intrapeptidique entre les cystéines 1 et 9, c'est-à-dire la 
SEQ ID n°:9 : CKAPETALCAAA
S-S
(c) un peptide ayant une longueur de 9 à 11 acides aminés ayant au moins une liaison intrapeptidique qui est une liaison disulfure et qui consiste en une séquence d'acides aminés choisie dans le groupe constitué de CKAPETALC ; CKAPETALCA ; et CKAPETALCAA ayant au moins une liaison disulfure intrapeptidique entre les cystéines 1 et 9, qui sont 
SEQ ID n°:10 : CKAPETALC
S-S
 
SEQ ID n°:11 CKAPETALCA
S-S
 
SEQ ID n°:12) CKAPETALCAA;
S-S
 et
(d) un peptide qui a 16 résidus acides aminés et comprend la séquence d'acides aminés CNCKAPETALCAAACH avec une liaison disulfure intrapeptidique entre la première et la troisième cystéine qui sont les cystéines 1 et 11, et entre la deuxième et la quatrième cystéine, qui sont les cystéines 3 et 15, qui est 
SEQ ID n°:13: CNCKAPETALCAAACH
S-S
S-S
(e) un peptide qui comprend la séquence d'acides aminés DapKAPETALD (SEQ ID n°:14).

5. Le composé de formule (I) selon la revendication 4, dans lequel P est un biradical d'un peptide choisi dans le groupe constitué par :
(a) le peptide ayant la séquence d'acides aminés DapKAPETALD avec une liaison intrapeptide entre le Dap et le D qui est une liaison amide (SEQ ID n°:7) ;
(b) le peptide ayant la séquence d'acides aminés CKAPETALC ayant au moins une liaison disulfure intrapeptidique entre les cystéines en position 1 et 9 (SEQ ID n°:10 ;
(c) le peptide ayant la séquence d'acides aminés DapKAPETALD (SEQ ID n°:14).

6. Le composé de formule (I) selon l'une quelconque des revendications 1 à 5, dans lequel
Lₐ' est un biradical choisi dans le groupe constitué de : -C(=O)-(CH₂)ᵣ-C(=O)-; -C(=O)-(CH₂)ᵣ-NH-, -C(=O)-(CH₂)ᵣ-S-; -C(=O)-(CH₂)ᵣ-O-; -C(=O)-NH-(CH₂)ᵣ-C(=O)-; L₁, L₂, L₃, L₄, L₅, L₆, L₇ et L₁₂.

7. Le composé selon l'une quelconque des revendications 1 à 6, dans lequel L est un lieur qui est un biradical composé de 3 à 8 biradicaux et n est un nombre entier de 1 à 6.

8. Le composé selon la revendication 7, dans lequel Lₐ' est L₃ de la formule ci-dessous et L_{c}' est -C(=O)-(CH₂)ᵣ-C(=O)-,

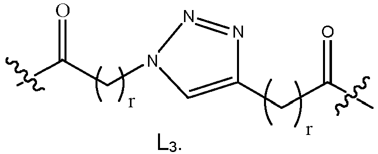

9. Le composé selon la revendication 8, dans lequel L_{b}' est choisi dans le groupe constitué de -NH-(CH₂)ᵣ-O-, -(CH₂)ᵣ-O- ; et -(CH₂)ᵣ-NH-, et des combinaisons de ceux-ci.

10. Le composé de formule (I) selon l'une quelconque des revendications 1 à 5, dans lequel
a) L est un lieur qui est un biradical composé de 2 biradicaux, n=0, Lₐ' est L₁₂ et L_{c}' est L₁₃ ; ou alternativement,
b) L est un lieur qui est un biradical composé de 2 biradicaux, n=0, Lₐ' est -C(=O)-NH-(CH₂)ᵣ-C(=O)-, et L_{c}' est L₁₅.

11. Le composé selon la revendication 1, qui est choisi dans la liste suivante :

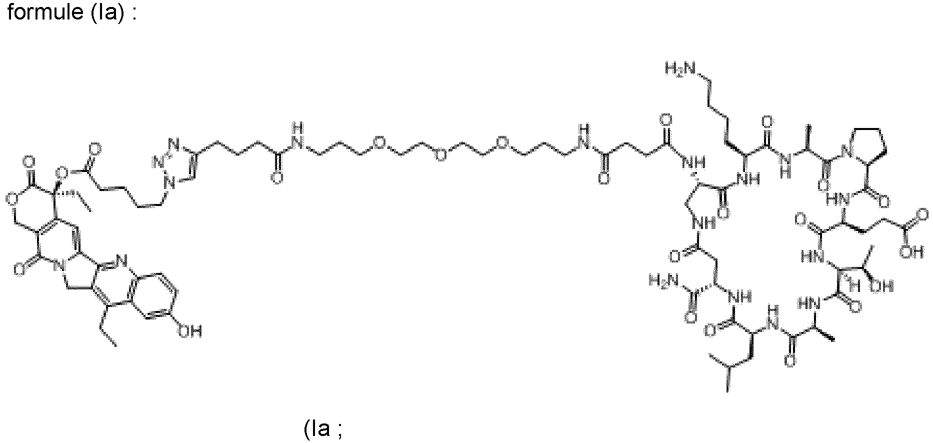

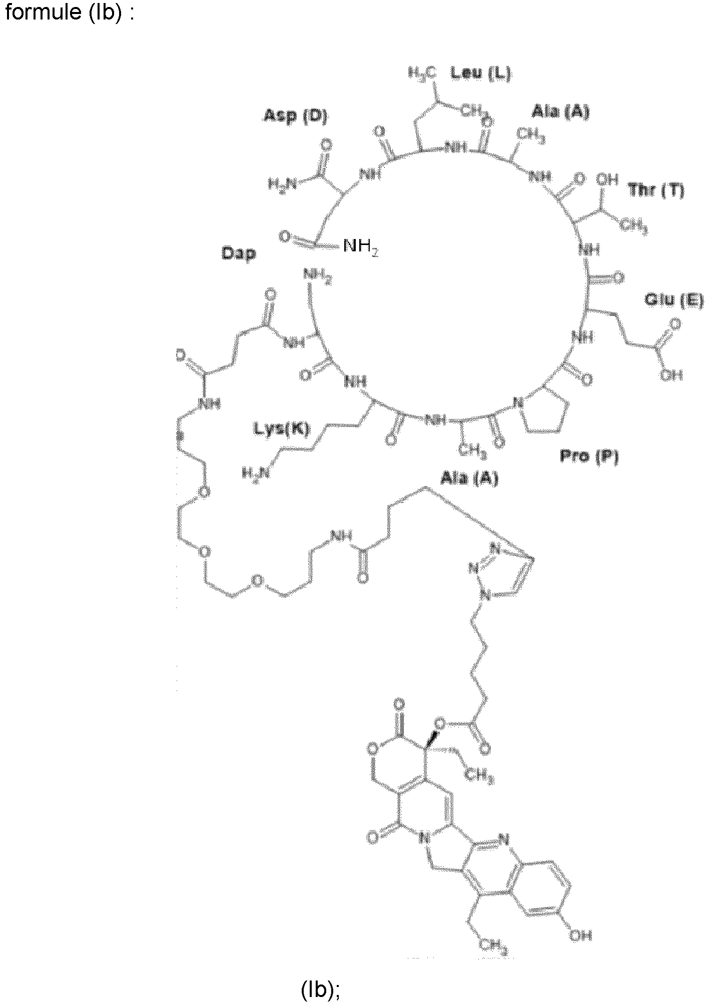

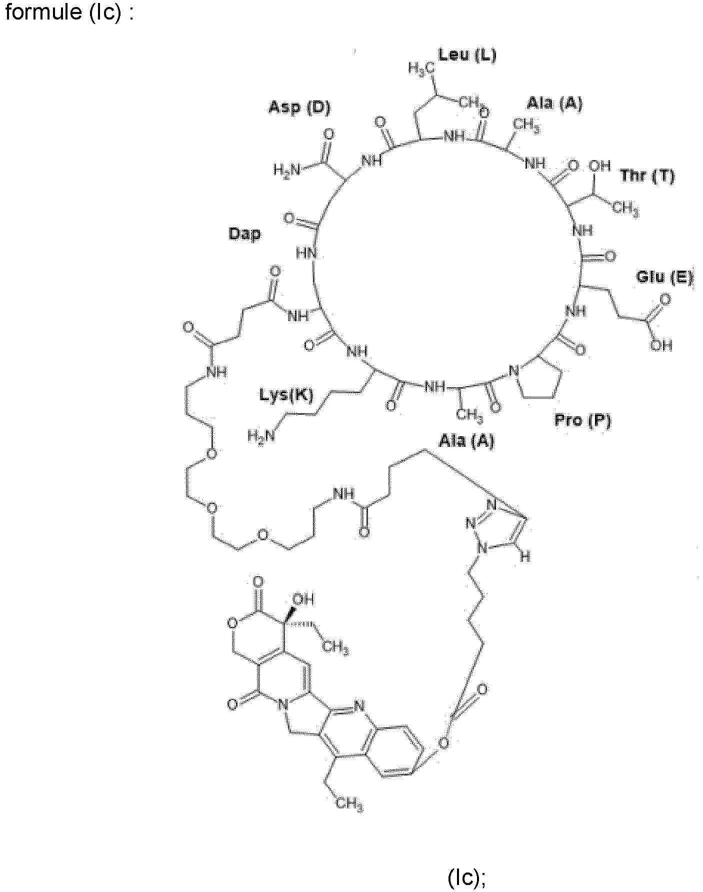

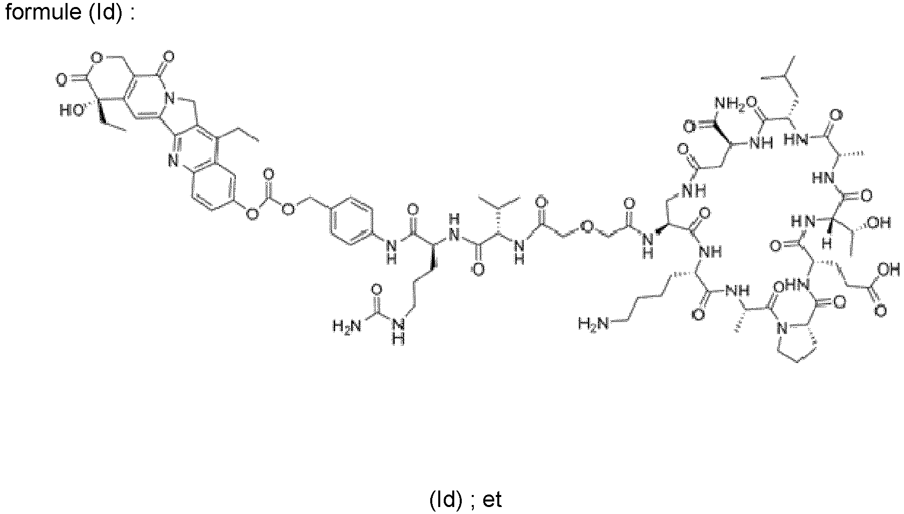

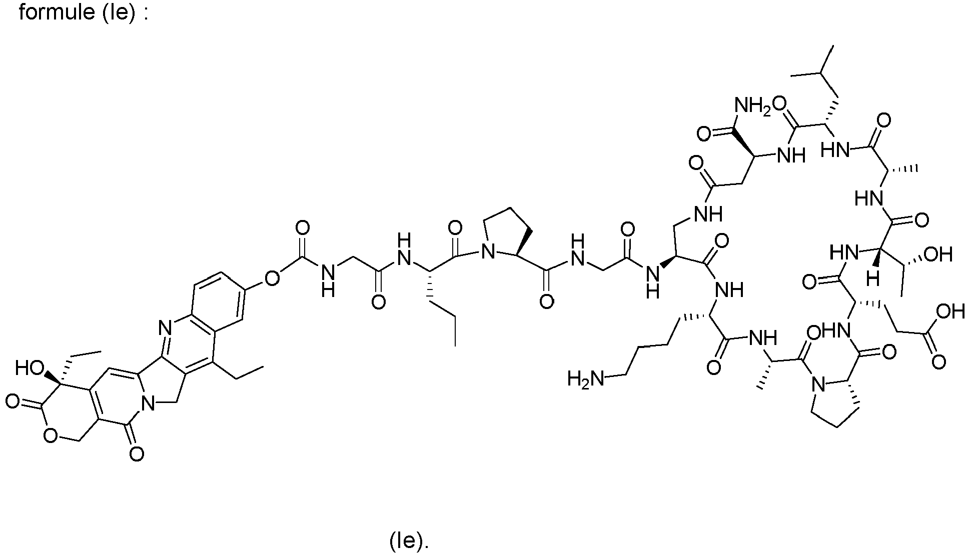

12. Une composition pharmaceutique comprenant une quantité thérapeutiquement efficace du composé tel que défini dans l'une quelconque des revendications 1 à 11, avec des quantités appropriées de véhicules ou d'excipients pharmaceutiquement acceptables.

13. Un composé tel que défini dans l'une quelconque des revendications 1 à 11, pour une utilisation en tant que médicament.

14. Un composé tel que défini dans l'une quelconque des revendications 1 à 11, pour l'utilisation dans le traitement du cancer chez un mammifère, y compris un humain.

15. Le composé pour l'utilisation selon la revendication 14, dans lequel le traitement du cancer comprend le traitement soit d'une tumeur choisie dans le groupe constitué des tumeurs solides extracrâniennes, des tumeurs oculaires et des tumeurs du SNC ; soit d'un cancer choisi dans le groupe constitué du gliome adulte, des gliomes pédiatriques, du rétinoblastome, du sarcome d'Ewing, du GITC, du neuroblastome et du rhabdomyosarcome.
